# EUROPEAN PATENT SPECIFICATION

(11) **EP 3 253 795 B1**
(45) Date of publication and mention of the grant of the patent: **17.04.2019**
(21) Application number: 16702746.5
(22) Date of filing: 04.02.2016
(51) Int. Cl.: C07K 16/28, C07K 16/32, C07K 14/00, C07K 19/00

(54) **NOVEL BINDING PROTEINS COMPRISING A UBIQUITIN MUTEIN AND ANTIBODIES OR ANTIBODY FRAGMENTS**
NEUE BINDENDE PROTEINE MIT UBIQUITINMUTEIN UND ANTIKÖRPERN ODER ANTIKÖRPERFRAGMENTEN
NOUVELLES PROTÉINES DE LIAISON COMPRENANT UNE MUTÉINE D'UBIQUITINE ET DES ANTICORPS OU DES FRAGMENTS D'ANTICORPS

(30) Priority: 06.02.2015 EP 15154150; 29.05.2015 EP 15169863
(43) Date of publication of application: 13.12.2017
(73) Proprietor: NAVIGO PROTEINS GMBH, 06120 Halle/Saale (DE)
(72) Inventor: BOSSE-DOENECKE, Eva, 06120 Halle/Saale (DE); KAHL, Mathias, 06120 Halle/Saale (DE); SETTELE, Florian, 06120 Halle/Saale (DE); KNICK, Paul, 06120 Halle/Saale (DE); LIEBSCHER, Markus, 06120 Halle/Saale (DE); FIEDLER, Erik, 06120 Halle/Saale (DE); HENNICKE, Julia, 06120 Halle/Saale (DE)
(74) Representative: Grünecker Patent- und Rechtsanwälte PartG mbB
(86) International application number: PCT/EP2016/052345
(87) International publication number: WO 2016/124670

(56) References cited:
- EP-A1- 1 591 527
- EP-A1- 2 727 942
- EP-A1- 2 829 552
- MIKAELA FRIEDMAN ET AL: "Engineering and characterization of a bispecific HER2 x EGFR-binding affibody molecule", BIOTECHNOLOGY AND APPLIED BIOCHEMISTRY, ACADEMIC PRESS, US, vol. 54, no. 2, 1 October 2009 (2009-10-01), pages 121-131, XP002679895, ISSN: 0885-4513, DOI: 10.1042/BA20090096 [retrieved on 2010-12-24]
- LOREY SUSAN ET AL: "Novel Ubiquitin-derived High Affinity Binding Proteins with Tumor Targeting Properties", JOURNAL OF BIOLOGICAL CHEMISTRY, vol. 289, no. 12, March 2014 (2014-03), pages 8493-8507, XP002742090,
- WEIDLE U H; AUER J; BRINKMANN U; GEORGES G; TIEFENTHALER G: "The emerging role of new protein scaffold-based agents for treatment of cancer", CANCER GENOMICS & PROTEOMICS, vol. 10, no. 4, 1 July 2013 (2013-07-01), pages 155-168, XP055200617, GR ISSN: 1109-6535

### SEQUENCE LISTING

<110> Scil Proteins GmbH
<120> Novel binding proteins comprising a ubiquitin mutein and antibodies or antibody fragments
<130> SP26 WO
<160> 80
<170> PatentIn version 3.5
<210> 1
   <211> 76
   <212> PRT
   <213> artificial
<220>
   <223> Ubiquitin reference
<400> 1
<210> 2
   <211> 76
   <212> PRT
   <213> artificial
<220>
   <223> Ubiquitin reference
<400> 2
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Trp Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Ala Ala
   65                  70                  75
<210> 3
   <211> 76
   <212> PRT
   <213> artificial
<220>
   <223> Ubiquitin reference
<220>
   <221> misc_feature
   <222> (62)..(66)
   <223> Xaa can be any naturally occurring amino acid
<400> 3
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ser Asp Tyr Asn Ile Xaa Xaa Xaa
       50                  55                  60
   Xaa Xaa Leu His Leu Val Leu Arg Leu Arg Ala Ala
   65                  70                  75
<210> 4
   <211> 152
   <212> PRT
   <213> artificial
<220>
   <223> Ubiquitin reference clone 139090
<400> 4
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Ala Ala Met Gln Ile Phe
   65                  70                  75                  80
   Val Lys Thr Leu Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Phe Ala Gly Lys Gln Leu Glu Asp
           115                 120                 125
   Gly Arg Thr Leu Ser Asp Tyr Asn Ile Gln Lys Glu Ser Thr Leu His
       130                 135                 140
   Leu Val Leu Arg Leu Arg Ala Ala
   145                 150
<210> 5
   <211> 468
   <212> PRT
   <213> artificial
<220>
   <223> Cetuximab HC
<400> 5
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
   340 345 350
   Ile Glu Lys Thr Ile Ser Lys Ala Lys Gly Gln Pro Arg Glu Pro Gln
   355 360 365
   Val Tyr Thr Leu Pro Pro Ser Arg Glu Glu Met Thr Lys Asn Gln Val
   370 375 380
   Ser Leu Thr Cys Leu Val Lys Gly Phe Tyr Pro Ser Asp Ile Ala Val
   385 390 395 400
   Glu Trp Glu Ser Asn Gly Gln Pro Glu Asn Asn Tyr Lys Thr Thr Pro
   405 410 415
   Pro Val Leu Asp Ser Asp Gly Ser Phe Phe Leu Tyr Ser Lys Leu Thr
   420 425 430
   Val Asp Lys Ser Arg Trp Gln Gln Gly Asn Val Phe Ser Cys Ser Val
   435 440 445
   Met His Glu Ala Leu His Asn His Tyr Thr Gln Lys Ser Leu Ser Leu
   450 455 460
   Ser Pro Gly Lys
   465
<210> 6
   <211> 234
   <212> PRT
   <213> artificial
<220>
   <223> Cetuximab LC
<400> 6
   Met Val Ser Thr Pro Gln Phe Leu Val Phe Leu Leu Phe Trp Ile Pro
   1 5 10 15
   Ala Ser Arg Ser Asp Ile Leu Leu Thr Gln Ser Pro Val Ile Leu Ser
   20 25 30
   Val Ser Pro Gly Glu Arg Val Ser Phe Ser Cys Arg Ala Ser Gln Ser
   35 40 45
   Ile Gly Thr Asn Ile His Trp Tyr Gln Gln Arg Thr Asn Gly Ser Pro
   50 55 60
   Arg Leu Leu Ile Lys Tyr Ala Ser Glu Ser Ile Ser Gly Ile Pro Ser
   65                  70                  75                  80
   Arg Phe Ser Gly Ser Gly Ser Gly Thr Asp Phe Thr Leu Ser Ile Asn
                   85                  90                  95
   Ser Val Glu Ser Glu Asp Ile Ala Asp Tyr Tyr Cys Gln Gln Asn Asn
               100                 105                 110
   Asn Trp Pro Thr Thr Phe Gly Ala Gly Thr Lys Leu Glu Leu Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 7
   <211> 82
   <212> PRT
   <213> artificial
<220>
   <223> Affilin 139791
<400> 7
   Met Gln Ile Phe Val Lys Thr Leu Thr Pro Trp Arg Gly Tyr Asp Gly
   1               5                   10                  15
   Lys Thr Ile Thr Leu Glu Val Glu Pro Ser Asp Thr Ile Glu Asn Val
               20                  25                  30
   Lys Ala Lys Ile Gln Asp Lys Glu Gly Ile Pro Pro Asp Gln Gln Arg
           35                  40                  45
   Leu Ile Trp Ala Gly Lys Gln Leu Glu Asp Gly Arg Thr Leu Ser Asp
       50                  55                  60
   Tyr Asn Ile Arg Arg Val Asp Val Leu His Leu Val Leu Arg Leu Arg
   65                  70                  75                  80
   Ala Ala
<210> 8
   <211> 155
   <212> PRT
   <213> artificial
<220>
   <223> Affilin 139864
<400> 8
   Met Gln Ile Phe Val His Thr Pro Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Trp Ala Gly Lys
           35                  40                  45
   Gln Leu Glu Asp Gly Arg Thr Leu Ser Asp Tyr Asn Ile Arg Asn Pro
       50                  55                  60
   Asp Ile Leu His Leu Val Leu Arg Leu Arg Ala Ala Gly Ile Gly Met
   65                  70                  75                  80
   Gln Ile Phe Val His Thr Met Thr Gly Lys Thr Ile Thr Leu Glu Val
                   85                  90                  95
   Glu Pro Ser Asp Thr Ile Glu Asn Val Lys Ala Lys Ile Gln Asp Lys
               100                 105                 110
   Glu Gly Ile Pro Pro Asp Gln Gln Arg Leu Ile Trp Ala Gly Lys Gln
           115                 120                 125
   Leu Glu Asp Gly Arg Thr Leu Ser Asp Tyr Asn Ile Gly Ala Gly Thr
       130                 135                 140
   Met Leu His Leu Val Leu Arg Leu Arg Ala Ala
   145                 150                 155
<210> 9
   <211> 82
   <212> PRT
   <213> artificial
<220>
   <223> Affilin 139819
<400> 9
   Met Gln Ile Phe Val Lys Thr Leu Thr Tyr Asn Pro Met Arg Tyr Gly
   1               5                   10                  15
   Lys Thr Ile Thr Leu Glu Val Glu Pro Ser Asp Thr Ile Glu Asn Val
               20                  25                  30
   Lys Ala Lys Ile Gln Asp Lys Glu Gly Ile Pro Pro Asp Gln Gln Arg
           35                  40                  45
   Leu Ile Trp Ala Gly Lys Gln Leu Glu Asp Gly Arg Thr Leu Ser Asp
       50                  55                  60
   Tyr Asn Ile Arg Asn Pro Asp Ile Leu His Leu Val Leu Arg Leu Arg
   65                  70                  75                  80
   Ala Ala
<210> 10
   <211> 152
   <212> PRT
   <213> artificial
<220>
   <223> Affilin 141926
<400> 10
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Ser Glu Trp Ala Ile Leu His
   130 135 140
   Leu Val Leu Arg Leu Arg Ala Ala
   145 150
<210> 11
   <211> 565
   <212> PRT
   <213> artificial
<220>
   <223> Fusion protein NH 139791
<400> 11
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Pro Trp Arg Gly
               20                  25                  30
   Tyr Asp Gly Lys Thr Ile Thr Leu Glu Val Glu Pro Ser Asp Thr Ile
           35                  40                  45
   Glu Asn Val Lys Ala Lys Ile Gln Asp Lys Glu Gly Ile Pro Pro Asp
       50                  55                  60
   Gln Gln Arg Leu Ile Trp Ala Gly Lys Gln Leu Glu Asp Gly Arg Thr
   65                  70                  75                  80
   Leu Ser Asp Tyr Asn Ile Arg Arg Val Asp Val Leu His Leu Val Leu
                   85                  90                  95
   Arg Leu Arg Ala Ala Gly Gly Gly Gly Ser Gly Gly Gly Gly Ser Gly
               100                 105                 110
   Gly Gly Gly Ser Gln Val Gln Leu Lys Gln Ser Gly Pro Gly Leu Val
           115                 120                 125
   Gln Pro Ser Gln Ser Leu Ser Ile Thr Cys Thr Val Ser Gly Phe Ser
       130                 135                 140
   Leu Thr Asn Tyr Gly Val His Trp Val Arg Gln Ser Pro Gly Lys Gly
   145                 150                 155                 160
   Leu Glu Trp Leu Gly Val Ile Trp Ser Gly Gly Asn Thr Asp Tyr Asn
                   165                 170                 175
   Thr Pro Phe Thr Ser Arg Leu Ser Ile Asn Lys Asp Asn Ser Lys Ser
               180                 185                 190
   Gln Val Phe Phe Lys Met Asn Ser Leu Gln Ser Asn Asp Thr Ala Ile
           195                 200                 205
   Tyr Tyr Cys Ala Arg Ala Leu Thr Tyr Tyr Asp Tyr Glu Phe Ala Tyr
       210                 215                 220
   Trp Gly Gln Gly Thr Leu Val Thr Val Ser Ala Ala Ser Thr Lys Gly
   225                 230                 235                 240
   Pro Ser Val Phe Pro Leu Ala Pro Ser Ser Lys Ser Thr Ser Gly Gly
                   245                 250                 255
   Thr Ala Ala Leu Gly Cys Leu Val Lys Asp Tyr Phe Pro Glu Pro Val
               260                 265                 270
   Thr Val Ser Trp Asn Ser Gly Ala Leu Thr Ser Gly Val His Thr Phe
           275                 280                 285
   Pro Ala Val Leu Gln Ser Ser Gly Leu Tyr Ser Leu Ser Ser Val Val
       290                 295                 300
   Thr Val Pro Ser Ser Ser Leu Gly Thr Gln Thr Tyr Ile Cys Asn Val
   305                 310                 315                 320
   Asn His Lys Pro Ser Asn Thr Lys Val Asp Lys Arg Val Glu Pro Lys
                   325                 330                 335
   Ser Cys Asp Lys Thr His Thr Cys Pro Pro Cys Pro Ala Pro Glu Leu
               340                 345                 350
   Leu Gly Gly Pro Ser Val Phe Leu Phe Pro Pro Lys Pro Lys Asp Thr
           355                 360                 365
   Leu Met Ile Ser Arg Thr Pro Glu Val Thr Cys Val Val Val Asp Val
   370 375 380
   Ser His Glu Asp Pro Glu Val Lys Phe Asn Trp Tyr Val Asp Gly Val
   385 390 395 400
   Glu Val His Asn Ala Lys Thr Lys Pro Arg Glu Glu Gln Tyr Asn Ser
   405 410 415
   Thr Tyr Arg Val Val Ser Val Leu Thr Val Leu His Gln Asp Trp Leu
   420 425 430
   Asn Gly Lys Glu Tyr Lys Cys Lys Val Ser Asn Lys Ala Leu Pro Ala
   435 440 445
   Pro Ile Glu Lys Thr Ile Ser Lys Ala Lys Gly Gln Pro Arg Glu Pro
   450 455 460
   Gln Val Tyr Thr Leu Pro Pro Ser Arg Glu Glu Met Thr Lys Asn Gln
   465 470 475 480
   Val Ser Leu Thr Cys Leu Val Lys Gly Phe Tyr Pro Ser Asp Ile Ala
   485 490 495
   Val Glu Trp Glu Ser Asn Gly Gln Pro Glu Asn Asn Tyr Lys Thr Thr
   500 505 510
   Pro Pro Val Leu Asp Ser Asp Gly Ser Phe Phe Leu Tyr Ser Lys Leu
   515 520 525
   Thr Val Asp Lys Ser Arg Trp Gln Gln Gly Asn Val Phe Ser Cys Ser
   530 535 540
   Val Met His Glu Ala Leu His Asn His Tyr Thr Gln Lys Ser Leu Ser
   545 550 555 560
   Leu Ser Pro Gly Lys
   565
<210> 12
   <211> 638
   <212> PRT
   <213> artificial
<220>
   <223> Fusion protein NH 139864
<400> 12
   Met Ala Val Leu Gly Leu Leu Phe Cys Leu Val Thr Phe Pro Ser Cys
   1               5                   10                  15
   Val Leu Ser Met Gln Ile Phe Val His Thr Pro Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Arg Leu Ile Trp
       50                  55                  60
   Ala Gly Lys Gln Leu Glu Asp Gly Arg Thr Leu Ser Asp Tyr Asn Ile
   65                  70                  75                  80
   Arg Asn Pro Asp Ile Leu His Leu Val Leu Arg Leu Arg Ala Ala Gly
                   85                  90                  95
   Ile Gly Met Gln Ile Phe Val His Thr Met Thr Gly Lys Thr Ile Thr
               100                 105                 110
   Leu Glu Val Glu Pro Ser Asp Thr Ile Glu Asn Val Lys Ala Lys Ile
           115                 120                 125
   Gln Asp Lys Glu Gly Ile Pro Pro Asp Gln Gln Arg Leu Ile Trp Ala
       130                 135                 140
   Gly Lys Gln Leu Glu Asp Gly Arg Thr Leu Ser Asp Tyr Asn Ile Gly
   145                 150                 155                 160
   Ala Gly Thr Met Leu His Leu Val Leu Arg Leu Arg Ala Ala Gly Gly
                   165                 170                 175
   Gly Gly Ser Gly Gly Gly Gly Ser Gly Gly Gly Gly Ser Gln Val Gln
               180                 185                 190
   Leu Lys Gln Ser Gly Pro Gly Leu Val Gln Pro Ser Gln Ser Leu Ser
           195                 200                 205
   Ile Thr Cys Thr Val Ser Gly Phe Ser Leu Thr Asn Tyr Gly Val His
       210                 215                 220
   Trp Val Arg Gln Ser Pro Gly Lys Gly Leu Glu Trp Leu Gly Val Ile
   225                 230                 235                 240
   Trp Ser Gly Gly Asn Thr Asp Tyr Asn Thr Pro Phe Thr Ser Arg Leu
                   245                 250                 255
   Ser Ile Asn Lys Asp Asn Ser Lys Ser Gln Val Phe Phe Lys Met Asn
   260 265 270
   Ser Leu Gln Ser Asn Asp Thr Ala Ile Tyr Tyr Cys Ala Arg Ala Leu
   275 280 285
   Thr Tyr Tyr Asp Tyr Glu Phe Ala Tyr Trp Gly Gln Gly Thr Leu Val
   290 295 300
   Thr Val Ser Ala Ala Ser Thr Lys Gly Pro Ser Val Phe Pro Leu Ala
   305 310 315 320
   Pro Ser Ser Lys Ser Thr Ser Gly Gly Thr Ala Ala Leu Gly Cys Leu
   325 330 335
   Val Lys Asp Tyr Phe Pro Glu Pro Val Thr Val Ser Trp Asn Ser Gly
   340 345 350
   Ala Leu Thr Ser Gly Val His Thr Phe Pro Ala Val Leu Gln Ser Ser
   355 360 365
   Gly Leu Tyr Ser Leu Ser Ser Val Val Thr Val Pro Ser Ser Ser Leu
   370 375 380
   Gly Thr Gln Thr Tyr Ile Cys Asn Val Asn His Lys Pro Ser Asn Thr
   385 390 395 400
   Lys Val Asp Lys Arg Val Glu Pro Lys Ser Cys Asp Lys Thr His Thr
   405 410 415
   Cys Pro Pro Cys Pro Ala Pro Glu Leu Leu Gly Gly Pro Ser Val Phe
   420 425 430
   Leu Phe Pro Pro Lys Pro Lys Asp Thr Leu Met Ile Ser Arg Thr Pro
   435 440 445
   Glu Val Thr Cys Val Val Val Asp Val Ser His Glu Asp Pro Glu Val
   450 455 460
   Lys Phe Asn Trp Tyr Val Asp Gly Val Glu Val His Asn Ala Lys Thr
   465 470 475 480
   Lys Pro Arg Glu Glu Gln Tyr Asn Ser Thr Tyr Arg Val Val Ser Val
   485 490 495
   Leu Thr Val Leu His Gln Asp Trp Leu Asn Gly Lys Glu Tyr Lys Cys
   500 505 510
   Lys Val Ser Asn Lys Ala Leu Pro Ala Pro Ile Glu Lys Thr Ile Ser
   515 520 525
   Lys Ala Lys Gly Gln Pro Arg Glu Pro Gln Val Tyr Thr Leu Pro Pro
   530 535 540
   Ser Arg Glu Glu Met Thr Lys Asn Gln Val Ser Leu Thr Cys Leu Val
   545 550 555 560
   Lys Gly Phe Tyr Pro Ser Asp Ile Ala Val Glu Trp Glu Ser Asn Gly
   565 570 575
   Gln Pro Glu Asn Asn Tyr Lys Thr Thr Pro Pro Val Leu Asp Ser Asp
   580 585 590
   Gly Ser Phe Phe Leu Tyr Ser Lys Leu Thr Val Asp Lys Ser Arg Trp
   595 600 605
   Gln Gln Gly Asn Val Phe Ser Cys Ser Val Met His Glu Ala Leu His
   610 615 620
   Asn His Tyr Thr Gln Lys Ser Leu Ser Leu Ser Pro Gly Lys
   625 630 635
<210> 13
   <211> 565
   <212> PRT
   <213> artificial
<220>
   <223> Fusion protein NH 139819
<400> 13
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Tyr Asn Pro Met
               20                  25                  30
   Arg Tyr Gly Lys Thr Ile Thr Leu Glu Val Glu Pro Ser Asp Thr Ile
           35                  40                  45
   Glu Asn Val Lys Ala Lys Ile Gln Asp Lys Glu Gly Ile Pro Pro Asp
       50                  55                  60
   Gln Gln Arg Leu Ile Trp Ala Gly Lys Gln Leu Glu Asp Gly Arg Thr
   65                  70                  75                  80
   Leu Ser Asp Tyr Asn Ile Arg Asn Pro Asp Ile Leu His Leu Val Leu
   85 90 95
   Arg Leu Arg Ala Ala Gly Gly Gly Gly Ser Gly Gly Gly Gly Ser Gly
   100 105 110
   Gly Gly Gly Ser Gln Val Gln Leu Lys Gln Ser Gly Pro Gly Leu Val
   115 120 125
   Gln Pro Ser Gln Ser Leu Ser Ile Thr Cys Thr Val Ser Gly Phe Ser
   130 135 140
   Leu Thr Asn Tyr Gly Val His Trp Val Arg Gln Ser Pro Gly Lys Gly
   145 150 155 160
   Leu Glu Trp Leu Gly Val Ile Trp Ser Gly Gly Asn Thr Asp Tyr Asn
   165 170 175
   Thr Pro Phe Thr Ser Arg Leu Ser Ile Asn Lys Asp Asn Ser Lys Ser
   180 185 190
   Gln Val Phe Phe Lys Met Asn Ser Leu Gln Ser Asn Asp Thr Ala Ile
   195 200 205
   Tyr Tyr Cys Ala Arg Ala Leu Thr Tyr Tyr Asp Tyr Glu Phe Ala Tyr
   210 215 220
   Trp Gly Gln Gly Thr Leu Val Thr Val Ser Ala Ala Ser Thr Lys Gly
   225 230 235 240
   Pro Ser Val Phe Pro Leu Ala Pro Ser Ser Lys Ser Thr Ser Gly Gly
   245 250 255
   Thr Ala Ala Leu Gly Cys Leu Val Lys Asp Tyr Phe Pro Glu Pro Val
   260 265 270
   Thr Val Ser Trp Asn Ser Gly Ala Leu Thr Ser Gly Val His Thr Phe
   275 280 285
   Pro Ala Val Leu Gln Ser Ser Gly Leu Tyr Ser Leu Ser Ser Val Val
   290 295 300
   Thr Val Pro Ser Ser Ser Leu Gly Thr Gln Thr Tyr Ile Cys Asn Val
   305 310 315 320
   Asn His Lys Pro Ser Asn Thr Lys Val Asp Lys Arg Val Glu Pro Lys
   325 330 335
   Ser Cys Asp Lys Thr His Thr Cys Pro Pro Cys Pro Ala Pro Glu Leu
               340                 345                 350
   Leu Gly Gly Pro Ser Val Phe Leu Phe Pro Pro Lys Pro Lys Asp Thr
           355                 360                 365
   Leu Met Ile Ser Arg Thr Pro Glu Val Thr Cys Val Val Val Asp Val
       370                 375                 380
   Ser His Glu Asp Pro Glu Val Lys Phe Asn Trp Tyr Val Asp Gly Val
   385                 390                 395                 400
   Glu Val His Asn Ala Lys Thr Lys Pro Arg Glu Glu Gln Tyr Asn Ser
                   405                 410                 415
   Thr Tyr Arg Val Val Ser Val Leu Thr Val Leu His Gln Asp Trp Leu
               420                 425                 430
   Asn Gly Lys Glu Tyr Lys Cys Lys Val Ser Asn Lys Ala Leu Pro Ala
           435                 440                 445
   Pro Ile Glu Lys Thr Ile Ser Lys Ala Lys Gly Gln Pro Arg Glu Pro
       450                 455                 460
   Gln Val Tyr Thr Leu Pro Pro Ser Arg Glu Glu Met Thr Lys Asn Gln
   465                 470                 475                 480
   Val Ser Leu Thr Cys Leu Val Lys Gly Phe Tyr Pro Ser Asp Ile Ala
                   485                 490                 495
   Val Glu Trp Glu Ser Asn Gly Gln Pro Glu Asn Asn Tyr Lys Thr Thr
               500                 505                 510
   Pro Pro Val Leu Asp Ser Asp Gly Ser Phe Phe Leu Tyr Ser Lys Leu
           515                 520                 525
   Thr Val Asp Lys Ser Arg Trp Gln Gln Gly Asn Val Phe Ser Cys Ser
       530                 535                 540
   Val Met His Glu Ala Leu His Asn His Tyr Thr Gln Lys Ser Leu Ser
   545                 550                 555                 560
   Leu Ser Pro Gly Lys
                   565
<210> 14
   <211> 565
   <212> PRT
   <213> artificial
<220>
   <223> Fusion protein CH 139791
<400> 14
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Glu Glu Met Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Lys Leu Thr
               420                 425                 430
   Val Asp Lys Ser Arg Trp Gln Gln Gly Asn Val Phe Ser Cys Ser Val
           435                 440                 445
   Met His Glu Ala Leu His Asn His Tyr Thr Gln Lys Ser Leu Ser Leu
       450                 455                 460
   Ser Pro Gly Lys Gly Gly Gly Gly Ser Gly Gly Gly Gly Ser Gly Gly
   465                 470                 475                 480
   Gly Gly Ser Met Gln Ile Phe Val Lys Thr Leu Thr Pro Trp Arg Gly
                   485                 490                 495
   Tyr Asp Gly Lys Thr Ile Thr Leu Glu Val Glu Pro Ser Asp Thr Ile
               500                 505                 510
   Glu Asn Val Lys Ala Lys Ile Gln Asp Lys Glu Gly Ile Pro Pro Asp
           515                 520                 525
   Gln Gln Arg Leu Ile Trp Ala Gly Lys Gln Leu Glu Asp Gly Arg Thr
       530                 535                 540
   Leu Ser Asp Tyr Asn Ile Arg Arg Val Asp Val Leu His Leu Val Leu
   545                 550                 555                 560
   Arg Leu Arg Ala Ala
                   565
<210> 15
   <211> 638
   <212> PRT
   <213> artificial
<220>
   <223> Fusion protein CH 139864
<400> 15
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Glu Glu Met Thr Lys Asn Gln Val
   370 375 380
   Ser Leu Thr Cys Leu Val Lys Gly Phe Tyr Pro Ser Asp Ile Ala Val
   385 390 395 400
   Glu Trp Glu Ser Asn Gly Gln Pro Glu Asn Asn Tyr Lys Thr Thr Pro
   405 410 415
   Pro Val Leu Asp Ser Asp Gly Ser Phe Phe Leu Tyr Ser Lys Leu Thr
   420 425 430
   Val Asp Lys Ser Arg Trp Gln Gln Gly Asn Val Phe Ser Cys Ser Val
   435 440 445
   Met His Glu Ala Leu His Asn His Tyr Thr Gln Lys Ser Leu Ser Leu
   450 455 460
   Ser Pro Gly Lys Gly Gly Gly Gly Ser Gly Gly Gly Gly Ser Gly Gly
   465 470 475 480
   Gly Gly Ser Met Gln Ile Phe Val His Thr Pro Thr Gly Lys Thr Ile
   485 490 495
   Thr Leu Glu Val Glu Pro Ser Asp Thr Ile Glu Asn Val Lys Ala Lys
   500 505 510
   Ile Gln Asp Lys Glu Gly Ile Pro Pro Asp Gln Gln Arg Leu Ile Trp
   515 520 525
   Ala Gly Lys Gln Leu Glu Asp Gly Arg Thr Leu Ser Asp Tyr Asn Ile
   530 535 540
   Arg Asn Pro Asp Ile Leu His Leu Val Leu Arg Leu Arg Ala Ala Gly
   545 550 555 560
   Ile Gly Met Gln Ile Phe Val His Thr Met Thr Gly Lys Thr Ile Thr
   565 570 575
   Leu Glu Val Glu Pro Ser Asp Thr Ile Glu Asn Val Lys Ala Lys Ile
   580 585 590
   Gln Asp Lys Glu Gly Ile Pro Pro Asp Gln Gln Arg Leu Ile Trp Ala
   595 600 605
   Gly Lys Gln Leu Glu Asp Gly Arg Thr Leu Ser Asp Tyr Asn Ile Gly
   610 615 620
   Ala Gly Thr Met Leu His Leu Val Leu Arg Leu Arg Ala Ala
   625 630 635
<210> 16
   <211> 565
   <212> PRT
   <213> artificial
<220>
   <223> Fusion protein CH 139819
<400> 16
   Met Ala Val Leu Gly Leu Leu Phe Cys Leu Val Thr Phe Pro Ser Cys
   1 5 10 15
   Val Leu Ser Gln Val Gln Leu Lys Gln Ser Gly Pro Gly Leu Val Gln
   20 25 30
   Pro Ser Gln Ser Leu Ser Ile Thr Cys Thr Val Ser Gly Phe Ser Leu
   35 40 45
   Thr Asn Tyr Gly Val His Trp Val Arg Gln Ser Pro Gly Lys Gly Leu
   50 55 60
   Glu Trp Leu Gly Val Ile Trp Ser Gly Gly Asn Thr Asp Tyr Asn Thr
   65 70 75 80
   Pro Phe Thr Ser Arg Leu Ser Ile Asn Lys Asp Asn Ser Lys Ser Gln
   85 90 95
   Val Phe Phe Lys Met Asn Ser Leu Gln Ser Asn Asp Thr Ala Ile Tyr
   100 105 110
   Tyr Cys Ala Arg Ala Leu Thr Tyr Tyr Asp Tyr Glu Phe Ala Tyr Trp
   115 120 125
   Gly Gln Gly Thr Leu Val Thr Val Ser Ala Ala Ser Thr Lys Gly Pro
   130 135 140
   Ser Val Phe Pro Leu Ala Pro Ser Ser Lys Ser Thr Ser Gly Gly Thr
   145 150 155 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Glu Glu Met Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Lys Leu Thr
               420                 425                 430
   Val Asp Lys Ser Arg Trp Gln Gln Gly Asn Val Phe Ser Cys Ser Val
           435                 440                 445
   Met His Glu Ala Leu His Asn His Tyr Thr Gln Lys Ser Leu Ser Leu
   450 455 460
   Ser Pro Gly Lys Gly Gly Gly Gly Ser Gly Gly Gly Gly Ser Gly Gly
   465 470 475 480
   Gly Gly Ser Met Gln Ile Phe Val Lys Thr Leu Thr Tyr Asn Pro Met
   485 490 495
   Arg Tyr Gly Lys Thr Ile Thr Leu Glu Val Glu Pro Ser Asp Thr Ile
   500 505 510
   Glu Asn Val Lys Ala Lys Ile Gln Asp Lys Glu Gly Ile Pro Pro Asp
   515 520 525
   Gln Gln Arg Leu Ile Trp Ala Gly Lys Gln Leu Glu Asp Gly Arg Thr
   530 535 540
   Leu Ser Asp Tyr Asn Ile Arg Asn Pro Asp Ile Leu His Leu Val Leu
   545 550 555 560
   Arg Leu Arg Ala Ala
   565
<210> 17
   <211> 331
   <212> PRT
   <213> artificial
<220>
   <223> Fusion protein NL 139791
<400> 17
   Met Val Ser Thr Pro Gln Phe Leu Val Phe Leu Leu Phe Trp Ile Pro
   1 5 10 15
   Ala Ser Arg Ser Met Gln Ile Phe Val Lys Thr Leu Thr Pro Trp Arg
   20 25 30
   Gly Tyr Asp Gly Lys Thr Ile Thr Leu Glu Val Glu Pro Ser Asp Thr
   35 40 45
   Ile Glu Asn Val Lys Ala Lys Ile Gln Asp Lys Glu Gly Ile Pro Pro
   50 55 60
   Asp Gln Gln Arg Leu Ile Trp Ala Gly Lys Gln Leu Glu Asp Gly Arg
   65 70 75 80
   Thr Leu Ser Asp Tyr Asn Ile Arg Arg Val Asp Val Leu His Leu Val
                   85                  90                  95
   Leu Arg Leu Arg Ala Ala Gly Gly Gly Gly Ser Gly Gly Gly Gly Ser
               100                 105                 110
   Gly Gly Gly Gly Ser Asp Ile Leu Leu Thr Gln Ser Pro Val Ile Leu
           115                 120                 125
   Ser Val Ser Pro Gly Glu Arg Val Ser Phe Ser Cys Arg Ala Ser Gln
       130                 135                 140
   Ser Ile Gly Thr Asn Ile His Trp Tyr Gln Gln Arg Thr Asn Gly Ser
   145                 150                 155                 160
   Pro Arg Leu Leu Ile Lys Tyr Ala Ser Glu Ser Ile Ser Gly Ile Pro
                   165                 170                 175
   Ser Arg Phe Ser Gly Ser Gly Ser Gly Thr Asp Phe Thr Leu Ser Ile
               180                 185                 190
   Asn Ser Val Glu Ser Glu Asp Ile Ala Asp Tyr Tyr Cys Gln Gln Asn
           195                 200                 205
   Asn Asn Trp Pro Thr Thr Phe Gly Ala Gly Thr Lys Leu Glu Leu Lys
       210                 215                 220
   Arg Thr Val Ala Ala Pro Ser Val Phe Ile Phe Pro Pro Ser Asp Glu
   225                 230                 235                 240
   Gln Leu Lys Ser Gly Thr Ala Ser Val Val Cys Leu Leu Asn Asn Phe
                   245                 250                 255
   Tyr Pro Arg Glu Ala Lys Val Gln Trp Lys Val Asp Asn Ala Leu Gln
               260                 265                 270
   Ser Gly Asn Ser Gln Glu Ser Val Thr Glu Gln Asp Ser Lys Asp Ser
           275                 280                 285
   Thr Tyr Ser Leu Ser Ser Thr Leu Thr Leu Ser Lys Ala Asp Tyr Glu
       290                 295                 300
   Lys His Lys Val Tyr Ala Cys Glu Val Thr His Gln Gly Leu Ser Ser
   305                 310                 315                 320
   Pro Val Thr Lys Ser Phe Asn Arg Gly Glu Cys
                   325                 330
<210> 18
   <211> 404
   <212> PRT
   <213> artificial
<220>
   <223> Fusion protein NL 139864
<400> 18
   Met Val Ser Thr Pro Gln Phe Leu Val Phe Leu Leu Phe Trp Ile Pro
   1               5                   10                  15
   Ala Ser Arg Ser Met Gln Ile Phe Val His Thr Pro Thr Gly Lys Thr
               20                  25                  30
   Ile Thr Leu Glu Val Glu Pro Ser Asp Thr Ile Glu Asn Val Lys Ala
           35                  40                  45
   Lys Ile Gln Asp Lys Glu Gly Ile Pro Pro Asp Gln Gln Arg Leu Ile
       50                  55                  60
   Trp Ala Gly Lys Gln Leu Glu Asp Gly Arg Thr Leu Ser Asp Tyr Asn
   65                  70                  75                  80
   Ile Arg Asn Pro Asp Ile Leu His Leu Val Leu Arg Leu Arg Ala Ala
                   85                  90                  95
   Gly Ile Gly Met Gln Ile Phe Val His Thr Met Thr Gly Lys Thr Ile
               100                 105                 110
   Thr Leu Glu Val Glu Pro Ser Asp Thr Ile Glu Asn Val Lys Ala Lys
           115                 120                 125
   Ile Gln Asp Lys Glu Gly Ile Pro Pro Asp Gln Gln Arg Leu Ile Trp
       130                 135                 140
   Ala Gly Lys Gln Leu Glu Asp Gly Arg Thr Leu Ser Asp Tyr Asn Ile
   145                 150                 155                 160
   Gly Ala Gly Thr Met Leu His Leu Val Leu Arg Leu Arg Ala Ala Gly
                   165                 170                 175
   Gly Gly Gly Ser Gly Gly Gly Gly Ser Gly Gly Gly Gly Ser Asp Ile
               180                 185                 190
   Leu Leu Thr Gln Ser Pro Val Ile Leu Ser Val Ser Pro Gly Glu Arg
           195                 200                 205
   Val Ser Phe Ser Cys Arg Ala Ser Gln Ser Ile Gly Thr Asn Ile His
       210                 215                 220
   Trp Tyr Gln Gln Arg Thr Asn Gly Ser Pro Arg Leu Leu Ile Lys Tyr
   225                 230                 235                 240
   Ala Ser Glu Ser Ile Ser Gly Ile Pro Ser Arg Phe Ser Gly Ser Gly
                   245                 250                 255
   Ser Gly Thr Asp Phe Thr Leu Ser Ile Asn Ser Val Glu Ser Glu Asp
               260                 265                 270
   Ile Ala Asp Tyr Tyr Cys Gln Gln Asn Asn Asn Trp Pro Thr Thr Phe
           275                 280                 285
   Gly Ala Gly Thr Lys Leu Glu Leu Lys Arg Thr Val Ala Ala Pro Ser
       290                 295                 300
   Val Phe Ile Phe Pro Pro Ser Asp Glu Gln Leu Lys Ser Gly Thr Ala
   305                 310                 315                 320
   Ser Val Val Cys Leu Leu Asn Asn Phe Tyr Pro Arg Glu Ala Lys Val
                   325                 330                 335
   Gln Trp Lys Val Asp Asn Ala Leu Gln Ser Gly Asn Ser Gln Glu Ser
               340                 345                 350
   Val Thr Glu Gln Asp Ser Lys Asp Ser Thr Tyr Ser Leu Ser Ser Thr
           355                 360                 365
   Leu Thr Leu Ser Lys Ala Asp Tyr Glu Lys His Lys Val Tyr Ala Cys
       370                 375                 380
   Glu Val Thr His Gln Gly Leu Ser Ser Pro Val Thr Lys Ser Phe Asn
   385                 390                 395                 400
   Arg Gly Glu Cys
<210> 19
   <211> 331
   <212> PRT
   <213> artificial
<220>
   <223> Fusion protein NL 139819
<400> 19
   Met Val Ser Thr Pro Gln Phe Leu Val Phe Leu Leu Phe Trp Ile Pro
   1               5                   10                  15
   Ala Ser Arg Ser Met Gln Ile Phe Val Lys Thr Leu Thr Tyr Asn Pro
               20                  25                  30
   Met Arg Tyr Gly Lys Thr Ile Thr Leu Glu Val Glu Pro Ser Asp Thr
           35                  40                  45
   Ile Glu Asn Val Lys Ala Lys Ile Gln Asp Lys Glu Gly Ile Pro Pro
       50                  55                  60
   Asp Gln Gln Arg Leu Ile Trp Ala Gly Lys Gln Leu Glu Asp Gly Arg
   65                  70                  75                  80
   Thr Leu Ser Asp Tyr Asn Ile Arg Asn Pro Asp Ile Leu His Leu Val
                   85                  90                  95
   Leu Arg Leu Arg Ala Ala Gly Gly Gly Gly Ser Gly Gly Gly Gly Ser
               100                 105                 110
   Gly Gly Gly Gly Ser Asp Ile Leu Leu Thr Gln Ser Pro Val Ile Leu
           115                 120                 125
   Ser Val Ser Pro Gly Glu Arg Val Ser Phe Ser Cys Arg Ala Ser Gln
       130                 135                 140
   Ser Ile Gly Thr Asn Ile His Trp Tyr Gln Gln Arg Thr Asn Gly Ser
   145                 150                 155                 160
   Pro Arg Leu Leu Ile Lys Tyr Ala Ser Glu Ser Ile Ser Gly Ile Pro
                   165                 170                 175
   Ser Arg Phe Ser Gly Ser Gly Ser Gly Thr Asp Phe Thr Leu Ser Ile
               180                 185                 190
   Asn Ser Val Glu Ser Glu Asp Ile Ala Asp Tyr Tyr Cys Gln Gln Asn
           195                 200                 205
   Asn Asn Trp Pro Thr Thr Phe Gly Ala Gly Thr Lys Leu Glu Leu Lys
       210                 215                 220
   Arg Thr Val Ala Ala Pro Ser Val Phe Ile Phe Pro Pro Ser Asp Glu
   225                 230                 235                 240
   Gln Leu Lys Ser Gly Thr Ala Ser Val Val Cys Leu Leu Asn Asn Phe
                   245                 250                 255
   Tyr Pro Arg Glu Ala Lys Val Gln Trp Lys Val Asp Asn Ala Leu Gln
   260 265 270
   Ser Gly Asn Ser Gln Glu Ser Val Thr Glu Gln Asp Ser Lys Asp Ser
   275 280 285
   Thr Tyr Ser Leu Ser Ser Thr Leu Thr Leu Ser Lys Ala Asp Tyr Glu
   290 295 300
   Lys His Lys Val Tyr Ala Cys Glu Val Thr His Gln Gly Leu Ser Ser
   305 310 315 320
   Pro Val Thr Lys Ser Phe Asn Arg Gly Glu Cys
   325 330
<210> 20
   <211> 331
   <212> PRT
   <213> artificial
<220>
   <223> Fusion protein CL 139791
<400> 20
   Met Val Ser Thr Pro Gln Phe Leu Val Phe Leu Leu Phe Trp Ile Pro
   1 5 10 15
   Ala Ser Arg Ser Asp Ile Leu Leu Thr Gln Ser Pro Val Ile Leu Ser
   20 25 30
   Val Ser Pro Gly Glu Arg Val Ser Phe Ser Cys Arg Ala Ser Gln Ser
   35 40 45
   Ile Gly Thr Asn Ile His Trp Tyr Gln Gln Arg Thr Asn Gly Ser Pro
   50 55 60
   Arg Leu Leu Ile Lys Tyr Ala Ser Glu Ser Ile Ser Gly Ile Pro Ser
   65 70 75 80
   Arg Phe Ser Gly Ser Gly Ser Gly Thr Asp Phe Thr Leu Ser Ile Asn
   85 90 95
   Ser Val Glu Ser Glu Asp Ile Ala Asp Tyr Tyr Cys Gln Gln Asn Asn
   100 105 110
   Asn Trp Pro Thr Thr Phe Gly Ala Gly Thr Lys Leu Glu Leu Lys Arg
   115 120 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys Gly Gly Gly Gly Ser Gly
   225                 230                 235                 240
   Gly Gly Gly Ser Gly Gly Gly Gly Ser Met Gln Ile Phe Val Lys Thr
                   245                 250                 255
   Leu Thr Pro Trp Arg Gly Tyr Asp Gly Lys Thr Ile Thr Leu Glu Val
               260                 265                 270
   Glu Pro Ser Asp Thr Ile Glu Asn Val Lys Ala Lys Ile Gln Asp Lys
           275                 280                 285
   Glu Gly Ile Pro Pro Asp Gln Gln Arg Leu Ile Trp Ala Gly Lys Gln
       290                 295                 300
   Leu Glu Asp Gly Arg Thr Leu Ser Asp Tyr Asn Ile Arg Arg Val Asp
   305                 310                 315                 320
   Val Leu His Leu Val Leu Arg Leu Arg Ala Ala
                   325                 330
<210> 21
   <211> 404
   <212> PRT
   <213> artificial
<220>
   <223> Fusion protein CL 139864
<400> 21
   Met Val Ser Thr Pro Gln Phe Leu Val Phe Leu Leu Phe Trp Ile Pro
   1               5                   10                  15
   Ala Ser Arg Ser Asp Ile Leu Leu Thr Gln Ser Pro Val Ile Leu Ser
               20                  25                  30
   Val Ser Pro Gly Glu Arg Val Ser Phe Ser Cys Arg Ala Ser Gln Ser
           35                  40                  45
   Ile Gly Thr Asn Ile His Trp Tyr Gln Gln Arg Thr Asn Gly Ser Pro
       50                  55                  60
   Arg Leu Leu Ile Lys Tyr Ala Ser Glu Ser Ile Ser Gly Ile Pro Ser
   65                  70                  75                  80
   Arg Phe Ser Gly Ser Gly Ser Gly Thr Asp Phe Thr Leu Ser Ile Asn
                   85                  90                  95
   Ser Val Glu Ser Glu Asp Ile Ala Asp Tyr Tyr Cys Gln Gln Asn Asn
               100                 105                 110
   Asn Trp Pro Thr Thr Phe Gly Ala Gly Thr Lys Leu Glu Leu Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys Gly Gly Gly Gly Ser Gly
   225                 230                 235                 240
   Gly Gly Gly Ser Gly Gly Gly Gly Ser Met Gln Ile Phe Val His Thr
                   245                 250                 255
   Pro Thr Gly Lys Thr Ile Thr Leu Glu Val Glu Pro Ser Asp Thr Ile
               260                 265                 270
   Glu Asn Val Lys Ala Lys Ile Gln Asp Lys Glu Gly Ile Pro Pro Asp
           275                 280                 285
   Gln Gln Arg Leu Ile Trp Ala Gly Lys Gln Leu Glu Asp Gly Arg Thr
       290                 295                 300
   Leu Ser Asp Tyr Asn Ile Arg Asn Pro Asp Ile Leu His Leu Val Leu
   305                 310                 315                 320
   Arg Leu Arg Ala Ala Gly Ile Gly Met Gln Ile Phe Val His Thr Met
                   325                 330                 335
   Thr Gly Lys Thr Ile Thr Leu Glu Val Glu Pro Ser Asp Thr Ile Glu
               340                 345                 350
   Asn Val Lys Ala Lys Ile Gln Asp Lys Glu Gly Ile Pro Pro Asp Gln
           355                 360                 365
   Gln Arg Leu Ile Trp Ala Gly Lys Gln Leu Glu Asp Gly Arg Thr Leu
       370                 375                 380
   Ser Asp Tyr Asn Ile Gly Ala Gly Thr Met Leu His Leu Val Leu Arg
   385                 390                 395                 400
   Leu Arg Ala Ala
<210> 22
   <211> 331
   <212> PRT
   <213> artificial
<220>
   <223> Fusion protein CL 139819
<400> 22
   Met Val Ser Thr Pro Gln Phe Leu Val Phe Leu Leu Phe Trp Ile Pro
   1               5                   10                  15
   Ala Ser Arg Ser Asp Ile Leu Leu Thr Gln Ser Pro Val Ile Leu Ser
               20                  25                  30
   Val Ser Pro Gly Glu Arg Val Ser Phe Ser Cys Arg Ala Ser Gln Ser
           35                  40                  45
   Ile Gly Thr Asn Ile His Trp Tyr Gln Gln Arg Thr Asn Gly Ser Pro
   50 55 60
   Arg Leu Leu Ile Lys Tyr Ala Ser Glu Ser Ile Ser Gly Ile Pro Ser
   65 70 75 80
   Arg Phe Ser Gly Ser Gly Ser Gly Thr Asp Phe Thr Leu Ser Ile Asn
   85 90 95
   Ser Val Glu Ser Glu Asp Ile Ala Asp Tyr Tyr Cys Gln Gln Asn Asn
   100 105 110
   Asn Trp Pro Thr Thr Phe Gly Ala Gly Thr Lys Leu Glu Leu Lys Arg
   115 120 125
   Thr Val Ala Ala Pro Ser Val Phe Ile Phe Pro Pro Ser Asp Glu Gln
   130 135 140
   Leu Lys Ser Gly Thr Ala Ser Val Val Cys Leu Leu Asn Asn Phe Tyr
   145 150 155 160
   Pro Arg Glu Ala Lys Val Gln Trp Lys Val Asp Asn Ala Leu Gln Ser
   165 170 175
   Gly Asn Ser Gln Glu Ser Val Thr Glu Gln Asp Ser Lys Asp Ser Thr
   180 185 190
   Tyr Ser Leu Ser Ser Thr Leu Thr Leu Ser Lys Ala Asp Tyr Glu Lys
   195 200 205
   His Lys Val Tyr Ala Cys Glu Val Thr His Gln Gly Leu Ser Ser Pro
   210 215 220
   Val Thr Lys Ser Phe Asn Arg Gly Glu Cys Gly Gly Gly Gly Ser Gly
   225 230 235 240
   Gly Gly Gly Ser Gly Gly Gly Gly Ser Met Gln Ile Phe Val Lys Thr
   245 250 255
   Leu Thr Tyr Asn Pro Met Arg Tyr Gly Lys Thr Ile Thr Leu Glu Val
   260 265 270
   Glu Pro Ser Asp Thr Ile Glu Asn Val Lys Ala Lys Ile Gln Asp Lys
   275 280 285
   Glu Gly Ile Pro Pro Asp Gln Gln Arg Leu Ile Trp Ala Gly Lys Gln
   290 295 300
   Leu Glu Asp Gly Arg Thr Leu Ser Asp Tyr Asn Ile Arg Asn Pro Asp
   305 310 315 320
   Ile Leu His Leu Val Leu Arg Leu Arg Ala Ala
   325 330
<210> 23
   <211> 643
   <212> PRT
   <213> artificial
<220>
   <223> Fusion protein NH 141926
<400> 23
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Thr Leu Ala Phe
       50                  55                  60
   Ala Gly Lys Gln Leu Glu Asp Gly Arg Thr Leu Ser Asp Tyr Asn Ile
   65                  70                  75                  80
   Gln Lys Glu Ser Thr Leu Trp Leu Tyr Leu Thr Trp Tyr Ala Ala Met
                   85                  90                  95
   Arg Ile Phe Val Thr Thr His Thr Gly Lys Thr Ile Thr Leu Glu Val
               100                 105                 110
   Glu Pro Ser Asp Thr Ile Glu Asn Val Lys Ala Lys Ile Gln Asp Lys
           115                 120                 125
   Glu Gly Ile Pro Pro Asp Gln Gln Arg Leu Ile Trp Ala Gly Lys Gln
       130                 135                 140
   Leu Glu Asp Gly Arg Thr Leu Ser Asp Tyr Asn Ile Ser Glu Trp Ala
   145                 150                 155                 160
   Ile Leu His Leu Val Leu Arg Leu Arg Ala Ala Gly Gly Gly Gly Ser
                   165                 170                 175
   Gly Gly Gly Gly Ser Gly Gly Gly Gly Ser Gln Val Gln Leu Lys Gln
               180                 185                 190
   Ser Gly Pro Gly Leu Val Gln Pro Ser Gln Ser Leu Ser Ile Thr Cys
           195                 200                 205
   Thr Val Ser Gly Phe Ser Leu Thr Asn Tyr Gly Val His Trp Val Arg
       210                 215                 220
   Gln Ser Pro Gly Lys Gly Leu Glu Trp Leu Gly Val Ile Trp Ser Gly
   225                 230                 235                 240
   Gly Asn Thr Asp Tyr Asn Thr Pro Phe Thr Ser Arg Leu Ser Ile Asn
                   245                 250                 255
   Lys Asp Asn Ser Lys Ser Gln Val Phe Phe Lys Met Asn Ser Leu Gln
               260                 265                 270
   Ser Asn Asp Thr Ala Ile Tyr Tyr Cys Ala Arg Ala Leu Thr Tyr Tyr
           275                 280                 285
   Asp Tyr Glu Phe Ala Tyr Trp Gly Gln Gly Thr Leu Val Thr Val Ser
       290                 295                 300
   Ala Ala Ser Thr Lys Gly Pro Ser Val Phe Pro Leu Ala Pro Ser Ser
   305                 310                 315                 320
   Lys Ser Thr Ser Gly Gly Thr Ala Ala Leu Gly Cys Leu Val Lys Asp
                   325                 330                 335
   Tyr Phe Pro Glu Pro Val Thr Val Ser Trp Asn Ser Gly Ala Leu Thr
               340                 345                 350
   Ser Gly Val His Thr Phe Pro Ala Val Leu Gln Ser Ser Gly Leu Tyr
           355                 360                 365
   Ser Leu Ser Ser Val Val Thr Val Pro Ser Ser Ser Leu Gly Thr Gln
       370                 375                 380
   Thr Tyr Ile Cys Asn Val Asn His Lys Pro Ser Asn Thr Lys Val Asp
   385                 390                 395                 400
   Lys Arg Val Glu Pro Lys Ser Cys Asp Lys Thr His Thr Cys Pro Pro
                   405                 410                 415
   Cys Pro Ala Pro Glu Leu Leu Gly Gly Pro Ser Val Phe Leu Phe Pro
               420                 425                 430
   Pro Lys Pro Lys Asp Thr Leu Met Ile Ser Arg Thr Pro Glu Val Thr
   435 440 445
   Cys Val Val Val Asp Val Ser His Glu Asp Pro Glu Val Lys Phe Asn
   450 455 460
   Trp Tyr Val Asp Gly Val Glu Val His Asn Ala Lys Thr Lys Pro Arg
   465 470 475 480
   Glu Glu Gln Tyr Asn Ser Thr Tyr Arg Val Val Ser Val Leu Thr Val
   485 490 495
   Leu His Gln Asp Trp Leu Asn Gly Lys Glu Tyr Lys Cys Lys Val Ser
   500 505 510
   Asn Lys Ala Leu Pro Ala Pro Ile Glu Lys Thr Ile Ser Lys Ala Lys
   515 520 525
   Gly Gln Pro Arg Glu Pro Gln Val Tyr Thr Leu Pro Pro Ser Arg Glu
   530 535 540
   Glu Met Thr Lys Asn Gln Val Ser Leu Thr Cys Leu Val Lys Gly Phe
   545 550 555 560
   Tyr Pro Ser Asp Ile Ala Val Glu Trp Glu Ser Asn Gly Gln Pro Glu
   565 570 575
   Asn Asn Tyr Lys Thr Thr Pro Pro Val Leu Asp Ser Asp Gly Ser Phe
   580 585 590
   Phe Leu Tyr Ser Lys Leu Thr Val Asp Lys Ser Arg Trp Gln Gln Gly
   595 600 605
   Asn Val Phe Ser Cys Ser Val Met His Glu Ala Leu His Asn His Tyr
   610 615 620
   Thr Gln Lys Ser Leu Ser Leu Ser Pro Gly Lys Trp Ser His Pro Gln
   625 630 635 640
   Phe Glu Lys
<210> 24
   <211> 635
   <212> PRT
   <213> artificial
<220>
   <223> Fusion protein CH 141926
<400> 24
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
   245 250 255
   Gly Gly Pro Ser Val Phe Leu Phe Pro Pro Lys Pro Lys Asp Thr Leu
   260 265 270
   Met Ile Ser Arg Thr Pro Glu Val Thr Cys Val Val Val Asp Val Ser
   275 280 285
   His Glu Asp Pro Glu Val Lys Phe Asn Trp Tyr Val Asp Gly Val Glu
   290 295 300
   Val His Asn Ala Lys Thr Lys Pro Arg Glu Glu Gln Tyr Asn Ser Thr
   305 310 315 320
   Tyr Arg Val Val Ser Val Leu Thr Val Leu His Gln Asp Trp Leu Asn
   325 330 335
   Gly Lys Glu Tyr Lys Cys Lys Val Ser Asn Lys Ala Leu Pro Ala Pro
   340 345 350
   Ile Glu Lys Thr Ile Ser Lys Ala Lys Gly Gln Pro Arg Glu Pro Gln
   355 360 365
   Val Tyr Thr Leu Pro Pro Ser Arg Glu Glu Met Thr Lys Asn Gln Val
   370 375 380
   Ser Leu Thr Cys Leu Val Lys Gly Phe Tyr Pro Ser Asp Ile Ala Val
   385 390 395 400
   Glu Trp Glu Ser Asn Gly Gln Pro Glu Asn Asn Tyr Lys Thr Thr Pro
   405 410 415
   Pro Val Leu Asp Ser Asp Gly Ser Phe Phe Leu Tyr Ser Lys Leu Thr
   420 425 430
   Val Asp Lys Ser Arg Trp Gln Gln Gly Asn Val Phe Ser Cys Ser Val
   435 440 445
   Met His Glu Ala Leu His Asn His Tyr Thr Gln Lys Ser Leu Ser Leu
   450 455 460
   Ser Pro Gly Lys Gly Gly Gly Gly Ser Gly Gly Gly Gly Ser Gly Gly
   465 470 475 480
   Gly Gly Ser Met Gln Ile Phe Val Lys Thr Leu Thr Gly Lys Thr Ile
   485 490 495
   Thr Leu Glu Val Glu Pro Ser Asp Thr Ile Glu Asn Val Lys Ala Lys
               500                 505                 510
   Ile Gln Asp Lys Glu Gly Ile Pro Pro Asp Gln Gln Thr Leu Ala Phe
           515                 520                 525
   Ala Gly Lys Gln Leu Glu Asp Gly Arg Thr Leu Ser Asp Tyr Asn Ile
       530                 535                 540
   Gln Lys Glu Ser Thr Leu Trp Leu Tyr Leu Thr Trp Tyr Ala Ala Met
   545                 550                 555                 560
   Arg Ile Phe Val Thr Thr His Thr Gly Lys Thr Ile Thr Leu Glu Val
                   565                 570                 575
   Glu Pro Ser Asp Thr Ile Glu Asn Val Lys Ala Lys Ile Gln Asp Lys
               580                 585                 590
   Glu Gly Ile Pro Pro Asp Gln Gln Arg Leu Ile Trp Ala Gly Lys Gln
           595                 600                 605
   Leu Glu Asp Gly Arg Thr Leu Ser Asp Tyr Asn Ile Ser Glu Trp Ala
       610                 615                 620
   Ile Leu His Leu Val Leu Arg Leu Arg Ala Ala
   625                 630                 635
<210> 25
   <211> 401
   <212> PRT
   <213> artificial
<220>
   <223> Fusion protein NL 141926
<400> 25
   Met Val Ser Thr Pro Gln Phe Leu Val Phe Leu Leu Phe Trp Ile Pro
   1               5                   10                  15
   Ala Ser Arg Ser Met Gln Ile Phe Val Lys Thr Leu Thr Gly Lys Thr
               20                  25                  30
   Ile Thr Leu Glu Val Glu Pro Ser Asp Thr Ile Glu Asn Val Lys Ala
           35                  40                  45
   Lys Ile Gln Asp Lys Glu Gly Ile Pro Pro Asp Gln Gln Thr Leu Ala
       50                  55                  60
   Phe Ala Gly Lys Gln Leu Glu Asp Gly Arg Thr Leu Ser Asp Tyr Asn
   65                  70                  75                  80
   Ile Gln Lys Glu Ser Thr Leu Trp Leu Tyr Leu Thr Trp Tyr Ala Ala
                   85                  90                  95
   Met Arg Ile Phe Val Thr Thr His Thr Gly Lys Thr Ile Thr Leu Glu
               100                 105                 110
   Val Glu Pro Ser Asp Thr Ile Glu Asn Val Lys Ala Lys Ile Gln Asp
           115                 120                 125
   Lys Glu Gly Ile Pro Pro Asp Gln Gln Arg Leu Ile Trp Ala Gly Lys
       130                 135                 140
   Gln Leu Glu Asp Gly Arg Thr Leu Ser Asp Tyr Asn Ile Ser Glu Trp
   145                 150                 155                 160
   Ala Ile Leu His Leu Val Leu Arg Leu Arg Ala Ala Gly Gly Gly Gly
                   165                 170                 175
   Ser Gly Gly Gly Gly Ser Gly Gly Gly Gly Ser Asp Ile Leu Leu Thr
               180                 185                 190
   Gln Ser Pro Val Ile Leu Ser Val Ser Pro Gly Glu Arg Val Ser Phe
           195                 200                 205
   Ser Cys Arg Ala Ser Gln Ser Ile Gly Thr Asn Ile His Trp Tyr Gln
       210                 215                 220
   Gln Arg Thr Asn Gly Ser Pro Arg Leu Leu Ile Lys Tyr Ala Ser Glu
   225                 230                 235                 240
   Ser Ile Ser Gly Ile Pro Ser Arg Phe Ser Gly Ser Gly Ser Gly Thr
                   245                 250                 255
   Asp Phe Thr Leu Ser Ile Asn Ser Val Glu Ser Glu Asp Ile Ala Asp
               260                 265                 270
   Tyr Tyr Cys Gln Gln Asn Asn Asn Trp Pro Thr Thr Phe Gly Ala Gly
           275                 280                 285
   Thr Lys Leu Glu Leu Lys Arg Thr Val Ala Ala Pro Ser Val Phe Ile
       290                 295                 300
   Phe Pro Pro Ser Asp Glu Gln Leu Lys Ser Gly Thr Ala Ser Val Val
   305                 310                 315                 320
   Cys Leu Leu Asn Asn Phe Tyr Pro Arg Glu Ala Lys Val Gln Trp Lys
   325 330 335
   Val Asp Asn Ala Leu Gln Ser Gly Asn Ser Gln Glu Ser Val Thr Glu
   340 345 350
   Gln Asp Ser Lys Asp Ser Thr Tyr Ser Leu Ser Ser Thr Leu Thr Leu
   355 360 365
   Ser Lys Ala Asp Tyr Glu Lys His Lys Val Tyr Ala Cys Glu Val Thr
   370 375 380
   His Gln Gly Leu Ser Ser Pro Val Thr Lys Ser Phe Asn Arg Gly Glu
   385 390 395 400
   Cys
<210> 26
   <211> 401
   <212> PRT
   <213> artificial
<220>
   <223> Fusion protein CL 141926
<400> 26
   Met Val Ser Thr Pro Gln Phe Leu Val Phe Leu Leu Phe Trp Ile Pro
   1 5 10 15
   Ala Ser Arg Ser Asp Ile Leu Leu Thr Gln Ser Pro Val Ile Leu Ser
   20 25 30
   Val Ser Pro Gly Glu Arg Val Ser Phe Ser Cys Arg Ala Ser Gln Ser
   35 40 45
   Ile Gly Thr Asn Ile His Trp Tyr Gln Gln Arg Thr Asn Gly Ser Pro
   50 55 60
   Arg Leu Leu Ile Lys Tyr Ala Ser Glu Ser Ile Ser Gly Ile Pro Ser
   65 70 75 80
   Arg Phe Ser Gly Ser Gly Ser Gly Thr Asp Phe Thr Leu Ser Ile Asn
   85 90 95
   Ser Val Glu Ser Glu Asp Ile Ala Asp Tyr Tyr Cys Gln Gln Asn Asn
   100 105 110
   Asn Trp Pro Thr Thr Phe Gly Ala Gly Thr Lys Leu Glu Leu Lys Arg
   115 120 125
   Thr Val Ala Ala Pro Ser Val Phe Ile Phe Pro Pro Ser Asp Glu Gln
   130 135 140
   Leu Lys Ser Gly Thr Ala Ser Val Val Cys Leu Leu Asn Asn Phe Tyr
   145 150 155 160
   Pro Arg Glu Ala Lys Val Gln Trp Lys Val Asp Asn Ala Leu Gln Ser
   165 170 175
   Gly Asn Ser Gln Glu Ser Val Thr Glu Gln Asp Ser Lys Asp Ser Thr
   180 185 190
   Tyr Ser Leu Ser Ser Thr Leu Thr Leu Ser Lys Ala Asp Tyr Glu Lys
   195 200 205
   His Lys Val Tyr Ala Cys Glu Val Thr His Gln Gly Leu Ser Ser Pro
   210 215 220
   Val Thr Lys Ser Phe Asn Arg Gly Glu Cys Gly Gly Gly Gly Ser Gly
   225 230 235 240
   Gly Gly Gly Ser Gly Gly Gly Gly Ser Met Gln Ile Phe Val Lys Thr
   245 250 255
   Leu Thr Gly Lys Thr Ile Thr Leu Glu Val Glu Pro Ser Asp Thr Ile
   260 265 270
   Glu Asn Val Lys Ala Lys Ile Gln Asp Lys Glu Gly Ile Pro Pro Asp
   275 280 285
   Gln Gln Thr Leu Ala Phe Ala Gly Lys Gln Leu Glu Asp Gly Arg Thr
   290 295 300
   Leu Ser Asp Tyr Asn Ile Gln Lys Glu Ser Thr Leu Trp Leu Tyr Leu
   305 310 315 320
   Thr Trp Tyr Ala Ala Met Arg Ile Phe Val Thr Thr His Thr Gly Lys
   325 330 335
   Thr Ile Thr Leu Glu Val Glu Pro Ser Asp Thr Ile Glu Asn Val Lys
   340 345 350
   Ala Lys Ile Gln Asp Lys Glu Gly Ile Pro Pro Asp Gln Gln Arg Leu
   355 360 365
   Ile Trp Ala Gly Lys Gln Leu Glu Asp Gly Arg Thr Leu Ser Asp Tyr
       370                 375                 380
   Asn Ile Ser Glu Trp Ala Ile Leu His Leu Val Leu Arg Leu Arg Ala
   385                 390                 395                 400
   Ala
<210> 27
   <211> 559
   <212> PRT
   <213> artificial
<220>
   <223> Fusion protein CH ubiquitin
<400> 27
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Glu Glu Met Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Lys Leu Thr
   420 425 430
   Val Asp Lys Ser Arg Trp Gln Gln Gly Asn Val Phe Ser Cys Ser Val
   435 440 445
   Met His Glu Ala Leu His Asn His Tyr Thr Gln Lys Ser Leu Ser Leu
   450 455 460
   Ser Pro Gly Lys Gly Gly Gly Gly Ser Gly Gly Gly Gly Ser Gly Gly
   465 470 475 480
   Gly Gly Ser Met Gln Ile Phe Val Lys Thr Leu Thr Gly Lys Thr Ile
   485 490 495
   Thr Leu Glu Val Glu Pro Ser Asp Thr Ile Glu Asn Val Lys Ala Lys
   500 505 510
   Ile Gln Asp Lys Glu Gly Ile Pro Pro Asp Gln Gln Arg Leu Ile Trp
   515 520 525
   Ala Gly Lys Gln Leu Glu Asp Gly Arg Thr Leu Ser Asp Tyr Asn Ile
   530 535 540
   Gln Lys Glu Ser Thr Leu His Leu Val Leu Arg Leu Arg Ala Ala
   545 550 555
<210> 28
   <211> 325
   <212> PRT
   <213> artificial
<220>
   <223> Fusion protein CL ubiquitin
<400> 28
   Met Val Ser Thr Pro Gln Phe Leu Val Phe Leu Leu Phe Trp Ile Pro
   1 5 10 15
   Ala Ser Arg Ser Asp Ile Leu Leu Thr Gln Ser Pro Val Ile Leu Ser
   20 25 30
   Val Ser Pro Gly Glu Arg Val Ser Phe Ser Cys Arg Ala Ser Gln Ser
   35 40 45
   Ile Gly Thr Asn Ile His Trp Tyr Gln Gln Arg Thr Asn Gly Ser Pro
   50 55 60
   Arg Leu Leu Ile Lys Tyr Ala Ser Glu Ser Ile Ser Gly Ile Pro Ser
   65                  70                  75                  80
   Arg Phe Ser Gly Ser Gly Ser Gly Thr Asp Phe Thr Leu Ser Ile Asn
                   85                  90                  95
   Ser Val Glu Ser Glu Asp Ile Ala Asp Tyr Tyr Cys Gln Gln Asn Asn
               100                 105                 110
   Asn Trp Pro Thr Thr Phe Gly Ala Gly Thr Lys Leu Glu Leu Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys Gly Gly Gly Gly Ser Gly
   225                 230                 235                 240
   Gly Gly Gly Ser Gly Gly Gly Gly Ser Met Gln Ile Phe Val Lys Thr
                   245                 250                 255
   Leu Thr Gly Lys Thr Ile Thr Leu Glu Val Glu Pro Ser Asp Thr Ile
               260                 265                 270
   Glu Asn Val Lys Ala Lys Ile Gln Asp Lys Glu Gly Ile Pro Pro Asp
           275                 280                 285
   Gln Gln Arg Leu Ile Trp Ala Gly Lys Gln Leu Glu Asp Gly Arg Thr
       290                 295                 300
   Leu Ser Asp Tyr Asn Ile Gln Lys Glu Ser Thr Leu His Leu Val Leu
   305                 310                 315                 320
   Arg Leu Arg Ala Ala
   325
<210> 29
   <211> 559
   <212> PRT
   <213> artificial
<220>
   <223> Fusion protein NH ubiquitin
<400> 29
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Arg Leu Ile Trp
       50                  55                  60
   Ala Gly Lys Gln Leu Glu Asp Gly Arg Thr Leu Ser Asp Tyr Asn Ile
   65                  70                  75                  80
   Gln Lys Glu Ser Thr Leu His Leu Val Leu Arg Leu Arg Ala Ala Gly
                   85                  90                  95
   Gly Gly Gly Ser Gly Gly Gly Gly Ser Gly Gly Gly Gly Ser Gln Val
               100                 105                 110
   Gln Leu Lys Gln Ser Gly Pro Gly Leu Val Gln Pro Ser Gln Ser Leu
           115                 120                 125
   Ser Ile Thr Cys Thr Val Ser Gly Phe Ser Leu Thr Asn Tyr Gly Val
       130                 135                 140
   His Trp Val Arg Gln Ser Pro Gly Lys Gly Leu Glu Trp Leu Gly Val
   145                 150                 155                 160
   Ile Trp Ser Gly Gly Asn Thr Asp Tyr Asn Thr Pro Phe Thr Ser Arg
                   165                 170                 175
   Leu Ser Ile Asn Lys Asp Asn Ser Lys Ser Gln Val Phe Phe Lys Met
               180                 185                 190
   Asn Ser Leu Gln Ser Asn Asp Thr Ala Ile Tyr Tyr Cys Ala Arg Ala
           195                 200                 205
   Leu Thr Tyr Tyr Asp Tyr Glu Phe Ala Tyr Trp Gly Gln Gly Thr Leu
       210                 215                 220
   Val Thr Val Ser Ala Ala Ser Thr Lys Gly Pro Ser Val Phe Pro Leu
   225                 230                 235                 240
   Ala Pro Ser Ser Lys Ser Thr Ser Gly Gly Thr Ala Ala Leu Gly Cys
                   245                 250                 255
   Leu Val Lys Asp Tyr Phe Pro Glu Pro Val Thr Val Ser Trp Asn Ser
               260                 265                 270
   Gly Ala Leu Thr Ser Gly Val His Thr Phe Pro Ala Val Leu Gln Ser
           275                 280                 285
   Ser Gly Leu Tyr Ser Leu Ser Ser Val Val Thr Val Pro Ser Ser Ser
       290                 295                 300
   Leu Gly Thr Gln Thr Tyr Ile Cys Asn Val Asn His Lys Pro Ser Asn
   305                 310                 315                 320
   Thr Lys Val Asp Lys Arg Val Glu Pro Lys Ser Cys Asp Lys Thr His
                   325                 330                 335
   Thr Cys Pro Pro Cys Pro Ala Pro Glu Leu Leu Gly Gly Pro Ser Val
               340                 345                 350
   Phe Leu Phe Pro Pro Lys Pro Lys Asp Thr Leu Met Ile Ser Arg Thr
           355                 360                 365
   Pro Glu Val Thr Cys Val Val Val Asp Val Ser His Glu Asp Pro Glu
       370                 375                 380
   Val Lys Phe Asn Trp Tyr Val Asp Gly Val Glu Val His Asn Ala Lys
   385                 390                 395                 400
   Thr Lys Pro Arg Glu Glu Gln Tyr Asn Ser Thr Tyr Arg Val Val Ser
                   405                 410                 415
   Val Leu Thr Val Leu His Gln Asp Trp Leu Asn Gly Lys Glu Tyr Lys
               420                 425                 430
   Cys Lys Val Ser Asn Lys Ala Leu Pro Ala Pro Ile Glu Lys Thr Ile
           435                 440                 445
   Ser Lys Ala Lys Gly Gln Pro Arg Glu Pro Gln Val Tyr Thr Leu Pro
       450                 455                 460
   Pro Ser Arg Glu Glu Met Thr Lys Asn Gln Val Ser Leu Thr Cys Leu
   465                 470                 475                 480
   Val Lys Gly Phe Tyr Pro Ser Asp Ile Ala Val Glu Trp Glu Ser Asn
                   485                 490                 495
   Gly Gln Pro Glu Asn Asn Tyr Lys Thr Thr Pro Pro Val Leu Asp Ser
               500                 505                 510
   Asp Gly Ser Phe Phe Leu Tyr Ser Lys Leu Thr Val Asp Lys Ser Arg
           515                 520                 525
   Trp Gln Gln Gly Asn Val Phe Ser Cys Ser Val Met His Glu Ala Leu
       530                 535                 540
   His Asn His Tyr Thr Gln Lys Ser Leu Ser Leu Ser Pro Gly Lys
   545                 550                 555
<210> 30
   <211> 325
   <212> PRT
   <213> artificial
<220>
   <223> Fusion protein NL ubiquitin
<400> 30
   Met Val Ser Thr Pro Gln Phe Leu Val Phe Leu Leu Phe Trp Ile Pro
   1               5                   10                  15
   Ala Ser Arg Ser Met Gln Ile Phe Val Lys Thr Leu Thr Gly Lys Thr
               20                  25                  30
   Ile Thr Leu Glu Val Glu Pro Ser Asp Thr Ile Glu Asn Val Lys Ala
           35                  40                  45
   Lys Ile Gln Asp Lys Glu Gly Ile Pro Pro Asp Gln Gln Arg Leu Ile
       50                  55                  60
   Trp Ala Gly Lys Gln Leu Glu Asp Gly Arg Thr Leu Ser Asp Tyr Asn
   65                  70                  75                  80
   Ile Gln Lys Glu Ser Thr Leu His Leu Val Leu Arg Leu Arg Ala Ala
                   85                  90                  95
   Gly Gly Gly Gly Ser Gly Gly Gly Gly Ser Gly Gly Gly Gly Ser Asp
   100 105 110
   Ile Leu Leu Thr Gln Ser Pro Val Ile Leu Ser Val Ser Pro Gly Glu
   115 120 125
   Arg Val Ser Phe Ser Cys Arg Ala Ser Gln Ser Ile Gly Thr Asn Ile
   130 135 140
   His Trp Tyr Gln Gln Arg Thr Asn Gly Ser Pro Arg Leu Leu Ile Lys
   145 150 155 160
   Tyr Ala Ser Glu Ser Ile Ser Gly Ile Pro Ser Arg Phe Ser Gly Ser
   165 170 175
   Gly Ser Gly Thr Asp Phe Thr Leu Ser Ile Asn Ser Val Glu Ser Glu
   180 185 190
   Asp Ile Ala Asp Tyr Tyr Cys Gln Gln Asn Asn Asn Trp Pro Thr Thr
   195 200 205
   Phe Gly Ala Gly Thr Lys Leu Glu Leu Lys Arg Thr Val Ala Ala Pro
   210 215 220
   Ser Val Phe Ile Phe Pro Pro Ser Asp Glu Gln Leu Lys Ser Gly Thr
   225 230 235 240
   Ala Ser Val Val Cys Leu Leu Asn Asn Phe Tyr Pro Arg Glu Ala Lys
   245 250 255
   Val Gln Trp Lys Val Asp Asn Ala Leu Gln Ser Gly Asn Ser Gln Glu
   260 265 270
   Ser Val Thr Glu Gln Asp Ser Lys Asp Ser Thr Tyr Ser Leu Ser Ser
   275 280 285
   Thr Leu Thr Leu Ser Lys Ala Asp Tyr Glu Lys His Lys Val Tyr Ala
   290 295 300
   Cys Glu Val Thr His Gln Gly Leu Ser Ser Pro Val Thr Lys Ser Phe
   305 310 315 320
   Asn Arg Gly Glu Cys
   325
<210> 31
   <211> 15
   <212> PRT
   <213> artificial
<220>
   <223> Linker
<400> 31
   Gly Gly Gly Gly Ser Gly Gly Gly Gly Ser Gly Gly Gly Gly Ser
   1               5                   10                  15
<210> 32
   <211> 15
   <212> PRT
   <213> artificial
<220>
   <223> Linker
<400> 32
   Ser Gly Gly Gly Gly Ser Gly Gly Gly Gly Ser Gly Gly Gly Gly
   1               5                   10                  15
<210> 33
   <211> 8
   <212> PRT
   <213> artificial
<220>
   <223> Linker
<400> 33
   Gly Ile Gly Ser Gly Gly Gly Gly
   1               5
<210> 34
   <211> 6
   <212> PRT
   <213> artificial
<220>
   <223> Linker
<400> 34
   Gly Gly Gly Gly Ile Gly
   1               5
<210> 35
   <211> 12
   <212> PRT
   <213> artificial
<220>
   <223> Linker
<400> 35
   Ser Gly Gly Gly Gly Ser Gly Gly Gly Gly Ile Gly
   1 5 10
<210> 36
   <211> 10
   <212> PRT
   <213> artificial
<220>
   <223> Linker
<400> 36
   Ser Gly Gly Gly Gly Ser Gly Gly Gly Gly
   1 5 10
<210> 37
   <211> 12
   <212> PRT
   <213> artificial
<220>
   <223> Linker
<400> 37
   Gly Gly Gly Ser Gly Gly Gly Ser Gly Gly Gly Ser
   1 5 10
<210> 38
   <211> 7
   <212> PRT
   <213> artificial
<220>
   <223> Linker
<400> 38
   Ser Gly Gly Gly Gly Ile Gly
   1 5
<210> 39
   <211> 20
   <212> PRT
   <213> artificial
<220>
   <223> Linker
<400> 39
   Gly Gly Gly Gly Ser Gly Gly Gly Gly Ser Gly Gly Gly Gly Ser Gly
   1 5 10 15
   Gly Gly Gly Ser
   20
<210> 40
   <211> 155
   <212> PRT
   <213> artificial
<220>
   <223> Unmodified ubiquitin clone 64156
<400> 40
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Arg Leu Ile Trp Ala Gly Lys
   35 40 45
   Gln Leu Glu Asp Gly Arg Thr Leu Ser Asp Tyr Asn Ile Gln Lys Glu
   50 55 60
   Ser Thr Leu His Leu Val Leu Arg Leu Arg Ala Ala Gly Ile Gly Met
   65 70 75 80
   Gln Ile Phe Val Lys Thr Leu Thr Gly Lys Thr Ile Thr Leu Glu Val
   85 90 95
   Glu Pro Ser Asp Thr Ile Glu Asn Val Lys Ala Lys Ile Gln Asp Lys
   100 105 110
   Glu Gly Ile Pro Pro Asp Gln Gln Arg Leu Ile Trp Ala Gly Lys Gln
   115 120 125
   Leu Glu Asp Gly Arg Thr Leu Ser Asp Tyr Asn Ile Gln Lys Glu Ser
   130 135 140
   Thr Leu His Leu Val Leu Arg Leu Arg Ala Ala
   145 150 155
<210> 41
   <211> 19
   <212> PRT
   <213> artificial
<220>
   <223> Signal sequence
<400> 41
   Met Ala Val Leu Gly Leu Leu Phe Cys Leu Val Thr Phe Pro Ser Cys
   1 5 10 15
   Val Leu Ser
<210> 42
   <211> 20
   <212> PRT
   <213> artificial
<220>
   <223> Signal sequence
<400> 42
   Met Val Ser Thr Pro Gln Phe Leu Val Phe Leu Leu Phe Trp Ile Pro
   1 5 10 15
   Ala Ser Arg Ser
   20
<210> 43
   <211> 152
   <212> PRT
   <213> artificial
<220>
   <223> Affilin-128187 (clone 18)
<400> 43
   Met Gln Ile Phe Val Pro Thr Asn Thr Gly Arg Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Trp Ala Gly Lys
           35                  40                  45
   Gln Leu Glu Asp Gly Arg Thr Leu Ser Asp Tyr Asn Ile Asn Ala Pro
       50                  55                  60
   Ser Gln Leu His Leu Val Arg Arg Leu Arg Ala Ala Met Gln Ile Phe
   65                  70                  75                  80
   Val Lys Thr Gln Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Trp Phe Tyr Tyr Ser Leu His
       130                 135                 140
   Leu Val Leu Arg Leu Arg Ala Ala
   145                 150
<210> 44
   <211> 214
   <212> PRT
   <213> artificial
<220>
   <223> Light chain of CD3 binding Fab fragment 145-2C11
<400> 44
   Asp Ile Gln Met Thr Gln Ser Pro Ser Ser Leu Pro Ala Ser Leu Gly
   1               5                   10                  15
   Asp Arg Val Thr Ile Asn Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Asn Lys Leu Ala Asp Gly Val Pro Ser Arg Phe Ser Gly
       50                  55                  60
   Ser Gly Ser Gly Arg Asp Ser Ser Phe Thr Ile Ser Ser Leu Glu Ser
   65                  70                  75                  80
   Glu Asp Ile Gly Ser Tyr Tyr Cys Gln Gln Tyr Tyr Asn Tyr Pro Trp
                   85                  90                  95
   Thr Phe Gly Pro Gly Thr Lys Leu Glu Ile Lys Arg Ala Asp Ala Lys
               100                 105                 110
   Pro Thr Val Ser Ile Phe Pro Pro Ser Ser Glu Gln Leu Gly Thr Gly
           115                 120                 125
   Ser Ala Thr Leu Val Cys Phe Val Asn Asn Phe Tyr Pro Lys Asp Ile
       130                 135                 140
   Asn Val Lys Trp Lys Val Asp Gly Ser Glu Lys Arg Asp Gly Val Leu
   145                 150                 155                 160
   Gln Ser Val Thr Asp Gln Asp Ser Lys Asp Ser Thr Tyr Ser Leu Ser
                   165                 170                 175
   Ser Thr Leu Ser Leu Thr Lys Ala Asp Tyr Glu Arg His Asn Leu Tyr
               180                 185                 190
   Thr Cys Glu Val Thr His Lys Thr Ser Thr Ala Ala Ile Val Lys Thr
           195                 200                 205
   Leu Asn Arg Asn Glu Cys
       210
<210> 45
   <211> 216
   <212> PRT
   <213> artificial
<220>
   <223> Heavy chain of CD3 binding Fab fragment 145-2C11
<400> 45
   Glu Val Gln Leu Val Glu Ser Gly Gly Gly Leu Val Gln Pro Gly Lys
   1               5                   10                  15
   Ser Leu Lys Leu Ser Cys Glu Ala Ser Gly Phe Thr Phe Ser Gly Tyr
               20                  25                  30
   Gly Met His Trp Val Arg Gln Ala Pro Gly Arg Gly Leu Glu Ser Val
           35                  40                  45
   Ala Tyr Ile Thr Ser Ser Ser Ile Asn Ile Lys Tyr Ala Asp Ala Val
       50                  55                  60
   Lys Gly Arg Phe Thr Val Ser Arg Asp Asn Ala Lys Asn Leu Leu Phe
   65                  70                  75                  80
   Leu Gln Met Asn Ile Leu Lys Ser Glu Asp Thr Ala Met Tyr Tyr Cys
                   85                  90                  95
   Ala Arg Phe Asp Trp Asp Lys Asn Tyr Trp Gly Gln Gly Thr Met Val
               100                 105                 110
   Thr Val Ser Ser Ala Lys Thr Thr Ala Pro Ser Val Tyr Pro Leu Ala
           115                 120                 125
   Pro Ala Cys Asp Ser Thr Thr Ser Thr Thr Asn Thr Val Thr Leu Gly
       130                 135                 140
   Cys Leu Val Lys Gly Tyr Phe Pro Glu Pro Val Thr Val Ile Trp Asn
   145                 150                 155                 160
   Ser Gly Ala Leu Thr Ser Gly Val His Thr Phe Pro Ser Val Leu His
                   165                 170                 175
   Ser Gly Leu Tyr Ser Leu Ser Ser Ser Val Thr Val Pro Ser Ser Thr
               180                 185                 190
   Trp Pro Ser Gln Thr Val Thr Cys Asn Val Ala His Pro Ala Ser Ser
           195                 200                 205
   Thr Thr Val Asp Leu Lys Ile Glu
       210                 215
<210> 46
   <211> 417
   <212> PRT
   <213> artificial
<220>
   <223> Fusion protein Affilin (Affilin-128187) fused c-terminal to Light chain of CD3 binding Fab fragment 145-2C11
<220>
   <221> SITE
   <222> (235)..(254)
   <223> Xaa can be Gly, Ser, Ala or Pro
<400> 46
   Met Val Ser Thr Pro Gln Phe Leu Val Phe Leu Leu Phe Trp Ile Pro
   1               5                   10                  15
   Ala Ser Arg Ser Asp Ile Gln Met Thr Gln Ser Pro Ser Ser Leu Pro
               20                  25                  30
   Ala Ser Leu Gly Asp Arg Val Thr Ile Asn Cys Gln Ala Ser Gln Asp
           35                  40                  45
   Ile Ser Asn Tyr Leu Asn Trp Tyr Gln Gln Lys Pro Gly Lys Ala Pro
       50                  55                  60
   Lys Leu Leu Ile Tyr Tyr Thr Asn Lys Leu Ala Asp Gly Val Pro Ser
   65                  70                  75                  80
   Arg Phe Ser Gly Ser Gly Ser Gly Arg Asp Ser Ser Phe Thr Ile Ser
                   85                  90                  95
   Ser Leu Glu Ser Glu Asp Ile Gly Ser Tyr Tyr Cys Gln Gln Tyr Tyr
               100                 105                 110
   Asn Tyr Pro Trp Thr Phe Gly Pro Gly Thr Lys Leu Glu Ile Lys Arg
               115                 120                 125
   Ala Asp Ala Lys Pro Thr Val Ser Ile Phe Pro Pro Ser Ser Glu Gln
       130                 135                 140
   Leu Gly Thr Gly Ser Ala Thr Leu Val Cys Phe Val Asn Asn Phe Tyr
   145                 150                 155                 160
   Pro Lys Asp Ile Asn Val Lys Trp Lys Val Asp Gly Ser Glu Lys Arg
                   165                 170                 175
   Asp Gly Val Leu Gln Ser Val Thr Asp Gln Asp Ser Lys Asp Ser Thr
               180                 185                 190
   Tyr Ser Leu Ser Ser Thr Leu Ser Leu Thr Lys Ala Asp Tyr Glu Arg
           195                 200                 205
   His Asn Leu Tyr Thr Cys Glu Val Thr His Lys Thr Ser Thr Ala Ala
       210                 215                 220
   Ile Val Lys Thr Leu Asn Arg Asn Glu Cys Xaa Xaa Xaa Xaa Xaa Xaa
   225                 230                 235                 240
   Xaa Xaa Xaa Xaa Xaa Xaa Xaa Xaa Xaa Xaa Xaa Xaa Xaa Xaa Met Gln
                   245                 250                 255
   Ile Phe Val Pro Thr Asn Thr Gly Arg Thr Ile Thr Leu Glu Val Glu
               260                 265                 270
   Pro Ser Asp Thr Ile Glu Asn Val Lys Ala Lys Ile Gln Asp Lys Glu
           275                 280                 285
   Gly Ile Pro Pro Asp Gln Gln Arg Leu Ile Trp Ala Gly Lys Gln Leu
       290                 295                 300
   Glu Asp Gly Arg Thr Leu Ser Asp Tyr Asn Ile Asn Ala Pro Ser Gln
   305                 310                 315                 320
   Leu His Leu Val Arg Arg Leu Arg Ala Ala Met Gln Ile Phe Val Lys
                   325                 330                 335
   Thr Gln Thr Gly Lys Thr Ile Thr Leu Glu Val Glu Pro Ser Asp Thr
               340                 345                 350
   Ile Glu Asn Val Lys Ala Lys Ile Gln Asp Lys Glu Gly Ile Pro Pro
           355                 360                 365
   Asp Gln Gln Arg Leu Ile Trp Ala Gly Lys Gln Leu Glu Asp Gly Arg
   370 375 380
   Thr Leu Ser Asp Tyr Asn Ile Trp Phe Tyr Tyr Ser Leu His Leu Val
   385 390 395 400
   Leu Arg Leu Arg Ala Ala Gly Leu Ser Trp Ser His Pro Gln Phe Glu
   405 410 415
   Lys
<210> 47
   <211> 417
   <212> PRT
   <213> artificial
<220>
   <223> Fusion protein Affilin (Affilin-128187) fused N-terminal to Light chain of CD3 binding Fab fragment 145-2C11
<220>
   <221> SITE
   <222> (174)..(195)
   <223> Xaa can be Gly, Ser, Ala or Pro
<400> 47
   Met Val Ser Thr Pro Gln Phe Leu Val Phe Leu Leu Phe Trp Ile Pro
   1 5 10 15
   Ala Ser Arg Ser Gly Met Gln Ile Phe Val Pro Thr Asn Thr Gly Arg
   20 25 30
   Thr Ile Thr Leu Glu Val Glu Pro Ser Asp Thr Ile Glu Asn Val Lys
   35 40 45
   Ala Lys Ile Gln Asp Lys Glu Gly Ile Pro Pro Asp Gln Gln Arg Leu
   50 55 60
   Ile Trp Ala Gly Lys Gln Leu Glu Asp Gly Arg Thr Leu Ser Asp Tyr
   65 70 75 80
   Asn Ile Asn Ala Pro Ser Gln Leu His Leu Val Arg Arg Leu Arg Ala
   85 90 95
   Ala Met Gln Ile Phe Val Lys Thr Gln Thr Gly Lys Thr Ile Thr Leu
   100 105 110
   Glu Val Glu Pro Ser Asp Thr Ile Glu Asn Val Lys Ala Lys Ile Gln
   115 120 125
   Asp Lys Glu Gly Ile Pro Pro Asp Gln Gln Arg Leu Ile Trp Ala Gly
       130                 135                 140
   Lys Gln Leu Glu Asp Gly Arg Thr Leu Ser Asp Tyr Asn Ile Trp Phe
   145                 150                 155                 160
   Tyr Tyr Ser Leu His Leu Val Leu Arg Leu Arg Ala Ala Xaa Xaa Xaa
                   165                 170                 175
   Xaa Xaa Xaa Xaa Xaa Xaa Xaa Xaa Xaa Xaa Xaa Xaa Xaa Xaa Xaa Xaa
               180                 185                 190
   Xaa Xaa Xaa Asp Ile Gln Met Thr Gln Ser Pro Ser Ser Leu Pro Ala
           195                 200                 205
   Ser Leu Gly Asp Arg Val Thr Ile Asn Cys Gln Ala Ser Gln Asp Ile
       210                 215                 220
   Ser Asn Tyr Leu Asn Trp Tyr Gln Gln Lys Pro Gly Lys Ala Pro Lys
   225                 230                 235                 240
   Leu Leu Ile Tyr Tyr Thr Asn Lys Leu Ala Asp Gly Val Pro Ser Arg
                   245                 250                 255
   Phe Ser Gly Ser Gly Ser Gly Arg Asp Ser Ser Phe Thr Ile Ser Ser
               260                 265                 270
   Leu Glu Ser Glu Asp Ile Gly Ser Tyr Tyr Cys Gln Gln Tyr Tyr Asn
           275                 280                 285
   Tyr Pro Trp Thr Phe Gly Pro Gly Thr Lys Leu Glu Ile Lys Arg Ala
       290                 295                 300
   Asp Ala Lys Pro Thr Val Ser Ile Phe Pro Pro Ser Ser Glu Gln Leu
   305                 310                 315                 320
   Gly Thr Gly Ser Ala Thr Leu Val Cys Phe Val Asn Asn Phe Tyr Pro
                   325                 330                 335
   Lys Asp Ile Asn Val Lys Trp Lys Val Asp Gly Ser Glu Lys Arg Asp
               340                 345                 350
   Gly Val Leu Gln Ser Val Thr Asp Gln Asp Ser Lys Asp Ser Thr Tyr
           355                 360                 365
   Ser Leu Ser Ser Thr Leu Ser Leu Thr Lys Ala Asp Tyr Glu Arg His
   370 375 380
   Asn Leu Tyr Thr Cys Glu Val Thr His Lys Thr Ser Thr Ala Ala Ile
   385 390 395 400
   Val Lys Thr Leu Asn Arg Asn Glu Cys Trp Ser His Pro Gln Phe Glu
   405 410 415
   Lys
<210> 48
   <211> 407
   <212> PRT
   <213> artificial
<220>
   <223> Fusion protein Affilin (Affilin-128187) fused C-terminal to heavy chain of CD3 binding Fab fragment 145-2C11
<220>
   <221> SITE
   <222> (236)..(255)
   <223> Xaa can be Gly, Ser, Ala or Pro
<400> 48
   Met Ala Val Leu Gly Leu Leu Phe Cys Leu Val Thr Phe Pro Ser Cys
   1 5 10 15
   Val Leu Ser Glu Val Gln Leu Val Glu Ser Gly Gly Gly Leu Val Gln
   20 25 30
   Pro Gly Lys Ser Leu Lys Leu Ser Cys Glu Ala Ser Gly Phe Thr Phe
   35 40 45
   Ser Gly Tyr Gly Met His Trp Val Arg Gln Ala Pro Gly Arg Gly Leu
   50 55 60
   Glu Ser Val Ala Tyr Ile Thr Ser Ser Ser Ile Asn Ile Lys Tyr Ala
   65 70 75 80
   Asp Ala Val Lys Gly Arg Phe Thr Val Ser Arg Asp Asn Ala Lys Asn
   85 90 95
   Leu Leu Phe Leu Gln Met Asn Ile Leu Lys Ser Glu Asp Thr Ala Met
   100 105 110
   Tyr Tyr Cys Ala Arg Phe Asp Trp Asp Lys Asn Tyr Trp Gly Gln Gly
   115 120 125
   Thr Met Val Thr Val Ser Ser Ala Lys Thr Thr Ala Pro Ser Val Tyr
       130                 135                 140
   Pro Leu Ala Pro Ala Cys Asp Ser Thr Thr Ser Thr Thr Asn Thr Val
   145                 150                 155                 160
   Thr Leu Gly Cys Leu Val Lys Gly Tyr Phe Pro Glu Pro Val Thr Val
                   165                 170                 175
   Ile Trp Asn Ser Gly Ala Leu Thr Ser Gly Val His Thr Phe Pro Ser
               180                 185                 190
   Val Leu His Ser Gly Leu Tyr Ser Leu Ser Ser Ser Val Thr Val Pro
           195                 200                 205
   Ser Ser Thr Trp Pro Ser Gln Thr Val Thr Cys Asn Val Ala His Pro
       210                 215                 220
   Ala Ser Ser Thr Thr Val Asp Leu Lys Ile Glu Xaa Xaa Xaa Xaa Xaa
   225                 230                 235                 240
   Xaa Xaa Xaa Xaa Xaa Xaa Xaa Xaa Xaa Xaa Xaa Xaa Xaa Xaa Xaa Met
                   245                 250                 255
   Gln Ile Phe Val Pro Thr Asn Thr Gly Arg Thr Ile Thr Leu Glu Val
               260                 265                 270
   Glu Pro Ser Asp Thr Ile Glu Asn Val Lys Ala Lys Ile Gln Asp Lys
           275                 280                 285
   Glu Gly Ile Pro Pro Asp Gln Gln Arg Leu Ile Trp Ala Gly Lys Gln
       290                 295                 300
   Leu Glu Asp Gly Arg Thr Leu Ser Asp Tyr Asn Ile Asn Ala Pro Ser
   305                 310                 315                 320
   Gln Leu His Leu Val Arg Arg Leu Arg Ala Ala Met Gln Ile Phe Val
                   325                 330                 335
   Lys Thr Gln Thr Gly Lys Thr Ile Thr Leu Glu Val Glu Pro Ser Asp
               340                 345                 350
   Thr Ile Glu Asn Val Lys Ala Lys Ile Gln Asp Lys Glu Gly Ile Pro
           355                 360                 365
   Pro Asp Gln Gln Arg Leu Ile Trp Ala Gly Lys Gln Leu Glu Asp Gly
       370                 375                 380
   Arg Thr Leu Ser Asp Tyr Asn Ile Trp Phe Tyr Tyr Ser Leu His Leu
   385                 390                 395                 400
   Val Leu Arg Leu Arg Ala Ala
                   405
<210> 49
   <211> 411
   <212> PRT
   <213> artificial
<220>
   <223> Fusion protein Affilin (Affilin-128187) fused N-terminal to heavy chain of CD3 binding Fab fragment 145-2C11
<220>
   <221> SITE
   <222> (173)..(195)
   <223> Xaa can be Gly, Ser, Ala or Pro
<400> 49
   Met Ala Val Leu Gly Leu Leu Phe Cys Leu Val Thr Phe Pro Ser Cys
   1               5                   10                  15
   Val Leu Ser Gly Met Gln Ile Phe Val Pro Thr Asn Thr Gly Arg Thr
               20                  25                  30
   Ile Thr Leu Glu Val Glu Pro Ser Asp Thr Ile Glu Asn Val Lys Ala
           35                  40                  45
   Lys Ile Gln Asp Lys Glu Gly Ile Pro Pro Asp Gln Gln Arg Leu Ile
       50                  55                  60
   Trp Ala Gly Lys Gln Leu Glu Asp Gly Arg Thr Leu Ser Asp Tyr Asn
   65                  70                  75                  80
   Ile Asn Ala Pro Ser Gln Leu His Leu Val Arg Arg Leu Arg Ala Ala
                   85                  90                  95
   Met Gln Ile Phe Val Lys Thr Gln Thr Gly Lys Thr Ile Thr Leu Glu
               100                 105                 110
   Val Glu Pro Ser Asp Thr Ile Glu Asn Val Lys Ala Lys Ile Gln Asp
           115                 120                 125
   Lys Glu Gly Ile Pro Pro Asp Gln Gln Arg Leu Ile Trp Ala Gly Lys
       130                 135                 140
   Gln Leu Glu Asp Gly Arg Thr Leu Ser Asp Tyr Asn Ile Trp Phe Tyr
   145                 150                 155                 160
   Tyr Ser Leu His Leu Val Leu Arg Leu Arg Ala Ala Xaa Xaa Xaa Xaa
                   165                 170                 175
   Xaa Xaa Xaa Xaa Xaa Xaa Xaa Xaa Xaa Xaa Xaa Xaa Xaa Xaa Xaa Xaa
               180                 185                 190
   Xaa Xaa Xaa Glu Val Gln Leu Val Glu Ser Gly Gly Gly Leu Val Gln
           195                 200                 205
   Pro Gly Lys Ser Leu Lys Leu Ser Cys Glu Ala Ser Gly Phe Thr Phe
       210                 215                 220
   Ser Gly Tyr Gly Met His Trp Val Arg Gln Ala Pro Gly Arg Gly Leu
   225                 230                 235                 240
   Glu Ser Val Ala Tyr Ile Thr Ser Ser Ser Ile Asn Ile Lys Tyr Ala
                   245                 250                 255
   Asp Ala Val Lys Gly Arg Phe Thr Val Ser Arg Asp Asn Ala Lys Asn
               260                 265                 270
   Leu Leu Phe Leu Gln Met Asn Ile Leu Lys Ser Glu Asp Thr Ala Met
           275                 280                 285
   Tyr Tyr Cys Ala Arg Phe Asp Trp Asp Lys Asn Tyr Trp Gly Gln Gly
       290                 295                 300
   Thr Met Val Thr Val Ser Ser Ala Lys Thr Thr Ala Pro Ser Val Tyr
   305                 310                 315                 320
   Pro Leu Ala Pro Ala Cys Asp Ser Thr Thr Ser Thr Thr Asn Thr Val
                   325                 330                 335
   Thr Leu Gly Cys Leu Val Lys Gly Tyr Phe Pro Glu Pro Val Thr Val
               340                 345                 350
   Ile Trp Asn Ser Gly Ala Leu Thr Ser Gly Val His Thr Phe Pro Ser
           355                 360                 365
   Val Leu His Ser Gly Leu Tyr Ser Leu Ser Ser Ser Val Thr Val Pro
       370                 375                 380
   Ser Ser Thr Trp Pro Ser Gln Thr Val Thr Cys Asn Val Ala His Pro
   385                 390                 395                 400
   Ala Ser Ser Thr Thr Val Asp Leu Lys Ile Glu
   405 410
<210> 50
   <211> 6
   <212> PRT
   <213> artificial
<220>
   <223> His-Tag-Sequence
<400> 50
   His His His His His His
   1 5
<210> 51
   <211> 8
   <212> PRT
   <213> Artificial Sequence
<220>
   <223> StrepTag
<400> 51
   Trp Ser His Pro Gln Phe Glu Lys
   1 5
<210> 52
   <211> 253
   <212> PRT
   <213> Artificial Sequence
<220>
   <223> Cetuximab Fab fragment heavy chain
<400> 52
   Met Ala Val Leu Gly Leu Leu Phe Cys Leu Val Thr Phe Pro Ser Cys
   1 5 10 15
   Val Leu Ser Gln Val Gln Leu Lys Gln Ser Gly Pro Gly Leu Val Gln
   20 25 30
   Pro Ser Gln Ser Leu Ser Ile Thr Cys Thr Val Ser Gly Phe Ser Leu
   35 40 45
   Thr Asn Tyr Gly Val His Trp Val Arg Gln Ser Pro Gly Lys Gly Leu
   50 55 60
   Glu Trp Leu Gly Val Ile Trp Ser Gly Gly Asn Thr Asp Tyr Asn Thr
   65 70 75 80
   Pro Phe Thr Ser Arg Leu Ser Ile Asn Lys Asp Asn Ser Lys Ser Gln
   85 90 95
   Val Phe Phe Lys Met Asn Ser Leu Gln Ser Asn Asp Thr Ala Ile Tyr
   100 105 110
   Tyr Cys Ala Arg Ala Leu Thr Tyr Tyr Asp Tyr Glu Phe Ala Tyr Trp
   115 120 125
   Gly Gln Gly Thr Leu Val Thr Val Ser Ala Ala Ser Thr Lys Gly Pro
   130 135 140
   Ser Val Phe Pro Leu Ala Pro Ser Ser Lys Ser Thr Ser Gly Gly Thr
   145 150 155 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
   195 200 205
   Val Pro Ser Ser Ser Leu Gly Thr Gln Thr Tyr Ile Cys Asn Val Asn
   210 215 220
   His Lys Pro Ser Asn Thr Lys Val Asp Lys Arg Val Glu Pro Lys Ser
   225 230 235 240
   Cys Asp Lys Thr His Trp Ser His Pro Gln Phe Glu Lys
   245 250
<210> 53
   <211> 420
   <212> PRT
   <213> Artificial Sequence
<220>
   <223> Cetuximab Fab NH 141926
<400> 53
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Thr Leu Ala Phe
       50                  55                  60
   Ala Gly Lys Gln Leu Glu Asp Gly Arg Thr Leu Ser Asp Tyr Asn Ile
   65                  70                  75                  80
   Gln Lys Glu Ser Thr Leu Trp Leu Tyr Leu Thr Trp Tyr Ala Ala Met
                   85                  90                  95
   Arg Ile Phe Val Thr Thr His Thr Gly Lys Thr Ile Thr Leu Glu Val
               100                 105                 110
   Glu Pro Ser Asp Thr Ile Glu Asn Val Lys Ala Lys Ile Gln Asp Lys
           115                 120                 125
   Glu Gly Ile Pro Pro Asp Gln Gln Arg Leu Ile Trp Ala Gly Lys Gln
       130                 135                 140
   Leu Glu Asp Gly Arg Thr Leu Ser Asp Tyr Asn Ile Ser Glu Trp Ala
   145                 150                 155                 160
   Ile Leu His Leu Val Leu Arg Leu Arg Ala Ala Gly Gly Gly Gly Ser
                   165                 170                 175
   Gly Gly Gly Gly Ser Gly Gly Gly Gly Ser Gln Val Gln Leu Lys Gln
               180                 185                 190
   Ser Gly Pro Gly Leu Val Gln Pro Ser Gln Ser Leu Ser Ile Thr Cys
           195                 200                 205
   Thr Val Ser Gly Phe Ser Leu Thr Asn Tyr Gly Val His Trp Val Arg
       210                 215                 220
   Gln Ser Pro Gly Lys Gly Leu Glu Trp Leu Gly Val Ile Trp Ser Gly
   225                 230                 235                 240
   Gly Asn Thr Asp Tyr Asn Thr Pro Phe Thr Ser Arg Leu Ser Ile Asn
                   245                 250                 255
   Lys Asp Asn Ser Lys Ser Gln Val Phe Phe Lys Met Asn Ser Leu Gln
               260                 265                 270
   Ser Asn Asp Thr Ala Ile Tyr Tyr Cys Ala Arg Ala Leu Thr Tyr Tyr
           275                 280                 285
   Asp Tyr Glu Phe Ala Tyr Trp Gly Gln Gly Thr Leu Val Thr Val Ser
       290                 295                 300
   Ala Ala Ser Thr Lys Gly Pro Ser Val Phe Pro Leu Ala Pro Ser Ser
   305                 310                 315                 320
   Lys Ser Thr Ser Gly Gly Thr Ala Ala Leu Gly Cys Leu Val Lys Asp
                   325                 330                 335
   Tyr Phe Pro Glu Pro Val Thr Val Ser Trp Asn Ser Gly Ala Leu Thr
               340                 345                 350
   Ser Gly Val His Thr Phe Pro Ala Val Leu Gln Ser Ser Gly Leu Tyr
           355                 360                 365
   Ser Leu Ser Ser Val Val Thr Val Pro Ser Ser Ser Leu Gly Thr Gln
       370                 375                 380
   Thr Tyr Ile Cys Asn Val Asn His Lys Pro Ser Asn Thr Lys Val Asp
   385                 390                 395                 400
   Lys Arg Val Glu Pro Lys Ser Cys Asp Lys Thr His Trp Ser His Pro
                   405                 410                 415
   Gln Phe Glu Lys
               420
<210> 54
   <211> 420
   <212> PRT
   <213> Artificial Sequence
<220>
   <223> Cetuximab Fab CH 141926
<400> 54
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Gly Gly Gly Gly Ser Gly Gly Gly Gly Ser Gly
                   245                 250                 255
   Gly Gly Gly Ser Met Gln Ile Phe Val Lys Thr Leu Thr Gly Lys Thr
               260                 265                 270
   Ile Thr Leu Glu Val Glu Pro Ser Asp Thr Ile Glu Asn Val Lys Ala
           275                 280                 285
   Lys Ile Gln Asp Lys Glu Gly Ile Pro Pro Asp Gln Gln Thr Leu Ala
       290                 295                 300
   Phe Ala Gly Lys Gln Leu Glu Asp Gly Arg Thr Leu Ser Asp Tyr Asn
   305                 310                 315                 320
   Ile Gln Lys Glu Ser Thr Leu Trp Leu Tyr Leu Thr Trp Tyr Ala Ala
                   325                 330                 335
   Met Arg Ile Phe Val Thr Thr His Thr Gly Lys Thr Ile Thr Leu Glu
               340                 345                 350
   Val Glu Pro Ser Asp Thr Ile Glu Asn Val Lys Ala Lys Ile Gln Asp
           355                 360                 365
   Lys Glu Gly Ile Pro Pro Asp Gln Gln Arg Leu Ile Trp Ala Gly Lys
       370                 375                 380
   Gln Leu Glu Asp Gly Arg Thr Leu Ser Asp Tyr Asn Ile Ser Glu Trp
   385                 390                 395                 400
   Ala Ile Leu His Leu Val Leu Arg Leu Arg Ala Ala Trp Ser His Pro
                   405                 410                 415
   Gln Phe Glu Lys
               420
<210> 55
   <211> 350
   <212> PRT
   <213> Artificial Sequence
<220>
   <223> Cetuximab Fab NH 139819
<400> 55
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Tyr Asn Pro Met
               20                  25                  30
   Arg Tyr Gly Lys Thr Ile Thr Leu Glu Val Glu Pro Ser Asp Thr Ile
           35                  40                  45
   Glu Asn Val Lys Ala Lys Ile Gln Asp Lys Glu Gly Ile Pro Pro Asp
       50                  55                  60
   Gln Gln Arg Leu Ile Trp Ala Gly Lys Gln Leu Glu Asp Gly Arg Thr
   65                  70                  75                  80
   Leu Ser Asp Tyr Asn Ile Arg Asn Pro Asp Ile Leu His Leu Val Leu
                   85                  90                  95
   Arg Leu Arg Ala Ala Gly Gly Gly Gly Ser Gly Gly Gly Gly Ser Gly
               100                 105                 110
   Gly Gly Gly Ser Gln Val Gln Leu Lys Gln Ser Gly Pro Gly Leu Val
           115                 120                 125
   Gln Pro Ser Gln Ser Leu Ser Ile Thr Cys Thr Val Ser Gly Phe Ser
       130                 135                 140
   Leu Thr Asn Tyr Gly Val His Trp Val Arg Gln Ser Pro Gly Lys Gly
   145                 150                 155                 160
   Leu Glu Trp Leu Gly Val Ile Trp Ser Gly Gly Asn Thr Asp Tyr Asn
                   165                 170                 175
   Thr Pro Phe Thr Ser Arg Leu Ser Ile Asn Lys Asp Asn Ser Lys Ser
               180                 185                 190
   Gln Val Phe Phe Lys Met Asn Ser Leu Gln Ser Asn Asp Thr Ala Ile
           195                 200                 205
   Tyr Tyr Cys Ala Arg Ala Leu Thr Tyr Tyr Asp Tyr Glu Phe Ala Tyr
       210                 215                 220
   Trp Gly Gln Gly Thr Leu Val Thr Val Ser Ala Ala Ser Thr Lys Gly
   225                 230                 235                 240
   Pro Ser Val Phe Pro Leu Ala Pro Ser Ser Lys Ser Thr Ser Gly Gly
                   245                 250                 255
   Thr Ala Ala Leu Gly Cys Leu Val Lys Asp Tyr Phe Pro Glu Pro Val
               260                 265                 270
   Thr Val Ser Trp Asn Ser Gly Ala Leu Thr Ser Gly Val His Thr Phe
           275                 280                 285
   Pro Ala Val Leu Gln Ser Ser Gly Leu Tyr Ser Leu Ser Ser Val Val
       290                 295                 300
   Thr Val Pro Ser Ser Ser Leu Gly Thr Gln Thr Tyr Ile Cys Asn Val
   305                 310                 315                 320
   Asn His Lys Pro Ser Asn Thr Lys Val Asp Lys Arg Val Glu Pro Lys
                   325                 330                 335
   Ser Cys Asp Lys Thr His Trp Ser His Pro Gln Phe Glu Lys
               340                 345                 350
<210> 56
   <211> 350
   <212> PRT
   <213> Artificial Sequence
<220>
   <223> Cetuximab Fab CH 139819
<400> 56
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225 230 235 240
   Cys Asp Lys Thr His Gly Gly Gly Gly Ser Gly Gly Gly Gly Ser Gly
   245 250 255
   Gly Gly Gly Ser Met Gln Ile Phe Val Lys Thr Leu Thr Tyr Asn Pro
   260 265 270
   Met Arg Tyr Gly Lys Thr Ile Thr Leu Glu Val Glu Pro Ser Asp Thr
   275 280 285
   Ile Glu Asn Val Lys Ala Lys Ile Gln Asp Lys Glu Gly Ile Pro Pro
   290 295 300
   Asp Gln Gln Arg Leu Ile Trp Ala Gly Lys Gln Leu Glu Asp Gly Arg
   305 310 315 320
   Thr Leu Ser Asp Tyr Asn Ile Arg Asn Pro Asp Ile Leu His Leu Val
   325 330 335
   Leu Arg Leu Arg Ala Ala Trp Ser His Pro Gln Phe Glu Lys
   340 345 350
<210> 57
   <211> 420
   <212> PRT
   <213> Artificial Sequence
<220>
   <223> Cetuximab Fab NH ubiquitin (dimer 139090)
<400> 57
   Met Ala Val Leu Gly Leu Leu Phe Cys Leu Val Thr Phe Pro Ser Cys
   1 5 10 15
   Val Leu Ser Met Gln Ile Phe Val Lys Thr Leu Thr Gly Lys Thr Ile
   20 25 30
   Thr Leu Glu Val Glu Pro Ser Asp Thr Ile Glu Asn Val Lys Ala Lys
   35 40 45
   Ile Gln Asp Lys Glu Gly Ile Pro Pro Asp Gln Gln Arg Leu Ile Phe
   50 55 60
   Ala Gly Lys Gln Leu Glu Asp Gly Arg Thr Leu Ser Asp Tyr Asn Ile
   65 70 75 80
   Gln Lys Glu Ser Thr Leu His Leu Val Leu Arg Leu Arg Ala Ala Met
                   85                  90                  95
   Gln Ile Phe Val Lys Thr Leu Thr Gly Lys Thr Ile Thr Leu Glu Val
               100                 105                 110
   Glu Pro Ser Asp Thr Ile Glu Asn Val Lys Ala Lys Ile Gln Asp Lys
           115                 120                 125
   Glu Gly Ile Pro Pro Asp Gln Gln Arg Leu Ile Trp Ala Gly Lys Gln
       130                 135                 140
   Leu Glu Asp Gly Arg Thr Leu Ser Asp Tyr Asn Ile Gln Lys Glu Ser
   145                 150                 155                 160
   Thr Leu His Leu Val Leu Arg Leu Arg Ala Ala Gly Gly Gly Gly Ser
                   165                 170                 175
   Gly Gly Gly Gly Ser Gly Gly Gly Gly Ser Gln Val Gln Leu Lys Gln
               180                 185                 190
   Ser Gly Pro Gly Leu Val Gln Pro Ser Gln Ser Leu Ser Ile Thr Cys
           195                 200                 205
   Thr Val Ser Gly Phe Ser Leu Thr Asn Tyr Gly Val His Trp Val Arg
       210                 215                 220
   Gln Ser Pro Gly Lys Gly Leu Glu Trp Leu Gly Val Ile Trp Ser Gly
   225                 230                 235                 240
   Gly Asn Thr Asp Tyr Asn Thr Pro Phe Thr Ser Arg Leu Ser Ile Asn
                   245                 250                 255
   Lys Asp Asn Ser Lys Ser Gln Val Phe Phe Lys Met Asn Ser Leu Gln
               260                 265                 270
   Ser Asn Asp Thr Ala Ile Tyr Tyr Cys Ala Arg Ala Leu Thr Tyr Tyr
           275                 280                 285
   Asp Tyr Glu Phe Ala Tyr Trp Gly Gln Gly Thr Leu Val Thr Val Ser
       290                 295                 300
   Ala Ala Ser Thr Lys Gly Pro Ser Val Phe Pro Leu Ala Pro Ser Ser
   305                 310                 315                 320
   Lys Ser Thr Ser Gly Gly Thr Ala Ala Leu Gly Cys Leu Val Lys Asp
                   325                 330                 335
   Tyr Phe Pro Glu Pro Val Thr Val Ser Trp Asn Ser Gly Ala Leu Thr
   340 345 350
   Ser Gly Val His Thr Phe Pro Ala Val Leu Gln Ser Ser Gly Leu Tyr
   355 360 365
   Ser Leu Ser Ser Val Val Thr Val Pro Ser Ser Ser Leu Gly Thr Gln
   370 375 380
   Thr Tyr Ile Cys Asn Val Asn His Lys Pro Ser Asn Thr Lys Val Asp
   385 390 395 400
   Lys Arg Val Glu Pro Lys Ser Cys Asp Lys Thr His Trp Ser His Pro
   405 410 415
   Gln Phe Glu Lys
   420
<210> 58
   <211> 420
   <212> PRT
   <213> Artificial Sequence
<220>
   <223> Cetuximab Fab CH ubiquitin (139090)
<400> 58
   Met Ala Val Leu Gly Leu Leu Phe Cys Leu Val Thr Phe Pro Ser Cys
   1 5 10 15
   Val Leu Ser Gln Val Gln Leu Lys Gln Ser Gly Pro Gly Leu Val Gln
   20 25 30
   Pro Ser Gln Ser Leu Ser Ile Thr Cys Thr Val Ser Gly Phe Ser Leu
   35 40 45
   Thr Asn Tyr Gly Val His Trp Val Arg Gln Ser Pro Gly Lys Gly Leu
   50 55 60
   Glu Trp Leu Gly Val Ile Trp Ser Gly Gly Asn Thr Asp Tyr Asn Thr
   65 70 75 80
   Pro Phe Thr Ser Arg Leu Ser Ile Asn Lys Asp Asn Ser Lys Ser Gln
   85 90 95
   Val Phe Phe Lys Met Asn Ser Leu Gln Ser Asn Asp Thr Ala Ile Tyr
   100 105 110
   Tyr Cys Ala Arg Ala Leu Thr Tyr Tyr Asp Tyr Glu Phe Ala Tyr Trp
   115 120 125
   Gly Gln Gly Thr Leu Val Thr Val Ser Ala Ala Ser Thr Lys Gly Pro
   130 135 140
   Ser Val Phe Pro Leu Ala Pro Ser Ser Lys Ser Thr Ser Gly Gly Thr
   145 150 155 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
   195 200 205
   Val Pro Ser Ser Ser Leu Gly Thr Gln Thr Tyr Ile Cys Asn Val Asn
   210 215 220
   His Lys Pro Ser Asn Thr Lys Val Asp Lys Arg Val Glu Pro Lys Ser
   225 230 235 240
   Cys Asp Lys Thr His Gly Gly Gly Gly Ser Gly Gly Gly Gly Ser Gly
   245 250 255
   Gly Gly Gly Ser Met Gln Ile Phe Val Lys Thr Leu Thr Gly Lys Thr
   260 265 270
   Ile Thr Leu Glu Val Glu Pro Ser Asp Thr Ile Glu Asn Val Lys Ala
   275 280 285
   Lys Ile Gln Asp Lys Glu Gly Ile Pro Pro Asp Gln Gln Arg Leu Ile
   290 295 300
   Phe Ala Gly Lys Gln Leu Glu Asp Gly Arg Thr Leu Ser Asp Tyr Asn
   305 310 315 320
   Ile Gln Lys Glu Ser Thr Leu His Leu Val Leu Arg Leu Arg Ala Ala
   325 330 335
   Met Gln Ile Phe Val Lys Thr Leu Thr Gly Lys Thr Ile Thr Leu Glu
   340 345 350
   Val Glu Pro Ser Asp Thr Ile Glu Asn Val Lys Ala Lys Ile Gln Asp
   355 360 365
   Lys Glu Gly Ile Pro Pro Asp Gln Gln Arg Leu Ile Trp Ala Gly Lys
   370 375 380
   Gln Leu Glu Asp Gly Arg Thr Leu Ser Asp Tyr Asn Ile Gln Lys Glu
   385 390 395 400
   Ser Thr Leu His Leu Val Leu Arg Leu Arg Ala Ala Trp Ser His Pro
   405 410 415
   Gln Phe Glu Lys
   420
<210> 59
   <211> 407
   <212> PRT
   <213> Artificial Sequence
<220>
   <223> Cetuximab Fab NL ubiquitin (139090)
<400> 59
   Met Val Ser Thr Pro Gln Phe Leu Val Phe Leu Leu Phe Trp Ile Pro
   1 5 10 15
   Ala Ser Arg Ser Met Gln Ile Phe Val Lys Thr Leu Thr Gly Lys Thr
   20 25 30
   Ile Thr Leu Glu Val Glu Pro Ser Asp Thr Ile Glu Asn Val Lys Ala
   35 40 45
   Lys Ile Gln Asp Lys Glu Gly Ile Pro Pro Asp Gln Gln Arg Leu Ile
   50 55 60
   Phe Ala Gly Lys Gln Leu Glu Asp Gly Arg Thr Leu Ser Asp Tyr Asn
   65 70 75 80
   Ile Gln Lys Glu Ser Thr Leu His Leu Val Leu Arg Leu Arg Ala Ala
   85 90 95
   Met Gln Ile Phe Val Lys Thr Leu Thr Gly Lys Thr Ile Thr Leu Glu
   100 105 110
   Val Glu Pro Ser Asp Thr Ile Glu Asn Val Lys Ala Lys Ile Gln Asp
   115 120 125
   Lys Glu Gly Ile Pro Pro Asp Gln Gln Arg Leu Ile Trp Ala Gly Lys
   130 135 140
   Gln Leu Glu Asp Gly Arg Thr Leu Ser Asp Tyr Asn Ile Gln Lys Glu
   145 150 155 160
   Ser Thr Leu His Leu Val Leu Arg Leu Arg Ala Ala Gly Gly Gly Gly
   165 170 175
   Ser Gly Gly Gly Gly Ser Gly Gly Gly Gly Ser Asp Ile Leu Leu Thr
   180 185 190
   Gln Ser Pro Val Ile Leu Ser Val Ser Pro Gly Glu Arg Val Ser Phe
   195 200 205
   Ser Cys Arg Ala Ser Gln Ser Ile Gly Thr Asn Ile His Trp Tyr Gln
   210 215 220
   Gln Arg Thr Asn Gly Ser Pro Arg Leu Leu Ile Lys Tyr Ala Ser Glu
   225 230 235 240
   Ser Ile Ser Gly Ile Pro Ser Arg Phe Ser Gly Ser Gly Ser Gly Thr
   245 250 255
   Asp Phe Thr Leu Ser Ile Asn Ser Val Glu Ser Glu Asp Ile Ala Asp
   260 265 270
   Tyr Tyr Cys Gln Gln Asn Asn Asn Trp Pro Thr Thr Phe Gly Ala Gly
   275 280 285
   Thr Lys Leu Glu Leu Lys Arg Thr Val Ala Ala Pro Ser Val Phe Ile
   290 295 300
   Phe Pro Pro Ser Asp Glu Gln Leu Lys Ser Gly Thr Ala Ser Val Val
   305 310 315 320
   Cys Leu Leu Asn Asn Phe Tyr Pro Arg Glu Ala Lys Val Gln Trp Lys
   325 330 335
   Val Asp Asn Ala Leu Gln Ser Gly Asn Ser Gln Glu Ser Val Thr Glu
   340 345 350
   Gln Asp Ser Lys Asp Ser Thr Tyr Ser Leu Ser Ser Thr Leu Thr Leu
   355 360 365
   Ser Lys Ala Asp Tyr Glu Lys His Lys Val Tyr Ala Cys Glu Val Thr
   370 375 380
   His Gln Gly Leu Ser Ser Pro Val Thr Lys Ser Phe Asn Arg Gly Glu
   385 390 395 400
   Cys His His His His His His
   405
<210> 60
   <211> 407
   <212> PRT
   <213> Artificial Sequence
<220>
   <223> Cetuximab Fab CL ubiquitin (139090)
<400> 60
   Met Val Ser Thr Pro Gln Phe Leu Val Phe Leu Leu Phe Trp Ile Pro
   1 5 10 15
   Ala Ser Arg Ser Asp Ile Leu Leu Thr Gln Ser Pro Val Ile Leu Ser
   20 25 30
   Val Ser Pro Gly Glu Arg Val Ser Phe Ser Cys Arg Ala Ser Gln Ser
   35 40 45
   Ile Gly Thr Asn Ile His Trp Tyr Gln Gln Arg Thr Asn Gly Ser Pro
   50 55 60
   Arg Leu Leu Ile Lys Tyr Ala Ser Glu Ser Ile Ser Gly Ile Pro Ser
   65 70 75 80
   Arg Phe Ser Gly Ser Gly Ser Gly Thr Asp Phe Thr Leu Ser Ile Asn
   85 90 95
   Ser Val Glu Ser Glu Asp Ile Ala Asp Tyr Tyr Cys Gln Gln Asn Asn
   100 105 110
   Asn Trp Pro Thr Thr Phe Gly Ala Gly Thr Lys Leu Glu Leu Lys Arg
   115 120 125
   Thr Val Ala Ala Pro Ser Val Phe Ile Phe Pro Pro Ser Asp Glu Gln
   130 135 140
   Leu Lys Ser Gly Thr Ala Ser Val Val Cys Leu Leu Asn Asn Phe Tyr
   145 150 155 160
   Pro Arg Glu Ala Lys Val Gln Trp Lys Val Asp Asn Ala Leu Gln Ser
   165 170 175
   Gly Asn Ser Gln Glu Ser Val Thr Glu Gln Asp Ser Lys Asp Ser Thr
   180 185 190
   Tyr Ser Leu Ser Ser Thr Leu Thr Leu Ser Lys Ala Asp Tyr Glu Lys
   195 200 205
   His Lys Val Tyr Ala Cys Glu Val Thr His Gln Gly Leu Ser Ser Pro
   210 215 220
   Val Thr Lys Ser Phe Asn Arg Gly Glu Cys Gly Gly Gly Gly Ser Gly
   225 230 235 240
   Gly Gly Gly Ser Gly Gly Gly Gly Ser Met Gln Ile Phe Val Lys Thr
   245 250 255
   Leu Thr Gly Lys Thr Ile Thr Leu Glu Val Glu Pro Ser Asp Thr Ile
   260 265 270
   Glu Asn Val Lys Ala Lys Ile Gln Asp Lys Glu Gly Ile Pro Pro Asp
   275 280 285
   Gln Gln Arg Leu Ile Phe Ala Gly Lys Gln Leu Glu Asp Gly Arg Thr
   290 295 300
   Leu Ser Asp Tyr Asn Ile Gln Lys Glu Ser Thr Leu His Leu Val Leu
   305 310 315 320
   Arg Leu Arg Ala Ala Met Gln Ile Phe Val Lys Thr Leu Thr Gly Lys
   325 330 335
   Thr Ile Thr Leu Glu Val Glu Pro Ser Asp Thr Ile Glu Asn Val Lys
   340 345 350
   Ala Lys Ile Gln Asp Lys Glu Gly Ile Pro Pro Asp Gln Gln Arg Leu
   355 360 365
   Ile Trp Ala Gly Lys Gln Leu Glu Asp Gly Arg Thr Leu Ser Asp Tyr
   370 375 380
   Asn Ile Gln Lys Glu Ser Thr Leu His Leu Val Leu Arg Leu Arg Ala
   385 390 395 400
   Ala His His His His His His
   405
<210> 61
   <211> 344
   <212> PRT
   <213> Artificial Sequence
<220>
   <223> Cetuximab Fab NH Ubiquitin
<400> 61
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Arg Leu Ile Trp
       50                  55                  60
   Ala Gly Lys Gln Leu Glu Asp Gly Arg Thr Leu Ser Asp Tyr Asn Ile
   65                  70                  75                  80
   Gln Lys Glu Ser Thr Leu His Leu Val Leu Arg Leu Arg Ala Ala Gly
                   85                  90                  95
   Gly Gly Gly Ser Gly Gly Gly Gly Ser Gly Gly Gly Gly Ser Gln Val
               100                 105                 110
   Gln Leu Lys Gln Ser Gly Pro Gly Leu Val Gln Pro Ser Gln Ser Leu
           115                 120                 125
   Ser Ile Thr Cys Thr Val Ser Gly Phe Ser Leu Thr Asn Tyr Gly Val
       130                 135                 140
   His Trp Val Arg Gln Ser Pro Gly Lys Gly Leu Glu Trp Leu Gly Val
   145                 150                 155                 160
   Ile Trp Ser Gly Gly Asn Thr Asp Tyr Asn Thr Pro Phe Thr Ser Arg
                   165                 170                 175
   Leu Ser Ile Asn Lys Asp Asn Ser Lys Ser Gln Val Phe Phe Lys Met
               180                 185                 190
   Asn Ser Leu Gln Ser Asn Asp Thr Ala Ile Tyr Tyr Cys Ala Arg Ala
           195                 200                 205
   Leu Thr Tyr Tyr Asp Tyr Glu Phe Ala Tyr Trp Gly Gln Gly Thr Leu
       210                 215                 220
   Val Thr Val Ser Ala Ala Ser Thr Lys Gly Pro Ser Val Phe Pro Leu
   225                 230                 235                 240
   Ala Pro Ser Ser Lys Ser Thr Ser Gly Gly Thr Ala Ala Leu Gly Cys
   245 250 255
   Leu Val Lys Asp Tyr Phe Pro Glu Pro Val Thr Val Ser Trp Asn Ser
   260 265 270
   Gly Ala Leu Thr Ser Gly Val His Thr Phe Pro Ala Val Leu Gln Ser
   275 280 285
   Ser Gly Leu Tyr Ser Leu Ser Ser Val Val Thr Val Pro Ser Ser Ser
   290 295 300
   Leu Gly Thr Gln Thr Tyr Ile Cys Asn Val Asn His Lys Pro Ser Asn
   305 310 315 320
   Thr Lys Val Asp Lys Arg Val Glu Pro Lys Ser Cys Asp Lys Thr His
   325 330 335
   Trp Ser His Pro Gln Phe Glu Lys
   340
<210> 62
   <211> 344
   <212> PRT
   <213> Artificial Sequence
<220>
   <223> Cetuximab Fab CH Ubiquitin
<400> 62
   Met Ala Val Leu Gly Leu Leu Phe Cys Leu Val Thr Phe Pro Ser Cys
   1 5 10 15
   Val Leu Ser Gln Val Gln Leu Lys Gln Ser Gly Pro Gly Leu Val Gln
   20 25 30
   Pro Ser Gln Ser Leu Ser Ile Thr Cys Thr Val Ser Gly Phe Ser Leu
   35 40 45
   Thr Asn Tyr Gly Val His Trp Val Arg Gln Ser Pro Gly Lys Gly Leu
   50 55 60
   Glu Trp Leu Gly Val Ile Trp Ser Gly Gly Asn Thr Asp Tyr Asn Thr
   65 70 75 80
   Pro Phe Thr Ser Arg Leu Ser Ile Asn Lys Asp Asn Ser Lys Ser Gln
   85 90 95
   Val Phe Phe Lys Met Asn Ser Leu Gln Ser Asn Asp Thr Ala Ile Tyr
   100 105 110
   Tyr Cys Ala Arg Ala Leu Thr Tyr Tyr Asp Tyr Glu Phe Ala Tyr Trp
   115 120 125
   Gly Gln Gly Thr Leu Val Thr Val Ser Ala Ala Ser Thr Lys Gly Pro
   130 135 140
   Ser Val Phe Pro Leu Ala Pro Ser Ser Lys Ser Thr Ser Gly Gly Thr
   145 150 155 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
   195 200 205
   Val Pro Ser Ser Ser Leu Gly Thr Gln Thr Tyr Ile Cys Asn Val Asn
   210 215 220
   His Lys Pro Ser Asn Thr Lys Val Asp Lys Arg Val Glu Pro Lys Ser
   225 230 235 240
   Cys Asp Lys Thr His Gly Gly Gly Gly Ser Gly Gly Gly Gly Ser Gly
   245 250 255
   Gly Gly Gly Ser Met Gln Ile Phe Val Lys Thr Leu Thr Gly Lys Thr
   260 265 270
   Ile Thr Leu Glu Val Glu Pro Ser Asp Thr Ile Glu Asn Val Lys Ala
   275 280 285
   Lys Ile Gln Asp Lys Glu Gly Ile Pro Pro Asp Gln Gln Arg Leu Ile
   290 295 300
   Trp Ala Gly Lys Gln Leu Glu Asp Gly Arg Thr Leu Ser Asp Tyr Asn
   305 310 315 320
   Ile Gln Lys Glu Ser Thr Leu His Leu Val Leu Arg Leu Arg Ala Ala
   325 330 335
   Trp Ser His Pro Gln Phe Glu Lys
   340
<210> 63
   <211> 152
   <212> PRT
   <213> Artificial Sequence
<220>
   <223> Affilin 142628
<400> 63
   Met Gln Ile Phe Val Lys Thr Leu Thr Gly Lys Thr Ile Thr Leu Glu
   1               5                   10                  15
   Val Glu Pro Ser Asp Thr Thr Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Asp Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 64
   <211> 19
   <212> PRT
   <213> Artificial Sequence
<220>
   <223> OKT3 signal sequence heavy chain
<400> 64
   Met Glu Arg His Trp Ile Phe Leu Leu Leu Leu Ser Val Thr Ala Gly
   1               5                   10                  15
   Val His Ser
<210> 65
   <211> 22
   <212> PRT
   <213> Artificial Sequence
<220>
   <223> OKT3 signal sequence light chain
<400> 65
   Met Asp Phe Gln Val Gln Ile Phe Ser Phe Leu Leu Ile Ser Ala Ser
   1               5                   10                  15
   Val Ile Ile Ser Arg Gly
               20
<210> 66
   <211> 476
   <212> PRT
   <213> Artificial Sequence
<220>
   <223> OKT3 heavy chain
<400> 66
   Met Glu Arg His Trp Ile Phe Leu Leu Leu Leu Ser Val Thr Ala Gly
   1               5                   10                  15
   Val His Ser Gln Val Gln Leu Gln Gln Ser Gly Ala Glu Leu Ala Arg
               20                  25                  30
   Pro Gly Ala Ser Val Lys Met Ser Cys Lys Ala Ser Gly Tyr Thr Phe
           35                  40                  45
   Thr Arg Tyr Thr Met His Trp Val Lys Gln Arg Pro Gly Gln Gly Leu
       50                  55                  60
   Glu Trp Ile Gly Tyr Ile Asn Pro Ser Arg Gly Tyr Thr Asn Tyr Asn
   65                  70                  75                  80
   Gln Lys Phe Lys Asp Lys Ala Thr Leu Thr Thr Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Tyr Tyr Asp Asp His Tyr Cys Leu Asp Tyr Trp
   115 120 125
   Gly Gln Gly Thr Thr Leu Thr Val Ser Ser Ala Lys Thr Thr Ala Pro
   130 135 140
   Ser Val Tyr Pro Leu Ala Pro Val Cys Gly Asp Thr Thr Gly Ser Ser
   145 150 155 160
   Val Thr Leu Gly Cys Leu Val Lys Gly Tyr Phe Pro Glu Pro Val Thr
   165 170 175
   Leu Thr Trp Asn Ser Gly Ser Leu Ser Ser Gly Val His Thr Phe Pro
   180 185 190
   Ala Val Leu Gln Ser Asp Leu Tyr Thr Leu Ser Ser Ser Val Thr Val
   195 200 205
   Thr Ser Ser Thr Trp Pro Ser Gln Ser Ile Thr Cys Asn Val Ala His
   210 215 220
   Pro Ala Ser Ser Thr Lys Val Asp Lys Lys Ile Glu Pro Arg Gly Pro
   225 230 235 240
   Thr Ile Lys Pro Cys Pro Pro Cys Lys Cys Pro Ala Pro Asn Leu Leu
   245 250 255
   Gly Gly Pro Ser Val Phe Ile Phe Pro Pro Lys Ile Lys Asp Val Leu
   260 265 270
   Met Ile Ser Leu Ser Pro Ile Val Thr Cys Val Val Val Asp Val Ser
   275 280 285
   Glu Asp Asp Pro Asp Val Gln Ile Ser Trp Phe Val Asn Asn Val Glu
   290 295 300
   Val His Thr Ala Gln Thr Gln Thr His Arg Glu Asp Tyr Asn Ser Thr
   305 310 315 320
   Leu Arg Val Val Ser Ala Leu Pro Ile Gln His Gln Asp Trp Met Ser
   325 330 335
   Gly Lys Glu Phe Lys Cys Lys Val Asn Asn Lys Asp Leu Pro Ala Pro
   340 345 350
   Ile Glu Arg Thr Ile Ser Lys Pro Lys Gly Ser Val Arg Ala Pro Gln
   355 360 365
   Val Tyr Val Leu Pro Pro Pro Glu Glu Glu Met Thr Lys Lys Gln Val
   370 375 380
   Thr Leu Thr Cys Met Val Thr Asp Phe Met Pro Glu Asp Ile Tyr Val
   385 390 395 400
   Glu Trp Thr Asn Asn Gly Lys Thr Glu Leu Asn Tyr Lys Asn Thr Glu
   405 410 415
   Pro Val Leu Asp Ser Asp Gly Ser Tyr Phe Met Tyr Ser Lys Leu Arg
   420 425 430
   Val Glu Lys Lys Asn Trp Val Glu Arg Asn Ser Tyr Ser Cys Ser Val
   435 440 445
   Val His Glu Gly Leu His Asn His His Thr Thr Lys Ser Phe Ser Arg
   450 455 460
   Thr Pro Gly Lys Trp Ser His Pro Gln Phe Glu Lys
   465 470 475
<210> 67
   <211> 241
   <212> PRT
   <213> Artificial Sequence
<220>
   <223> OKT3 light chain
<400> 67
   Met Asp Phe Gln Val Gln Ile Phe Ser Phe Leu Leu Ile Ser Ala Ser
   1               5                   10                  15
   Val Ile Ile Ser Arg Gly Gln Ile Val Leu Thr Gln Ser Pro Ala Ile
               20                  25                  30
   Met Ser Ala Ser Pro Gly Glu Lys Val Thr Met Thr Cys Ser Ala Ser
           35                  40                  45
   Ser Ser Val Ser Tyr Met Asn Trp Tyr Gln Gln Lys Ser Gly Thr Ser
       50                  55                  60
   Pro Lys Arg Trp Ile Tyr Asp Thr Ser Lys Leu Ala Ser Gly Val Pro
   65                  70                  75                  80
   Ala His Phe Arg Gly Ser Gly Ser Gly Thr Ser Tyr Ser Leu Thr Ile
                   85                  90                  95
   Ser Gly Met Glu Ala Glu Asp Ala Ala Thr Tyr Tyr Cys Gln Gln Trp
   100 105 110
   Ser Ser Asn Pro Phe Thr Phe Gly Ser Gly Thr Lys Leu Glu Ile Asn
   115 120 125
   Arg Ala Asp Thr Ala Pro Thr Val Ser Ile Phe Pro Pro Ser Ser Glu
   130 135 140
   Gln Leu Thr Ser Gly Gly Ala Ser Val Val Cys Phe Leu Asn Asn Phe
   145 150 155 160
   Tyr Pro Lys Asp Ile Asn Val Lys Trp Lys Ile Asp Gly Ser Glu Arg
   165 170 175
   Gln Asn Gly Val Leu Asn Ser Trp Thr Asp Gln Asp Ser Lys Asp Ser
   180 185 190
   Thr Tyr Ser Met Ser Ser Thr Leu Thr Leu Thr Lys Asp Glu Tyr Glu
   195 200 205
   Arg His Asn Ser Tyr Thr Cys Glu Ala Thr His Lys Thr Ser Thr Ser
   210 215 220
   Pro Ile Val Lys Ser Phe Asn Arg Asn Glu Cys His His His His His
   225 230 235 240
   His
<210> 68
   <211> 246
   <212> PRT
   <213> Artificial Sequence
<220>
   <223> OKT3 Fab heavy chain
<400> 68
   Met Glu Arg His Trp Ile Phe Leu Leu Leu Leu Ser Val Thr Ala Gly
   1               5                   10                  15
   Val His Ser Gln Val Gln Leu Gln Gln Ser Gly Ala Glu Leu Ala Arg
               20                  25                  30
   Pro Gly Ala Ser Val Lys Met Ser Cys Lys Ala Ser Gly Tyr Thr Phe
           35                  40                  45
   Thr Arg Tyr Thr Met His Trp Val Lys Gln Arg Pro Gly Gln Gly Leu
       50                  55                  60
   Glu Trp Ile Gly Tyr Ile Asn Pro Ser Arg Gly Tyr Thr Asn Tyr Asn
   65                  70                  75                  80
   Gln Lys Phe Lys Asp Lys Ala Thr Leu Thr Thr Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Tyr Tyr Asp Asp His Tyr Cys Leu Asp Tyr Trp
           115                 120                 125
   Gly Gln Gly Thr Thr Leu Thr Val Ser Ser Ala Lys Thr Thr Ala Pro
       130                 135                 140
   Ser Val Tyr Pro Leu Ala Pro Val Cys Gly Asp Thr Thr Gly Ser Ser
   145                 150                 155                 160
   Val Thr Leu Gly Cys Leu Val Lys Gly Tyr Phe Pro Glu Pro Val Thr
                   165                 170                 175
   Leu Thr Trp Asn Ser Gly Ser Leu Ser Ser Gly Val His Thr Phe Pro
               180                 185                 190
   Ala Val Leu Gln Ser Asp Leu Tyr Thr Leu Ser Ser Ser Val Thr Val
           195                 200                 205
   Thr Ser Ser Thr Trp Pro Ser Gln Ser Ile Thr Cys Asn Val Ala His
       210                 215                 220
   Pro Ala Ser Ser Thr Lys Val Asp Lys Lys Ile Glu Pro Arg Trp Ser
   225                 230                 235                 240
   His Pro Gln Phe Glu Lys
                   245
<210> 69
   <211> 643
   <212> PRT
   <213> Artificial Sequence
<220>
   <223> OKT3 mAb NH 142628
<400> 69
   Met Glu Arg His Trp Ile Phe Leu Leu Leu Leu Ser Val Thr Ala Gly
   1               5                   10                  15
   Val His Ser Met Gln Ile Phe Val Lys Thr Leu Thr Gly Lys Thr Ile
               20                  25                  30
   Thr Leu Glu Val Glu Pro Ser Asp Thr Thr Glu Asn Val Lys Ala Lys
           35                  40                  45
   Ile Gln Asp Lys Glu Gly Ile Pro Pro Asp Gln Gln Thr Leu Ala Phe
       50                  55                  60
   Val Gly Lys Gln Leu Glu Asp Gly Arg Thr Leu Ser Asp Tyr Asn Ile
   65                  70                  75                  80
   Gln Lys Glu Ser Thr Leu Trp Leu Tyr Leu Thr Trp Tyr Ala Ala Met
                   85                  90                  95
   Arg Ile Phe Val Thr Thr His Thr Gly Lys Thr Ile Thr Leu Asp Val
               100                 105                 110
   Glu Pro Ser Asp Thr Ile Glu Asn Val Lys Ala Lys Ile Gln Asp Lys
           115                 120                 125
   Glu Gly Ile Pro Pro Asp Gln Gln Arg Leu Ile Trp Ala Gly Lys Gln
       130                 135                 140
   Leu Glu Asp Gly Arg Thr Leu Ser Asp Tyr Asn Ile Ser Glu Trp Ala
   145                 150                 155                 160
   Ile Leu His Leu Val Leu Arg Leu Arg Ala Ala Gly Gly Gly Gly Ser
                   165                 170                 175
   Gly Gly Gly Gly Ser Gly Gly Gly Gly Ser Gln Val Gln Leu Gln Gln
               180                 185                 190
   Ser Gly Ala Glu Leu Ala Arg Pro Gly Ala Ser Val Lys Met Ser Cys
           195                 200                 205
   Lys Ala Ser Gly Tyr Thr Phe Thr Arg Tyr Thr Met His Trp Val Lys
       210                 215                 220
   Gln Arg Pro Gly Gln Gly Leu Glu Trp Ile Gly Tyr Ile Asn Pro Ser
   225                 230                 235                 240
   Arg Gly Tyr Thr Asn Tyr Asn Gln Lys Phe Lys Asp Lys Ala Thr Leu
                   245                 250                 255
   Thr Thr Asp Lys Ser Ser Ser Thr Ala Tyr Met Gln Leu Ser Ser Leu
               260                 265                 270
   Thr Ser Glu Asp Ser Ala Val Tyr Tyr Cys Ala Arg Tyr Tyr Asp Asp
   275 280 285
   His Tyr Cys Leu Asp Tyr Trp Gly Gln Gly Thr Thr Leu Thr Val Ser
   290 295 300
   Ser Ala Lys Thr Thr Ala Pro Ser Val Tyr Pro Leu Ala Pro Val Cys
   305 310 315 320
   Gly Asp Thr Thr Gly Ser Ser Val Thr Leu Gly Cys Leu Val Lys Gly
   325 330 335
   Tyr Phe Pro Glu Pro Val Thr Leu Thr Trp Asn Ser Gly Ser Leu Ser
   340 345 350
   Ser Gly Val His Thr Phe Pro Ala Val Leu Gln Ser Asp Leu Tyr Thr
   355 360 365
   Leu Ser Ser Ser Val Thr Val Thr Ser Ser Thr Trp Pro Ser Gln Ser
   370 375 380
   Ile Thr Cys Asn Val Ala His Pro Ala Ser Ser Thr Lys Val Asp Lys
   385 390 395 400
   Lys Ile Glu Pro Arg Gly Pro Thr Ile Lys Pro Cys Pro Pro Cys Lys
   405 410 415
   Cys Pro Ala Pro Asn Leu Leu Gly Gly Pro Ser Val Phe Ile Phe Pro
   420 425 430
   Pro Lys Ile Lys Asp Val Leu Met Ile Ser Leu Ser Pro Ile Val Thr
   435 440 445
   Cys Val Val Val Asp Val Ser Glu Asp Asp Pro Asp Val Gln Ile Ser
   450 455 460
   Trp Phe Val Asn Asn Val Glu Val His Thr Ala Gln Thr Gln Thr His
   465 470 475 480
   Arg Glu Asp Tyr Asn Ser Thr Leu Arg Val Val Ser Ala Leu Pro Ile
   485 490 495
   Gln His Gln Asp Trp Met Ser Gly Lys Glu Phe Lys Cys Lys Val Asn
   500 505 510
   Asn Lys Asp Leu Pro Ala Pro Ile Glu Arg Thr Ile Ser Lys Pro Lys
   515 520 525
   Gly Ser Val Arg Ala Pro Gln Val Tyr Val Leu Pro Pro Pro Glu Glu
   530 535 540
   Glu Met Thr Lys Lys Gln Val Thr Leu Thr Cys Met Val Thr Asp Phe
   545 550 555 560
   Met Pro Glu Asp Ile Tyr Val Glu Trp Thr Asn Asn Gly Lys Thr Glu
   565 570 575
   Leu Asn Tyr Lys Asn Thr Glu Pro Val Leu Asp Ser Asp Gly Ser Tyr
   580 585 590
   Phe Met Tyr Ser Lys Leu Arg Val Glu Lys Lys Asn Trp Val Glu Arg
   595 600 605
   Asn Ser Tyr Ser Cys Ser Val Val His Glu Gly Leu His Asn His His
   610 615 620
   Thr Thr Lys Ser Phe Ser Arg Thr Pro Gly Lys Trp Ser His Pro Gln
   625 630 635 640
   Phe Glu Lys
<210> 70
   <211> 643
   <212> PRT
   <213> Artificial Sequence
<220>
   <223> OKT3 mAB CH 142628
<400> 70
   Met Glu Arg His Trp Ile Phe Leu Leu Leu Leu Ser Val Thr Ala Gly
   1 5 10 15
   Val His Ser Gln Val Gln Leu Gln Gln Ser Gly Ala Glu Leu Ala Arg
   20 25 30
   Pro Gly Ala Ser Val Lys Met Ser Cys Lys Ala Ser Gly Tyr Thr Phe
   35 40 45
   Thr Arg Tyr Thr Met His Trp Val Lys Gln Arg Pro Gly Gln Gly Leu
   50 55 60
   Glu Trp Ile Gly Tyr Ile Asn Pro Ser Arg Gly Tyr Thr Asn Tyr Asn
   65 70 75 80
   Gln Lys Phe Lys Asp Lys Ala Thr Leu Thr Thr Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Tyr Tyr Asp Asp His Tyr Cys Leu Asp Tyr Trp
           115                 120                 125
   Gly Gln Gly Thr Thr Leu Thr Val Ser Ser Ala Lys Thr Thr Ala Pro
       130                 135                 140
   Ser Val Tyr Pro Leu Ala Pro Val Cys Gly Asp Thr Thr Gly Ser Ser
   145                 150                 155                 160
   Val Thr Leu Gly Cys Leu Val Lys Gly Tyr Phe Pro Glu Pro Val Thr
                   165                 170                 175
   Leu Thr Trp Asn Ser Gly Ser Leu Ser Ser Gly Val His Thr Phe Pro
               180                 185                 190
   Ala Val Leu Gln Ser Asp Leu Tyr Thr Leu Ser Ser Ser Val Thr Val
           195                 200                 205
   Thr Ser Ser Thr Trp Pro Ser Gln Ser Ile Thr Cys Asn Val Ala His
       210                 215                 220
   Pro Ala Ser Ser Thr Lys Val Asp Lys Lys Ile Glu Pro Arg Gly Pro
   225                 230                 235                 240
   Thr Ile Lys Pro Cys Pro Pro Cys Lys Cys Pro Ala Pro Asn Leu Leu
                   245                 250                 255
   Gly Gly Pro Ser Val Phe Ile Phe Pro Pro Lys Ile Lys Asp Val Leu
               260                 265                 270
   Met Ile Ser Leu Ser Pro Ile Val Thr Cys Val Val Val Asp Val Ser
           275                 280                 285
   Glu Asp Asp Pro Asp Val Gln Ile Ser Trp Phe Val Asn Asn Val Glu
       290                 295                 300
   Val His Thr Ala Gln Thr Gln Thr His Arg Glu Asp Tyr Asn Ser Thr
   305                 310                 315                 320
   Leu Arg Val Val Ser Ala Leu Pro Ile Gln His Gln Asp Trp Met Ser
                   325                 330                 335
   Gly Lys Glu Phe Lys Cys Lys Val Asn Asn Lys Asp Leu Pro Ala Pro
               340                 345                 350
   Ile Glu Arg Thr Ile Ser Lys Pro Lys Gly Ser Val Arg Ala Pro Gln
           355                 360                 365
   Val Tyr Val Leu Pro Pro Pro Glu Glu Glu Met Thr Lys Lys Gln Val
       370                 375                 380
   Thr Leu Thr Cys Met Val Thr Asp Phe Met Pro Glu Asp Ile Tyr Val
   385                 390                 395                 400
   Glu Trp Thr Asn Asn Gly Lys Thr Glu Leu Asn Tyr Lys Asn Thr Glu
                   405                 410                 415
   Pro Val Leu Asp Ser Asp Gly Ser Tyr Phe Met Tyr Ser Lys Leu Arg
               420                 425                 430
   Val Glu Lys Lys Asn Trp Val Glu Arg Asn Ser Tyr Ser Cys Ser Val
           435                 440                 445
   Val His Glu Gly Leu His Asn His His Thr Thr Lys Ser Phe Ser Arg
       450                 455                 460
   Thr Pro Gly Lys Gly Gly Gly Gly Ser Gly Gly Gly Gly Ser Gly Gly
   465                 470                 475                 480
   Gly Gly Ser Met Gln Ile Phe Val Lys Thr Leu Thr Gly Lys Thr Ile
                   485                 490                 495
   Thr Leu Glu Val Glu Pro Ser Asp Thr Thr Glu Asn Val Lys Ala Lys
               500                 505                 510
   Ile Gln Asp Lys Glu Gly Ile Pro Pro Asp Gln Gln Thr Leu Ala Phe
           515                 520                 525
   Val Gly Lys Gln Leu Glu Asp Gly Arg Thr Leu Ser Asp Tyr Asn Ile
       530                 535                 540
   Gln Lys Glu Ser Thr Leu Trp Leu Tyr Leu Thr Trp Tyr Ala Ala Met
   545                 550                 555                 560
   Arg Ile Phe Val Thr Thr His Thr Gly Lys Thr Ile Thr Leu Asp Val
                   565                 570                 575
   Glu Pro Ser Asp Thr Ile Glu Asn Val Lys Ala Lys Ile Gln Asp Lys
   580 585 590
   Glu Gly Ile Pro Pro Asp Gln Gln Arg Leu Ile Trp Ala Gly Lys Gln
   595 600 605
   Leu Glu Asp Gly Arg Thr Leu Ser Asp Tyr Asn Ile Ser Glu Trp Ala
   610 615 620
   Ile Leu His Leu Val Leu Arg Leu Arg Ala Ala Trp Ser His Pro Gln
   625 630 635 640
   Phe Glu Lys
<210> 71
   <211> 408
   <212> PRT
   <213> Artificial Sequence
<220>
   <223> OKT3 Mab NL 142628
<400> 71
   Met Asp Phe Gln Val Gln Ile Phe Ser Phe Leu Leu Ile Ser Ala Ser
   1               5                   10                  15
   Val Ile Ile Ser Arg Gly Met Gln Ile Phe Val Lys Thr Leu Thr Gly
               20                  25                  30
   Lys Thr Ile Thr Leu Glu Val Glu Pro Ser Asp Thr Thr Glu Asn Val
           35                  40                  45
   Lys Ala Lys Ile Gln Asp Lys Glu Gly Ile Pro Pro Asp Gln Gln Thr
       50                  55                  60
   Leu Ala Phe Val Gly Lys Gln Leu Glu Asp Gly Arg Thr Leu Ser Asp
   65                  70                  75                  80
   Tyr Asn Ile Gln Lys Glu Ser Thr Leu Trp Leu Tyr Leu Thr Trp Tyr
                   85                  90                  95
   Ala Ala Met Arg Ile Phe Val Thr Thr His Thr Gly Lys Thr Ile Thr
               100                 105                 110
   Leu Asp Val Glu Pro Ser Asp Thr Ile Glu Asn Val Lys Ala Lys Ile
           115                 120                 125
   Gln Asp Lys Glu Gly Ile Pro Pro Asp Gln Gln Arg Leu Ile Trp Ala
       130                 135                 140
   Gly Lys Gln Leu Glu Asp Gly Arg Thr Leu Ser Asp Tyr Asn Ile Ser
   145 150 155 160
   Glu Trp Ala Ile Leu His Leu Val Leu Arg Leu Arg Ala Ala Gly Gly
   165 170 175
   Gly Gly Ser Gly Gly Gly Gly Ser Gly Gly Gly Gly Ser Gln Ile Val
   180 185 190
   Leu Thr Gln Ser Pro Ala Ile Met Ser Ala Ser Pro Gly Glu Lys Val
   195 200 205
   Thr Met Thr Cys Ser Ala Ser Ser Ser Val Ser Tyr Met Asn Trp Tyr
   210 215 220
   Gln Gln Lys Ser Gly Thr Ser Pro Lys Arg Trp Ile Tyr Asp Thr Ser
   225 230 235 240
   Lys Leu Ala Ser Gly Val Pro Ala His Phe Arg Gly Ser Gly Ser Gly
   245 250 255
   Thr Ser Tyr Ser Leu Thr Ile Ser Gly Met Glu Ala Glu Asp Ala Ala
   260 265 270
   Thr Tyr Tyr Cys Gln Gln Trp Ser Ser Asn Pro Phe Thr Phe Gly Ser
   275 280 285
   Gly Thr Lys Leu Glu Ile Asn Arg Ala Asp Thr Ala Pro Thr Val Ser
   290 295 300
   Ile Phe Pro Pro Ser Ser Glu Gln Leu Thr Ser Gly Gly Ala Ser Val
   305 310 315 320
   Val Cys Phe Leu Asn Asn Phe Tyr Pro Lys Asp Ile Asn Val Lys Trp
   325 330 335
   Lys Ile Asp Gly Ser Glu Arg Gln Asn Gly Val Leu Asn Ser Trp Thr
   340 345 350
   Asp Gln Asp Ser Lys Asp Ser Thr Tyr Ser Met Ser Ser Thr Leu Thr
   355 360 365
   Leu Thr Lys Asp Glu Tyr Glu Arg His Asn Ser Tyr Thr Cys Glu Ala
   370 375 380
   Thr His Lys Thr Ser Thr Ser Pro Ile Val Lys Ser Phe Asn Arg Asn
   385 390 395 400
   Glu Cys His His His His His His
   405
<210> 72
   <211> 408
   <212> PRT
   <213> Artificial Sequence
<220>
   <223> OKT3 mAB CL 142628
<400> 72
   Met Asp Phe Gln Val Gln Ile Phe Ser Phe Leu Leu Ile Ser Ala Ser
   1               5                   10                  15
   Val Ile Ile Ser Arg Gly Gln Ile Val Leu Thr Gln Ser Pro Ala Ile
               20                  25                  30
   Met Ser Ala Ser Pro Gly Glu Lys Val Thr Met Thr Cys Ser Ala Ser
           35                  40                  45
   Ser Ser Val Ser Tyr Met Asn Trp Tyr Gln Gln Lys Ser Gly Thr Ser
       50                  55                  60
   Pro Lys Arg Trp Ile Tyr Asp Thr Ser Lys Leu Ala Ser Gly Val Pro
   65                  70                  75                  80
   Ala His Phe Arg Gly Ser Gly Ser Gly Thr Ser Tyr Ser Leu Thr Ile
                   85                  90                  95
   Ser Gly Met Glu Ala Glu Asp Ala Ala Thr Tyr Tyr Cys Gln Gln Trp
               100                 105                 110
   Ser Ser Asn Pro Phe Thr Phe Gly Ser Gly Thr Lys Leu Glu Ile Asn
           115                 120                 125
   Arg Ala Asp Thr Ala Pro Thr Val Ser Ile Phe Pro Pro Ser Ser Glu
       130                 135                 140
   Gln Leu Thr Ser Gly Gly Ala Ser Val Val Cys Phe Leu Asn Asn Phe
   145                 150                 155                 160
   Tyr Pro Lys Asp Ile Asn Val Lys Trp Lys Ile Asp Gly Ser Glu Arg
                   165                 170                 175
   Gln Asn Gly Val Leu Asn Ser Trp Thr Asp Gln Asp Ser Lys Asp Ser
               180                 185                 190
   Thr Tyr Ser Met Ser Ser Thr Leu Thr Leu Thr Lys Asp Glu Tyr Glu
   195 200 205
   Arg His Asn Ser Tyr Thr Cys Glu Ala Thr His Lys Thr Ser Thr Ser
   210 215 220
   Pro Ile Val Lys Ser Phe Asn Arg Asn Glu Cys Gly Gly Gly Gly Ser
   225 230 235 240
   Gly Gly Gly Gly Ser Gly Gly Gly Gly Ser Met Gln Ile Phe Val Lys
   245 250 255
   Thr Leu Thr Gly Lys Thr Ile Thr Leu Glu Val Glu Pro Ser Asp Thr
   260 265 270
   Thr Glu Asn Val Lys Ala Lys Ile Gln Asp Lys Glu Gly Ile Pro Pro
   275 280 285
   Asp Gln Gln Thr Leu Ala Phe Val Gly Lys Gln Leu Glu Asp Gly Arg
   290 295 300
   Thr Leu Ser Asp Tyr Asn Ile Gln Lys Glu Ser Thr Leu Trp Leu Tyr
   305 310 315 320
   Leu Thr Trp Tyr Ala Ala Met Arg Ile Phe Val Thr Thr His Thr Gly
   325 330 335
   Lys Thr Ile Thr Leu Asp Val Glu Pro Ser Asp Thr Ile Glu Asn Val
   340 345 350
   Lys Ala Lys Ile Gln Asp Lys Glu Gly Ile Pro Pro Asp Gln Gln Arg
   355 360 365
   Leu Ile Trp Ala Gly Lys Gln Leu Glu Asp Gly Arg Thr Leu Ser Asp
   370 375 380
   Tyr Asn Ile Ser Glu Trp Ala Ile Leu His Leu Val Leu Arg Leu Arg
   385 390 395 400
   Ala Ala His His His His His His
   405
<210> 73
   <211> 643
   <212> PRT
   <213> Artificial Sequence
<220>
   <223> OKT3 mAb NH ubiquitin 139090
<400> 73
   Met Glu Arg His Trp Ile Phe Leu Leu Leu Leu Ser Val Thr Ala Gly
   1               5                   10                  15
   Val His Ser Met Gln Ile Phe Val Lys Thr Leu Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Arg Leu Ile Phe
       50                  55                  60
   Ala Gly Lys Gln Leu Glu Asp Gly Arg Thr Leu Ser Asp Tyr Asn Ile
   65                  70                  75                  80
   Gln Lys Glu Ser Thr Leu His Leu Val Leu Arg Leu Arg Ala Ala Met
                   85                  90                  95
   Gln Ile Phe Val Lys Thr Leu Thr Gly Lys Thr Ile Thr Leu Glu Val
               100                 105                 110
   Glu Pro Ser Asp Thr Ile Glu Asn Val Lys Ala Lys Ile Gln Asp Lys
           115                 120                 125
   Glu Gly Ile Pro Pro Asp Gln Gln Arg Leu Ile Trp Ala Gly Lys Gln
       130                 135                 140
   Leu Glu Asp Gly Arg Thr Leu Ser Asp Tyr Asn Ile Gln Lys Glu Ser
   145                 150                 155                 160
   Thr Leu His Leu Val Leu Arg Leu Arg Ala Ala Gly Gly Gly Gly Ser
                   165                 170                 175
   Gly Gly Gly Gly Ser Gly Gly Gly Gly Ser Gln Val Gln Leu Gln Gln
               180                 185                 190
   Ser Gly Ala Glu Leu Ala Arg Pro Gly Ala Ser Val Lys Met Ser Cys
           195                 200                 205
   Lys Ala Ser Gly Tyr Thr Phe Thr Arg Tyr Thr Met His Trp Val Lys
       210                 215                 220
   Gln Arg Pro Gly Gln Gly Leu Glu Trp Ile Gly Tyr Ile Asn Pro Ser
   225                 230                 235                 240
   Arg Gly Tyr Thr Asn Tyr Asn Gln Lys Phe Lys Asp Lys Ala Thr Leu
   245 250 255
   Thr Thr Asp Lys Ser Ser Ser Thr Ala Tyr Met Gln Leu Ser Ser Leu
   260 265 270
   Thr Ser Glu Asp Ser Ala Val Tyr Tyr Cys Ala Arg Tyr Tyr Asp Asp
   275 280 285
   His Tyr Cys Leu Asp Tyr Trp Gly Gln Gly Thr Thr Leu Thr Val Ser
   290 295 300
   Ser Ala Lys Thr Thr Ala Pro Ser Val Tyr Pro Leu Ala Pro Val Cys
   305 310 315 320
   Gly Asp Thr Thr Gly Ser Ser Val Thr Leu Gly Cys Leu Val Lys Gly
   325 330 335
   Tyr Phe Pro Glu Pro Val Thr Leu Thr Trp Asn Ser Gly Ser Leu Ser
   340 345 350
   Ser Gly Val His Thr Phe Pro Ala Val Leu Gln Ser Asp Leu Tyr Thr
   355 360 365
   Leu Ser Ser Ser Val Thr Val Thr Ser Ser Thr Trp Pro Ser Gln Ser
   370 375 380
   Ile Thr Cys Asn Val Ala His Pro Ala Ser Ser Thr Lys Val Asp Lys
   385 390 395 400
   Lys Ile Glu Pro Arg Gly Pro Thr Ile Lys Pro Cys Pro Pro Cys Lys
   405 410 415
   Cys Pro Ala Pro Asn Leu Leu Gly Gly Pro Ser Val Phe Ile Phe Pro
   420 425 430
   Pro Lys Ile Lys Asp Val Leu Met Ile Ser Leu Ser Pro Ile Val Thr
   435 440 445
   Cys Val Val Val Asp Val Ser Glu Asp Asp Pro Asp Val Gln Ile Ser
   450 455 460
   Trp Phe Val Asn Asn Val Glu Val His Thr Ala Gln Thr Gln Thr His
   465 470 475 480
   Arg Glu Asp Tyr Asn Ser Thr Leu Arg Val Val Ser Ala Leu Pro Ile
   485 490 495
   Gln His Gln Asp Trp Met Ser Gly Lys Glu Phe Lys Cys Lys Val Asn
   500 505 510
   Asn Lys Asp Leu Pro Ala Pro Ile Glu Arg Thr Ile Ser Lys Pro Lys
   515 520 525
   Gly Ser Val Arg Ala Pro Gln Val Tyr Val Leu Pro Pro Pro Glu Glu
   530 535 540
   Glu Met Thr Lys Lys Gln Val Thr Leu Thr Cys Met Val Thr Asp Phe
   545 550 555 560
   Met Pro Glu Asp Ile Tyr Val Glu Trp Thr Asn Asn Gly Lys Thr Glu
   565 570 575
   Leu Asn Tyr Lys Asn Thr Glu Pro Val Leu Asp Ser Asp Gly Ser Tyr
   580 585 590
   Phe Met Tyr Ser Lys Leu Arg Val Glu Lys Lys Asn Trp Val Glu Arg
   595 600 605
   Asn Ser Tyr Ser Cys Ser Val Val His Glu Gly Leu His Asn His His
   610 615 620
   Thr Thr Lys Ser Phe Ser Arg Thr Pro Gly Lys Trp Ser His Pro Gln
   625 630 635 640
   Phe Glu Lys
<210> 74
   <211> 643
   <212> PRT
   <213> Artificial Sequence
<220>
   <223> OKT3 mAb CH ubiquitin 139090
<400> 74
   Met Glu Arg His Trp Ile Phe Leu Leu Leu Leu Ser Val Thr Ala Gly
   1               5                   10                  15
   Val His Ser Gln Val Gln Leu Gln Gln Ser Gly Ala Glu Leu Ala Arg
               20                  25                  30
   Pro Gly Ala Ser Val Lys Met Ser Cys Lys Ala Ser Gly Tyr Thr Phe
           35                  40                  45
   Thr Arg Tyr Thr Met His Trp Val Lys Gln Arg Pro Gly Gln Gly Leu
       50                  55                  60
   Glu Trp Ile Gly Tyr Ile Asn Pro Ser Arg Gly Tyr Thr Asn Tyr Asn
   65                  70                  75                  80
   Gln Lys Phe Lys Asp Lys Ala Thr Leu Thr Thr Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Tyr Tyr Asp Asp His Tyr Cys Leu Asp Tyr Trp
           115                 120                 125
   Gly Gln Gly Thr Thr Leu Thr Val Ser Ser Ala Lys Thr Thr Ala Pro
       130                 135                 140
   Ser Val Tyr Pro Leu Ala Pro Val Cys Gly Asp Thr Thr Gly Ser Ser
   145                 150                 155                 160
   Val Thr Leu Gly Cys Leu Val Lys Gly Tyr Phe Pro Glu Pro Val Thr
                   165                 170                 175
   Leu Thr Trp Asn Ser Gly Ser Leu Ser Ser Gly Val His Thr Phe Pro
               180                 185                 190
   Ala Val Leu Gln Ser Asp Leu Tyr Thr Leu Ser Ser Ser Val Thr Val
           195                 200                 205
   Thr Ser Ser Thr Trp Pro Ser Gln Ser Ile Thr Cys Asn Val Ala His
       210                 215                 220
   Pro Ala Ser Ser Thr Lys Val Asp Lys Lys Ile Glu Pro Arg Gly Pro
   225                 230                 235                 240
   Thr Ile Lys Pro Cys Pro Pro Cys Lys Cys Pro Ala Pro Asn Leu Leu
                   245                 250                 255
   Gly Gly Pro Ser Val Phe Ile Phe Pro Pro Lys Ile Lys Asp Val Leu
               260                 265                 270
   Met Ile Ser Leu Ser Pro Ile Val Thr Cys Val Val Val Asp Val Ser
           275                 280                 285
   Glu Asp Asp Pro Asp Val Gln Ile Ser Trp Phe Val Asn Asn Val Glu
   290 295 300
   Val His Thr Ala Gln Thr Gln Thr His Arg Glu Asp Tyr Asn Ser Thr
   305 310 315 320
   Leu Arg Val Val Ser Ala Leu Pro Ile Gln His Gln Asp Trp Met Ser
   325 330 335
   Gly Lys Glu Phe Lys Cys Lys Val Asn Asn Lys Asp Leu Pro Ala Pro
   340 345 350
   Ile Glu Arg Thr Ile Ser Lys Pro Lys Gly Ser Val Arg Ala Pro Gln
   355 360 365
   Val Tyr Val Leu Pro Pro Pro Glu Glu Glu Met Thr Lys Lys Gln Val
   370 375 380
   Thr Leu Thr Cys Met Val Thr Asp Phe Met Pro Glu Asp Ile Tyr Val
   385 390 395 400
   Glu Trp Thr Asn Asn Gly Lys Thr Glu Leu Asn Tyr Lys Asn Thr Glu
   405 410 415
   Pro Val Leu Asp Ser Asp Gly Ser Tyr Phe Met Tyr Ser Lys Leu Arg
   420 425 430
   Val Glu Lys Lys Asn Trp Val Glu Arg Asn Ser Tyr Ser Cys Ser Val
   435 440 445
   Val His Glu Gly Leu His Asn His His Thr Thr Lys Ser Phe Ser Arg
   450 455 460
   Thr Pro Gly Lys Gly Gly Gly Gly Ser Gly Gly Gly Gly Ser Gly Gly
   465 470 475 480
   Gly Gly Ser Met Gln Ile Phe Val Lys Thr Leu Thr Gly Lys Thr Ile
   485 490 495
   Thr Leu Glu Val Glu Pro Ser Asp Thr Ile Glu Asn Val Lys Ala Lys
   500 505 510
   Ile Gln Asp Lys Glu Gly Ile Pro Pro Asp Gln Gln Arg Leu Ile Phe
   515 520 525
   Ala Gly Lys Gln Leu Glu Asp Gly Arg Thr Leu Ser Asp Tyr Asn Ile
   530 535 540
   Gln Lys Glu Ser Thr Leu His Leu Val Leu Arg Leu Arg Ala Ala Met
   545                 550                 555                 560
   Gln Ile Phe Val Lys Thr Leu Thr Gly Lys Thr Ile Thr Leu Glu Val
                   565                 570                 575
   Glu Pro Ser Asp Thr Ile Glu Asn Val Lys Ala Lys Ile Gln Asp Lys
               580                 585                 590
   Glu Gly Ile Pro Pro Asp Gln Gln Arg Leu Ile Trp Ala Gly Lys Gln
           595                 600                 605
   Leu Glu Asp Gly Arg Thr Leu Ser Asp Tyr Asn Ile Gln Lys Glu Ser
       610                 615                 620
   Thr Leu His Leu Val Leu Arg Leu Arg Ala Ala Trp Ser His Pro Gln
   625                 630                 635                 640
   Phe Glu Lys
<210> 75
   <211> 408
   <212> PRT
   <213> Artificial Sequence
<220>
   <223> OKT3 NL ubiquitin 139090
<400> 75
   Met Asp Phe Gln Val Gln Ile Phe Ser Phe Leu Leu Ile Ser Ala Ser
   1               5                   10                  15
   Val Ile Ile Ser Arg Gly Met Gln Ile Phe Val Lys Thr Leu Thr Gly
               20                  25                  30
   Lys Thr Ile Thr Leu Glu Val Glu Pro Ser Asp Thr Ile Glu Asn Val
           35                  40                  45
   Lys Ala Lys Ile Gln Asp Lys Glu Gly Ile Pro Pro Asp Gln Gln Arg
       50                  55                  60
   Leu Ile Phe Ala Gly Lys Gln Leu Glu Asp Gly Arg Thr Leu Ser Asp
   65                  70                  75                  80
   Tyr Asn Ile Gln Lys Glu Ser Thr Leu His Leu Val Leu Arg Leu Arg
                   85                  90                  95
   Ala Ala Met Gln Ile Phe Val Lys Thr Leu Thr Gly Lys Thr Ile Thr
               100                 105                 110
   Leu Glu Val Glu Pro Ser Asp Thr Ile Glu Asn Val Lys Ala Lys Ile
           115                 120                 125
   Gln Asp Lys Glu Gly Ile Pro Pro Asp Gln Gln Arg Leu Ile Trp Ala
       130                 135                 140
   Gly Lys Gln Leu Glu Asp Gly Arg Thr Leu Ser Asp Tyr Asn Ile Gln
   145                 150                 155                 160
   Lys Glu Ser Thr Leu His Leu Val Leu Arg Leu Arg Ala Ala Gly Gly
                   165                 170                 175
   Gly Gly Ser Gly Gly Gly Gly Ser Gly Gly Gly Gly Ser Gln Ile Val
               180                 185                 190
   Leu Thr Gln Ser Pro Ala Ile Met Ser Ala Ser Pro Gly Glu Lys Val
           195                 200                 205
   Thr Met Thr Cys Ser Ala Ser Ser Ser Val Ser Tyr Met Asn Trp Tyr
       210                 215                 220
   Gln Gln Lys Ser Gly Thr Ser Pro Lys Arg Trp Ile Tyr Asp Thr Ser
   225                 230                 235                 240
   Lys Leu Ala Ser Gly Val Pro Ala His Phe Arg Gly Ser Gly Ser Gly
                   245                 250                 255
   Thr Ser Tyr Ser Leu Thr Ile Ser Gly Met Glu Ala Glu Asp Ala Ala
               260                 265                 270
   Thr Tyr Tyr Cys Gln Gln Trp Ser Ser Asn Pro Phe Thr Phe Gly Ser
           275                 280                 285
   Gly Thr Lys Leu Glu Ile Asn Arg Ala Asp Thr Ala Pro Thr Val Ser
       290                 295                 300
   Ile Phe Pro Pro Ser Ser Glu Gln Leu Thr Ser Gly Gly Ala Ser Val
   305                 310                 315                 320
   Val Cys Phe Leu Asn Asn Phe Tyr Pro Lys Asp Ile Asn Val Lys Trp
                   325                 330                 335
   Lys Ile Asp Gly Ser Glu Arg Gln Asn Gly Val Leu Asn Ser Trp Thr
               340                 345                 350
   Asp Gln Asp Ser Lys Asp Ser Thr Tyr Ser Met Ser Ser Thr Leu Thr
           355                 360                 365
   Leu Thr Lys Asp Glu Tyr Glu Arg His Asn Ser Tyr Thr Cys Glu Ala
       370                 375                 380
   Thr His Lys Thr Ser Thr Ser Pro Ile Val Lys Ser Phe Asn Arg Asn
   385                 390                 395                 400
   Glu Cys His His His His His His
                   405
<210> 76
   <211> 408
   <212> PRT
   <213> Artificial Sequence
<220>
   <223> OKT3 CL ubiquitin 139090
<400> 76
   Met Asp Phe Gln Val Gln Ile Phe Ser Phe Leu Leu Ile Ser Ala Ser
   1               5                   10                  15
   Val Ile Ile Ser Arg Gly Gln Ile Val Leu Thr Gln Ser Pro Ala Ile
               20                  25                  30
   Met Ser Ala Ser Pro Gly Glu Lys Val Thr Met Thr Cys Ser Ala Ser
           35                  40                  45
   Ser Ser Val Ser Tyr Met Asn Trp Tyr Gln Gln Lys Ser Gly Thr Ser
       50                  55                  60
   Pro Lys Arg Trp Ile Tyr Asp Thr Ser Lys Leu Ala Ser Gly Val Pro
   65                  70                  75                  80
   Ala His Phe Arg Gly Ser Gly Ser Gly Thr Ser Tyr Ser Leu Thr Ile
                   85                  90                  95
   Ser Gly Met Glu Ala Glu Asp Ala Ala Thr Tyr Tyr Cys Gln Gln Trp
               100                 105                 110
   Ser Ser Asn Pro Phe Thr Phe Gly Ser Gly Thr Lys Leu Glu Ile Asn
           115                 120                 125
   Arg Ala Asp Thr Ala Pro Thr Val Ser Ile Phe Pro Pro Ser Ser Glu
       130                 135                 140
   Gln Leu Thr Ser Gly Gly Ala Ser Val Val Cys Phe Leu Asn Asn Phe
   145 150 155 160
   Tyr Pro Lys Asp Ile Asn Val Lys Trp Lys Ile Asp Gly Ser Glu Arg
   165 170 175
   Gln Asn Gly Val Leu Asn Ser Trp Thr Asp Gln Asp Ser Lys Asp Ser
   180 185 190
   Thr Tyr Ser Met Ser Ser Thr Leu Thr Leu Thr Lys Asp Glu Tyr Glu
   195 200 205
   Arg His Asn Ser Tyr Thr Cys Glu Ala Thr His Lys Thr Ser Thr Ser
   210 215 220
   Pro Ile Val Lys Ser Phe Asn Arg Asn Glu Cys Gly Gly Gly Gly Ser
   225 230 235 240
   Gly Gly Gly Gly Ser Gly Gly Gly Gly Ser Met Gln Ile Phe Val Lys
   245 250 255
   Thr Leu Thr Gly Lys Thr Ile Thr Leu Glu Val Glu Pro Ser Asp Thr
   260 265 270
   Ile Glu Asn Val Lys Ala Lys Ile Gln Asp Lys Glu Gly Ile Pro Pro
   275 280 285
   Asp Gln Gln Arg Leu Ile Phe Ala Gly Lys Gln Leu Glu Asp Gly Arg
   290 295 300
   Thr Leu Ser Asp Tyr Asn Ile Gln Lys Glu Ser Thr Leu His Leu Val
   305 310 315 320
   Leu Arg Leu Arg Ala Ala Met Gln Ile Phe Val Lys Thr Leu Thr Gly
   325 330 335
   Lys Thr Ile Thr Leu Glu Val Glu Pro Ser Asp Thr Ile Glu Asn Val
   340 345 350
   Lys Ala Lys Ile Gln Asp Lys Glu Gly Ile Pro Pro Asp Gln Gln Arg
   355 360 365
   Leu Ile Trp Ala Gly Lys Gln Leu Glu Asp Gly Arg Thr Leu Ser Asp
   370 375 380
   Tyr Asn Ile Gln Lys Glu Ser Thr Leu His Leu Val Leu Arg Leu Arg
   385 390 395 400
   Ala Ala His His His His His His
   405
<210> 77
   <211> 413
   <212> PRT
   <213> Artificial Sequence
<220>
   <223> OKT3 Fab NH 142628
<400> 77
   Met Glu Arg His Trp Ile Phe Leu Leu Leu Leu Ser Val Thr Ala Gly
   1               5                   10                  15
   Val His Ser Met Gln Ile Phe Val Lys Thr Leu Thr Gly Lys Thr Ile
               20                  25                  30
   Thr Leu Glu Val Glu Pro Ser Asp Thr Thr Glu Asn Val Lys Ala Lys
           35                  40                  45
   Ile Gln Asp Lys Glu Gly Ile Pro Pro Asp Gln Gln Thr Leu Ala Phe
       50                  55                  60
   Val Gly Lys Gln Leu Glu Asp Gly Arg Thr Leu Ser Asp Tyr Asn Ile
   65                  70                  75                  80
   Gln Lys Glu Ser Thr Leu Trp Leu Tyr Leu Thr Trp Tyr Ala Ala Met
                   85                  90                  95
   Arg Ile Phe Val Thr Thr His Thr Gly Lys Thr Ile Thr Leu Asp Val
               100                 105                 110
   Glu Pro Ser Asp Thr Ile Glu Asn Val Lys Ala Lys Ile Gln Asp Lys
           115                 120                 125
   Glu Gly Ile Pro Pro Asp Gln Gln Arg Leu Ile Trp Ala Gly Lys Gln
       130                 135                 140
   Leu Glu Asp Gly Arg Thr Leu Ser Asp Tyr Asn Ile Ser Glu Trp Ala
   145                 150                 155                 160
   Ile Leu His Leu Val Leu Arg Leu Arg Ala Ala Gly Gly Gly Gly Ser
                   165                 170                 175
   Gly Gly Gly Gly Ser Gly Gly Gly Gly Ser Gln Val Gln Leu Gln Gln
               180                 185                 190
   Ser Gly Ala Glu Leu Ala Arg Pro Gly Ala Ser Val Lys Met Ser Cys
                   195                 200                 205
   Lys Ala Ser Gly Tyr Thr Phe Thr Arg Tyr Thr Met His Trp Val Lys
       210                 215                 220
   Gln Arg Pro Gly Gln Gly Leu Glu Trp Ile Gly Tyr Ile Asn Pro Ser
   225                 230                 235                 240
   Arg Gly Tyr Thr Asn Tyr Asn Gln Lys Phe Lys Asp Lys Ala Thr Leu
                   245                 250                 255
   Thr Thr Asp Lys Ser Ser Ser Thr Ala Tyr Met Gln Leu Ser Ser Leu
               260                 265                 270
   Thr Ser Glu Asp Ser Ala Val Tyr Tyr Cys Ala Arg Tyr Tyr Asp Asp
           275                 280                 285
   His Tyr Cys Leu Asp Tyr Trp Gly Gln Gly Thr Thr Leu Thr Val Ser
       290                 295                 300
   Ser Ala Lys Thr Thr Ala Pro Ser Val Tyr Pro Leu Ala Pro Val Cys
   305                 310                 315                 320
   Gly Asp Thr Thr Gly Ser Ser Val Thr Leu Gly Cys Leu Val Lys Gly
                   325                 330                 335
   Tyr Phe Pro Glu Pro Val Thr Leu Thr Trp Asn Ser Gly Ser Leu Ser
               340                 345                 350
   Ser Gly Val His Thr Phe Pro Ala Val Leu Gln Ser Asp Leu Tyr Thr
           355                 360                 365
   Leu Ser Ser Ser Val Thr Val Thr Ser Ser Thr Trp Pro Ser Gln Ser
       370                 375                 380
   Ile Thr Cys Asn Val Ala His Pro Ala Ser Ser Thr Lys Val Asp Lys
   385                 390                 395                 400
   Lys Ile Glu Pro Arg Trp Ser His Pro Gln Phe Glu Lys
                   405                 410
<210> 78
   <211> 413
   <212> PRT
   <213> Artificial Sequence
<220>
   <223> OKT3 Fab CH 142628
<400> 78
   Met Glu Arg His Trp Ile Phe Leu Leu Leu Leu Ser Val Thr Ala Gly
   1 5 10 15
   Val His Ser Gln Val Gln Leu Gln Gln Ser Gly Ala Glu Leu Ala Arg
   20 25 30
   Pro Gly Ala Ser Val Lys Met Ser Cys Lys Ala Ser Gly Tyr Thr Phe
   35 40 45
   Thr Arg Tyr Thr Met His Trp Val Lys Gln Arg Pro Gly Gln Gly Leu
   50 55 60
   Glu Trp Ile Gly Tyr Ile Asn Pro Ser Arg Gly Tyr Thr Asn Tyr Asn
   65 70 75 80
   Gln Lys Phe Lys Asp Lys Ala Thr Leu Thr Thr Asp Lys Ser Ser Ser
   85 90 95
   Thr Ala Tyr Met Gln Leu Ser Ser Leu Thr Ser Glu Asp Ser Ala Val
   100 105 110
   Tyr Tyr Cys Ala Arg Tyr Tyr Asp Asp His Tyr Cys Leu Asp Tyr Trp
   115 120 125
   Gly Gln Gly Thr Thr Leu Thr Val Ser Ser Ala Lys Thr Thr Ala Pro
   130 135 140
   Ser Val Tyr Pro Leu Ala Pro Val Cys Gly Asp Thr Thr Gly Ser Ser
   145 150 155 160
   Val Thr Leu Gly Cys Leu Val Lys Gly Tyr Phe Pro Glu Pro Val Thr
   165 170 175
   Leu Thr Trp Asn Ser Gly Ser Leu Ser Ser Gly Val His Thr Phe Pro
   180 185 190
   Ala Val Leu Gln Ser Asp Leu Tyr Thr Leu Ser Ser Ser Val Thr Val
   195 200 205
   Thr Ser Ser Thr Trp Pro Ser Gln Ser Ile Thr Cys Asn Val Ala His
   210 215 220
   Pro Ala Ser Ser Thr Lys Val Asp Lys Lys Ile Glu Pro Arg Gly Gly
   225 230 235 240
   Gly Gly Ser Gly Gly Gly Gly Ser Gly Gly Gly Gly Ser Met Gln Ile
   245 250 255
   Phe Val Lys Thr Leu Thr Gly Lys Thr Ile Thr Leu Glu Val Glu Pro
   260 265 270
   Ser Asp Thr Thr Glu Asn Val Lys Ala Lys Ile Gln Asp Lys Glu Gly
   275 280 285
   Ile Pro Pro Asp Gln Gln Thr Leu Ala Phe Val Gly Lys Gln Leu Glu
   290 295 300
   Asp Gly Arg Thr Leu Ser Asp Tyr Asn Ile Gln Lys Glu Ser Thr Leu
   305 310 315 320
   Trp Leu Tyr Leu Thr Trp Tyr Ala Ala Met Arg Ile Phe Val Thr Thr
   325 330 335
   His Thr Gly Lys Thr Ile Thr Leu Asp Val Glu Pro Ser Asp Thr Ile
   340 345 350
   Glu Asn Val Lys Ala Lys Ile Gln Asp Lys Glu Gly Ile Pro Pro Asp
   355 360 365
   Gln Gln Arg Leu Ile Trp Ala Gly Lys Gln Leu Glu Asp Gly Arg Thr
   370 375 380
   Leu Ser Asp Tyr Asn Ile Ser Glu Trp Ala Ile Leu His Leu Val Leu
   385 390 395 400
   Arg Leu Arg Ala Ala Trp Ser His Pro Gln Phe Glu Lys
   405 410
<210> 79
   <211> 413
   <212> PRT
   <213> Artificial Sequence
<220>
   <223> OKT3 Fab NH ubiquitin 139090
<400> 79
   Met Glu Arg His Trp Ile Phe Leu Leu Leu Leu Ser Val Thr Ala Gly
   1               5                   10                  15
   Val His Ser Met Gln Ile Phe Val Lys Thr Leu Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Arg Leu Ile Phe
       50                  55                  60
   Ala Gly Lys Gln Leu Glu Asp Gly Arg Thr Leu Ser Asp Tyr Asn Ile
   65                  70                  75                  80
   Gln Lys Glu Ser Thr Leu His Leu Val Leu Arg Leu Arg Ala Ala Met
                   85                  90                  95
   Gln Ile Phe Val Lys Thr Leu Thr Gly Lys Thr Ile Thr Leu Glu Val
               100                 105                 110
   Glu Pro Ser Asp Thr Ile Glu Asn Val Lys Ala Lys Ile Gln Asp Lys
           115                 120                 125
   Glu Gly Ile Pro Pro Asp Gln Gln Arg Leu Ile Trp Ala Gly Lys Gln
       130                 135                 140
   Leu Glu Asp Gly Arg Thr Leu Ser Asp Tyr Asn Ile Gln Lys Glu Ser
   145                 150                 155                 160
   Thr Leu His Leu Val Leu Arg Leu Arg Ala Ala Gly Gly Gly Gly Ser
                   165                 170                 175
   Gly Gly Gly Gly Ser Gly Gly Gly Gly Ser Gln Val Gln Leu Gln Gln
               180                 185                 190
   Ser Gly Ala Glu Leu Ala Arg Pro Gly Ala Ser Val Lys Met Ser Cys
           195                 200                 205
   Lys Ala Ser Gly Tyr Thr Phe Thr Arg Tyr Thr Met His Trp Val Lys
       210                 215                 220
   Gln Arg Pro Gly Gln Gly Leu Glu Trp Ile Gly Tyr Ile Asn Pro Ser
   225                 230                 235                 240
   Arg Gly Tyr Thr Asn Tyr Asn Gln Lys Phe Lys Asp Lys Ala Thr Leu
                   245                 250                 255
   Thr Thr Asp Lys Ser Ser Ser Thr Ala Tyr Met Gln Leu Ser Ser Leu
               260                 265                 270
   Thr Ser Glu Asp Ser Ala Val Tyr Tyr Cys Ala Arg Tyr Tyr Asp Asp
           275                 280                 285
   His Tyr Cys Leu Asp Tyr Trp Gly Gln Gly Thr Thr Leu Thr Val Ser
           290                 295                 300
   Ser Ala Lys Thr Thr Ala Pro Ser Val Tyr Pro Leu Ala Pro Val Cys
   305                 310                 315                 320
   Gly Asp Thr Thr Gly Ser Ser Val Thr Leu Gly Cys Leu Val Lys Gly
                   325                 330                 335
   Tyr Phe Pro Glu Pro Val Thr Leu Thr Trp Asn Ser Gly Ser Leu Ser
               340                 345                 350
   Ser Gly Val His Thr Phe Pro Ala Val Leu Gln Ser Asp Leu Tyr Thr
           355                 360                 365
   Leu Ser Ser Ser Val Thr Val Thr Ser Ser Thr Trp Pro Ser Gln Ser
       370                 375                 380
   Ile Thr Cys Asn Val Ala His Pro Ala Ser Ser Thr Lys Val Asp Lys
   385                 390                 395                 400
   Lys Ile Glu Pro Arg Trp Ser His Pro Gln Phe Glu Lys
                   405                 410
<210> 80
   <211> 413
   <212> PRT
   <213> Artificial Sequence
<220>
   <223> OKT3 Fab CH ubiquitin 139090
<400> 80
   Met Glu Arg His Trp Ile Phe Leu Leu Leu Leu Ser Val Thr Ala Gly
   1               5                   10                  15
   Val His Ser Gln Val Gln Leu Gln Gln Ser Gly Ala Glu Leu Ala Arg
               20                  25                  30
   Pro Gly Ala Ser Val Lys Met Ser Cys Lys Ala Ser Gly Tyr Thr Phe
           35                  40                  45
   Thr Arg Tyr Thr Met His Trp Val Lys Gln Arg Pro Gly Gln Gly Leu
       50                  55                  60
   Glu Trp Ile Gly Tyr Ile Asn Pro Ser Arg Gly Tyr Thr Asn Tyr Asn
   65                  70                  75                  80
   Gln Lys Phe Lys Asp Lys Ala Thr Leu Thr Thr Asp Lys Ser Ser Ser
                   85                  90                  95
   Thr Ala Tyr Met Gln Leu Ser Ser Leu Thr Ser Glu Asp Ser Ala Val
   100 105 110
   Tyr Tyr Cys Ala Arg Tyr Tyr Asp Asp His Tyr Cys Leu Asp Tyr Trp
   115 120 125
   Gly Gln Gly Thr Thr Leu Thr Val Ser Ser Ala Lys Thr Thr Ala Pro
   130 135 140
   Ser Val Tyr Pro Leu Ala Pro Val Cys Gly Asp Thr Thr Gly Ser Ser
   145 150 155 160
   Val Thr Leu Gly Cys Leu Val Lys Gly Tyr Phe Pro Glu Pro Val Thr
   165 170 175
   Leu Thr Trp Asn Ser Gly Ser Leu Ser Ser Gly Val His Thr Phe Pro
   180 185 190
   Ala Val Leu Gln Ser Asp Leu Tyr Thr Leu Ser Ser Ser Val Thr Val
   195 200 205
   Thr Ser Ser Thr Trp Pro Ser Gln Ser Ile Thr Cys Asn Val Ala His
   210 215 220
   Pro Ala Ser Ser Thr Lys Val Asp Lys Lys Ile Glu Pro Arg Gly Gly
   225 230 235 240
   Gly Gly Ser Gly Gly Gly Gly Ser Gly Gly Gly Gly Ser Met Gln Ile
   245 250 255
   Phe Val Lys Thr Leu Thr Gly Lys Thr Ile Thr Leu Glu Val Glu Pro
   260 265 270
   Ser Asp Thr Ile Glu Asn Val Lys Ala Lys Ile Gln Asp Lys Glu Gly
   275 280 285
   Ile Pro Pro Asp Gln Gln Arg Leu Ile Phe Ala Gly Lys Gln Leu Glu
   290 295 300
   Asp Gly Arg Thr Leu Ser Asp Tyr Asn Ile Gln Lys Glu Ser Thr Leu
   305 310 315 320
   His Leu Val Leu Arg Leu Arg Ala Ala Met Gln Ile Phe Val Lys Thr
   325 330 335
   Leu Thr Gly Lys Thr Ile Thr Leu Glu Val Glu Pro Ser Asp Thr Ile
                       340                 345                 350
   Glu Asn Val Lys Ala Lys Ile Gln Asp Lys Glu Gly Ile Pro Pro Asp
           355                 360                 365
   Gln Gln Arg Leu Ile Trp Ala Gly Lys Gln Leu Glu Asp Gly Arg Thr
       370                 375                 380
   Leu Ser Asp Tyr Asn Ile Gln Lys Glu Ser Thr Leu His Leu Val Leu
   385                 390                 395                 400
   Arg Leu Arg Ala Ala Trp Ser His Pro Gln Phe Glu Lys
                   405                 410

## Description

### Field of the invention

The present invention relates to new binding molecules comprising a ubiquitin mutein (Affilin®) and a monoclonal antibody or antibody fragments. The invention refers to bispecific and/or bivalent binding proteins optionally fused to a pharmacokinetic moiety modulating serum half-life or to a therapeutically or diagnostically active component. The invention further relates to the use of these binding proteins in medicine, preferably for use in the treatment of cancer or autoimmune disorders.

### Background

Antibodies are widely used as binding proteins in different areas such as treatment of diseases or diagnostic applications. Most antibodies are complex molecules comprising two light chains and two heavy chains which combine to form two identical binding sites, i.e. an antibody is bivalent but monospecific and its size is about 150 kDa.

In many medical applications, a bispecific binding molecule is desired. Bispecific binding molecules which can address two targets at the same time might enable innovative treatment modalities that are not possible using conventional antibodies. Thereby, the natural core structure of antibodies is maintained but functionality is extended to result in bispecific, multivalent binding proteins. Furthermore, bispecific molecules can be used to bring the respective target substances in spatial proximity to each other.

Although methods exist to engineer antibodies to obtain bispecific molecules, these approaches are complex and often require the combination of two different heavy and two different light chains resulting in molecules with undesired biophysical properties, low stability, or low expression. The limitations of pairing different heavy and light chains are inefficient processes often leading to heterogeneous and instable products preventing commercialization. There is a large medical need for novel bispecific molecules which are suitable for diagnostic and therapeutic applications.

An alternative approach to bispecific antibody formats is the fusion of antibodies or antibody fragments with small non-antibody-based binding proteins. However, there are still major drawbacks, for example suboptimal physicochemical properties or low production yields of those molecules. Thus, there is an ongoing need for novel molecules with improved formats and with drug-like properties, such as high specificities for given target antigens, favorable physicochemical properties (such as solubility and stability), long serum half-life, and high-level expression, and easy production methods.

Accordingly, one objective of the present invention is the provision of binding molecules for new and improved strategies in the treatment and diagnosis of various diseases, including cancer and inflammatory diseases. In particular, it is an object to provide novel binding proteins which have high affinity to epitopes of target antigens which are involved in many diseases.

Further objectives of the invention are the provision of binding proteins with binding specificity to cancer cells characterized by specific tumor-associated antigens.

Among non-immunoglobulin-derived small binding proteins, molecules based on ubiquitin muteins are particularly interesting. Ubiquitin is a highly conserved, small, monomeric protein present in all known eukaryotic cells and is 100 % conserved amongst all vertebrates. In addition, ubiquitin naturally occurs in serum lowering the immunogenic potential. This facilitates preclinical development in different species required for toxicological and efficacy studies. Ubiquitin muteins known as Affilin® molecules are specific for target antigens. Affilin proteins are engineered proteins with *de novo* binding affinity towards desired targets well-suited for several applications.

Said objectives of the present invention are solved by providing binding proteins comprising Affilin molecules with binding specificity to a first epitope fused to a second binding protein selected from a monoclonal antibody or a fragment thereof with binding specificity to a second epitope wherein the first and the second epitopes are different.

This approach provides *inter alia* key differentiators and advantages compared to conventional antibody technologies.

The above-described objectives are solved and the advantages are achieved by the subject-matters of the enclosed independent claims. Preferred embodiments of the invention are included in the dependent claims as well as in the following description, examples and figures. The above overview does not necessarily describe all problems solved by the present invention.

Friedman et al. (Biotechnology and Applied Biochemistry, 2009, Vol. 54(2):121-131) relates to the fusion of affibody molecules, and describes a bivalent HER2-binding affibody molecule fused to a bivalent EGFR-binding affibody molecule, resulting in a construct (Z_{HER2})₂-linker-(Z_{EGFR})₂.

EP 2 829 552 A1 relates to chimeric molecules of DARPins and antibodies, and describes the generation of anti-EGFR DARPins and bispecific fusions thereof with anti-c-Met or anti-HER2/3 antibodies.

Lorey et al. (Journal of Biological Chemistry, 2014, Vol. 289(12):8493-8507) describes ubiquitin muteins as high affinity binding proteins.

EP 2 727 942 A1 relates to antibodies with dual specificity cross-reactive with ErbB family members, and describes the generation of a bispecific antibody by immunizing a rabbit with the antigens EGFR (HER1), HER2 and HER3. Weidle et al. (Cancer Genomics & Proteomics, 2013, Vol. 10(4):155-168) is a review article discussing protein-based scaffold structures as antibody mimetics.

EP 1 591 527 A1 describes a single chain diabody cross-linking PD-1 and CD3 generated from a fusion protein combining the VH and VH domains of an anti-PD-1 and an anti-CD3 antibody.

### Summary of the invention

In a first aspect, the present invention relates to a bispecific and/or bivalent binding protein comprising at least a first binding protein wherein the first binding protein is a ubiquitin mutein with specific binding affinity (K_{D}) of less than 700 nM to a first epitope and wherein the ubiquitin mutein has a sequence identity of at least 80 % to the ubiquitin amino acid sequence defined by SEQ ID NOs: 1 to 4, and at least a second binding protein wherein the second binding protein is a monoclonal antibody or a fragment thereof, each with binding specificity to a second epitope, and optionally a linker between the first binding protein and the second binding protein.

In a second aspect, the present invention relates to a binding protein as defined above wherein the first binding protein is linked to the heavy chain or to the light chain of the second binding protein, preferably wherein the first binding protein is linked to the C-terminus of the light chain of the monoclonal antibody or a fragment thereof, or the first binding protein is linked to the C-terminus of the heavy chain of the monoclonal antibody or a fragment thereof, or the first binding protein is linked to the N-terminus of the light chain of the monoclonal antibody or a fragment thereof, or the first binding protein is linked to the N-terminus of the heavy chain of the a monoclonal antibody or a fragment thereof, or a combination thereof.

In a third aspect, the present invention relates to the first binding protein and the second binding protein which have specific binding affinity to an epitope on a target antigen selected from at least one member of the group consisting of a cancer target antigen, a receptor target antigen on immune cells, or a soluble target antigen selected from polypeptides, hormones or cytokines, wherein the first and the second binding protein bind to different epitopes on said target antigens and wherein said epitopes are located on one or two different target antigens.

A further aspect of the present invention relates to a binding protein wherein the first binding protein comprises a ubiquitin mutein with specific binding affinity (K_{D}) of less than 700 nM to EGFR or HER2 and wherein the second binding protein comprises or consists of a monoclonal antibody or fragment thereof with binding specificity to EGFR. In particular, fusion proteins comprising an EGFR-specific Affilin and an EGFR-monoclonal antibody (referred to as Mabfilin EGFR/EGFR herein) or an EGFR-specific Affilin and an EGFR-Fab fragment (referred to as Fabfilin EGFR/EGFR herein) or fusion proteins comprising an HER2-specific Affilin and an EGFR-monoclonal antibody

(referred to as Mabfilin HER2/EGFR) or an HER2-specific Affilin and EGFR-Fab fragment (referred to as Fabfilin HER2/EGFR) are described herein. In addition, fusion proteins comprising a PD1-specific Affilin and a CD3-monoclonal antibody (referred to as Mabfilin PD1/CD3) or a PD1-specific Affilin and a CD3-Fab fragment are described herein (referred to as Fabfilin PD1/CD3). In addition, fusion proteins comprising an HER2-specific Affilin and a CD3-monoclonal antibody (referred to as Mabfilin HER2/CD3) or an HER2-specific Affilin and a CD3-Fab fragment (referred to as Fabfilin HER2/CD3) are described herein.

Another aspect of the present invention relates to a binding protein further comprising at least one additional protein or molecule, preferably further comprising a further binding protein, particularly an Affilin binding protein or a monoclonal antibody.

The present invention also relates, in further aspects, to a nucleic acid or nucleic acids encoding the bispecific and/or bivalent construct of the present invention, as well as a vector or vectors comprising said nucleic acid or nucleic acids, and a host cell or host cells comprising said vector or vectors.

Another aspect of the present invention relates to a method for the production of a binding protein as discussed above that specifically binds to a pre-defined antigen comprising culturing of a host cell under suitable conditions in order to obtain said binding protein and optionally isolating said binding protein.

This summary does not necessarily describe all features of the present invention, which is defined by the appended claims. Other aspects will become apparent from a review of the ensuing detailed description.

### Brief description of the Figures

Figure 1 shows schematic drawings of binding proteins comprising a ubiquitin mutein (Affilin®) and a monoclonal antibody ("Mabfilin") or an antibody fragment ("Fabfilin"); a first binding protein (small circle) joined to the heavy chain (dark grey rectangle) or to the light chain (light grey rectangle) of a second binding protein.
Figure 1A shows binding proteins comprising an Affilin linked to a monoclonal antibody at the N-terminus of the heavy chain, at the N-terminus of the light chain, at the C-terminus of the heavy chain, or at the C-terminus of the light chain of a monoclonal antibody.
Figure 1B shows combinations of binding proteins shown in Figure 1A wherein Affilin is linked to the N-terminus of the heavy chain of a monoclonal antibody and to the N-terminus of the light chain of a monoclonal antibody; to the C-terminus of the heavy chain and to the N-terminus of the light chain; to the C-terminus of the heavy chain and to the C-terminus of the light chain; or to the N-terminus of the heavy chain and to the C-terminus of the light chain.
Figure 1C shows binding proteins comprising an Affilin with an antibody Fab-Fragment: Affilin is linked to the N-terminus of the heavy chain of a Fab-fragment; to the C-terminus of the heavy chain; to the N-terminus of the light chain; or to the C-terminus of the light chain.
Figure 1D shows binding proteins comprising an Affilin with an antibody Fab-Fragment: Affilin is linked to the N-terminus of the heavy chain and to the N-terminus of the light chain of a Fab-fragment; to the C-terminus of the heavy chain and to the C-terminus of the light chain; to the N-terminus of the light chain and to the C-terminus of the heavy chain; to the N-terminus of the heavy chain and to the C-terminus of the light chain.
Figure 2 illustrates the general cloning strategy for binding proteins of the invention. Arrows refer to sites for restriction endonucleases.
Figure 3 shows a SE-HPLC profile of a Mabfilin EGFR/EGFR protein (PID 13).
Figure 4 shows RP-HPLCprofile of Mabfilin EGFR/EGFR protein (PID 22).
Figure 5 shows a SE-HPLC profile of Fabfilin EGFR/EGFR (PID 55)
Figure 6 shows a RP-HPLC profile of Fabfilin HER2/EGFR (PID 50).
Figure 7 shows a RP-HPLC profile of Fabfilin PD1/CD3 protein (PID 46)
Figure 8 shows an SE-HPLC profile of Fabfilin HER2/CD3 (PID 79)
Figure 9 shows EGFR binding of different Mabfilins EGFR/EGFR- Figure 9A compares the EGFR binding of Mabfilin EGFR/EGFR (dark grey line, PID 22) to Cetuximab (light grey line, PID 5) and to a control fusion protein with ubiquitin (medium grey line, PID 28)(SPR analysis). Figure 9B shows the measurement of binding-rate values of a complex Mabfilin EGFR/EGFR (PID 35).
Figure 10 shows the simultaneous binding of Mabfilin HER2/EGFR proteins (PID 23, PID 24, PID 25, PID 26) to EGFR and HER2.
Figure 11 shows the simultaneous binding of Fabfilin HER2/EGFR (PID 52) to HER2 and EGFR. PID 52 was injected to a HER2-coupled chip followed by injection of EGFR in different concentrations..
Figure 12 shows the simultaneous binding of Fabfilin PD1/CD3 (PID 46) to PD1 and CD3. 200 nM PID 46 was injected to a PD1-coupled chip followed by injection of CD3 in different concentrations.
Figure 13 shows the simultaneous binding of Mabfilin HER2/CD3 (PID 71) to HER2 and CD3. PID 71 was injected to a HER2-coupled chip followed by injection of CD3 in different concentrations.

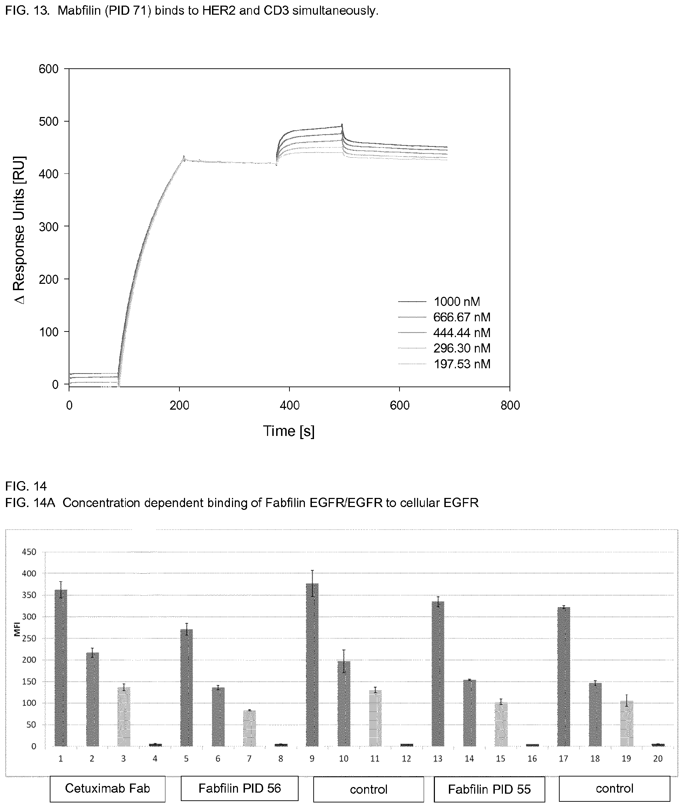

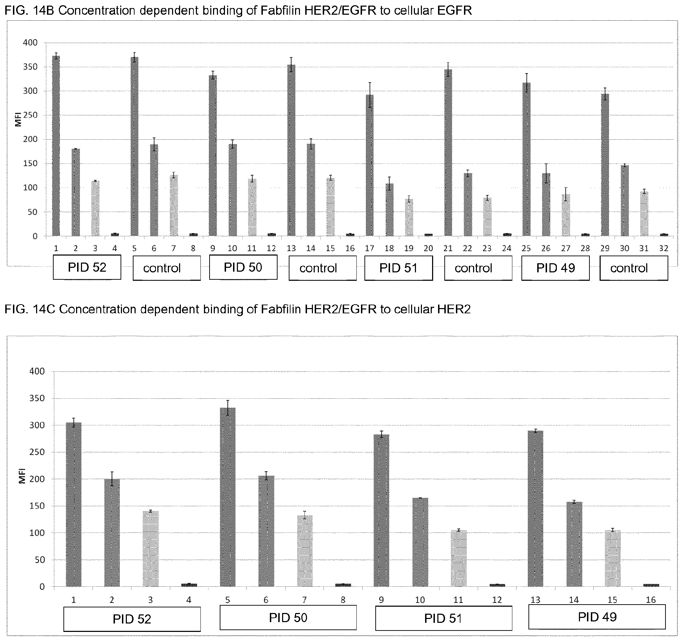

Figure 14 shows concentration dependent binding of Fabfilin proteins to EGFR or HER2 overexpressing cells, Figure 14A Fabfilin EGFR/EGFR binding to EGFR overexpressing cells, Figure 14B. Fabfilin HER2/EGFR binding to EGFR overexpressing cells. Figure 14C. Fabfilin HER2/EGFR binding to HER2 overexpressing cells. Figure 14A. Every quartet shows binding of a Fabfilin and control proteins in different concentrations (10 nM, 1 nM, 0.5 nM) to EGFR overexpressing CHO K1 cell line or to non EGFR expressing CHO K1 cell line for negative control. Column 1-4: Cetuximab Fab (PID 48); column 5-8: PID 56, column 9-12: control fusion protein with ubiquitin (PID 64); column 13-16: PID 55; column 17-20: control fusion protein with ubiquitin (PID 63). Figure 14B. Every quartet shows binding of Fabfilins HER2/EGFR and control fusion proteins in different concentrations (10 nM, 1 nM, 0.5 nM) to EGFR overexpressing CHO K1 cell line or to non EGFR expressing CHO K1 cell line. Column 1-4: PID 52; column 5-8: control with ubiquitin PID 60, column 9-12: PID 50; column 13-16: control with ubiquitin PID 58; column 17-20: PID 51; column 21-24: control with ubiquitin PID 59; column 25-28: PID 49; column 29-32: control with ubiquitin PID 57.
Figure 14C. Every quartet shows binding of Fabfilins in different concentrations (50 nM, 10 nM, 5 nM) to HER2 overexpressing CHO K1 cell line or to non HER2 expressing CHO K1 cell line. Column 1-4: PID 52; column 5-8: PID 50, column 9-12: PID 51; column 13-16: PID 49. Control fusion proteins with ubiquitin showed no binding to HER2 expressing cell lines.
Figure 15 shows concentration dependent binding of Fabfilin PD1/CD3 to cellular target protein. Figure 15A. PD-1 overexpressing HeLa cells, Figure 15B. CD3 expressing cells (T-cells), columns 1-3, CD3 specific Fab fragment (PID 38); columns 4-6: Fabfilin PD1/CD3 (PID 46); columns 7-9: Affilin-128187, column 10: negative control. concentrations of 400 nM (column 1, 4, 7), 40 nM (column 2, 5, 8), and 4 nM (column 3, 6, 9).

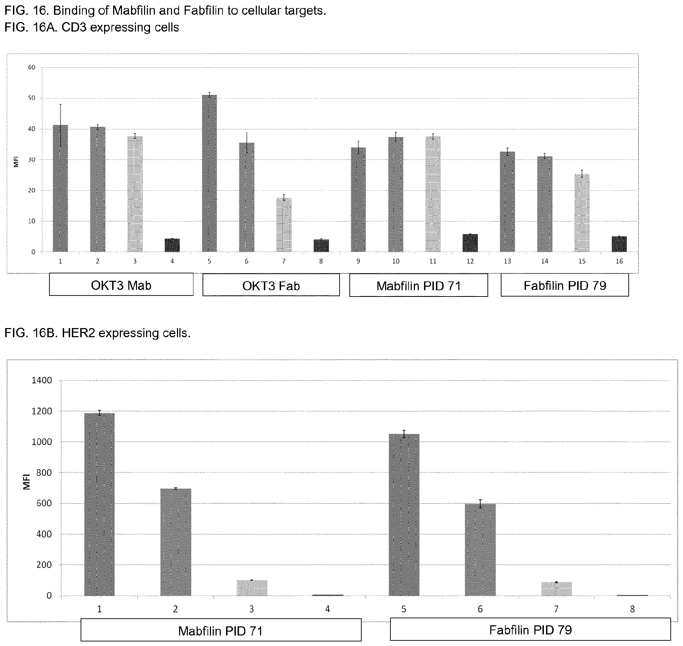

Figure 16 shows binding of Mabfilin HER2/CD3 and Fabfilin HER2/CD3 to their cellular target proteins. Figure 16A. binding to CD3 expressing Jurkat cells. Shown are different concentrations of binding protein (100 nM, medium grey bar; 33.3 nM, light grey bar; binding to K562 cell line (CD3 negative; black bar). Column 1-4: OKT3 mAb (PID 66); column 5-8: OKT3 Fab (PID 67), column 9-12: Mabfilin (PID 71); column 13-16: Fabfilin (PID 79). Figure 16B. binding to HER2 overexpressing CHO K1 cells at concentration 50 nM (columns 1 and 5), 10 nM (columns 2 and 6), and 1 nM (columns 3 and 7). No binding is observed to non HER2 expressing cell line (columns 4 and 8). Column 1-4: Mabfilin (fusion to light chain PID 71); column 5-8: (Fabfilin, fusion to light chain PID 79). MFI, median fluorescence intensity.
Figure 17 shows inhibition of proliferation of EGFR overexpressing cell line A431 by Mabfilin EGFR/EGFR (Figure 17A) (fusion to light chain PID 22, control with ubiquitin, PID 28, Cetuximab, PID 5), Fabfilin EGFR/EGFR (Figure 17B)(fusions to light chain PID 56 and PID 55, control fusion proteins PID 64 and PID 63, Cetuximab Fab, PID 48)

### Detailed description of the invention

Before the present invention is described in more detail below, it is to be understood that the present specification is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art to which this invention belongs.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variants such as "comprises" and "comprising", will be understood to imply the inclusion of a stated integer or step or group of integers or steps but not the exclusion of any other integer or step or group of integers or steps. Several documents (for example: patents, patent applications, scientific publications, manufacturer's specifications, instructions, GenBank Accession Number sequence submissions etc.) are cited throughout the text of this application. Nothing herein is to be construed as an admission that the invention is not entitled to antedate such disclosure by virtue of prior invention. In the event of a conflict between the definitions or teachings of documents cited herein and definitions or teachings recited in the present specification, the text of the present specification takes precedence.

All sequences referred to herein are disclosed in the attached sequence listing that, with its whole content and disclosure, is a part of this specification.

### General definitions of important terms used in the application

The terms "protein" and "polypeptide" refer to any chain of two or more amino acids linked by peptide bonds, and does not refer to a specific length of the product. Thus, "peptides", "protein", "amino acid chain", or any other term used to refer to a chain of two or more amino acids, are included within the definition of "polypeptide," and the term "polypeptide" may be used instead of, or interchangeably with any of these terms. The term "polypeptide" is also intended to refer to the products of post-translational modifications of the polypeptide, including without limitation glycosylation, acetylation, phosphorylation, amidation, proteolytic cleavage, modification by non-naturally occurring amino acids and similar modifications which are well known in the art. Thus, binding proteins comprising two or more protein moieties also fall under the definition of the term "protein" or "polypeptides".

The term "ubiquitin" or "unmodified ubiquitin" or "wild type ubiquitin" refers to the ubiquitin in accordance with SEQ ID NO: 1 and according to SEQ ID NO: 2 (point mutations in positions 45, 75, 76 which do not influence binding to a target) and according to SEQ ID NO: 3 (point mutations in positions 75 and 76 which do not influence binding to a target) and according to SEQ ID NO: 4 and according to the following definition. Ubiquitin proteins with at least 95 % identity to SEQ ID NO: 1 are considered as wild type ubiquitins, for example, SEQ ID NO: 2 and SEQ ID NO: 3. Unmodified dimeric ubiquitins consist of two wild type ubiquitins, for example SEQ ID NO: 4. Ubiquitin proteins with at least 95 % identity to SEQ ID NO: 4 are considered as unmodified dimeric ubiquitins. Particularly preferred are ubiquitin molecules from mammals, e.g. humans, primates, pigs, and rodents. On the other hand, the ubiquitin origin is not relevant since according to the art all eukaryotic ubiquitins are highly conserved and the mammalian ubiquitins examined up to now are even identical with respect to their amino acid sequence. In addition, ubiquitin from any other eukaryotic source can be used. For instance, ubiquitin of yeast differs only in three amino acids from the unmodified human ubiquitin.

The terms "modified ubiquitin" or "ubiquitin mutein" or "Affilin" are all used synonymously and can be exchanged. The term "modified ubiquitin" or "ubiquitin mutein" or "Affilin" as used herein refers to derivatives of ubiquitin which differ from said unmodified ubiquitin by amino acid exchanges, insertions, deletions or any combination thereof, provided that the modified ubiquitin or ubiquitin mutein has a specific binding affinity to a target epitope or antigen which is at least 10fold lower or absent in unmodified ubiquitin. This functional property of an ubiquitin mutein (Affilin; modified ubiquitin) is a *de novo* created function.

The term "Affilin®" (registered trademark of Scil Proteins GmbH) refers to non-immunoglobulin derived binding proteins based on ubiquitin muteins. An Affilin molecule as used in this invention comprises either one modified ubiquitin moiety or comprises two differently modified ubiquitin moieties linked together in a head-to-tail fusion. A "head-to-tail fusion" is to be understood as fusing two proteins together by connecting them in the direction (head) N-C-N-C- (tail) (tandem molecule), as described for example in EP2379581B1. The head part is designated as the first moiety and the tail part as the second moiety. In this head-to-tail fusion, the ubiquitin moieties may be connected directly without any linker. Alternatively, the fusion of ubiquitin moieties can be performed via linkers, for example, a polypeptide linker.

The term "insertions" comprises the addition of amino acids to the original amino acid sequence of ubiquitin wherein the ubiquitin remains stable without significant structural change. Naturally, loop regions connect regular secondary structure elements. The structure of human ubiquitin reveals six loops at amino acid regions 8-11, 17-22, 35-40, 45-47, and 50-63 which connect secondary structure elements such as beta sheets and alpha helix. Preferred are ubiquitin muteins comprising a combination of insertions and substitutions, as described in EP2721152. Preferred ubiquitin muteins have insertions of 2-10 amino acids, preferably in the most N-terminal loop within amino acids 8-11 or in the most C-terminal loop within amino acids 50-63. However, other locations for insertions are possible. Specifically, the number of amino acids to be inserted is 2, 3, 4, 5, 6, 7, 8, 9, 10, preferably 2, 3, 4, 5, 6, 7, 8 amino acids, most preferred 4, 5, 6, 7, 8 amino acids.

The term "antibody" as used in accordance with the present invention comprises monoclonal antibodies having two heavy chains and two light chains (immunoglobulin or IgG antibodies). Heavy and light chains are connected via non-covalent interactions and disulfide bonds. Furthermore, fragments or derivatives of monoclonal antibodies, which still retain the binding specificity, are comprised in the term "antibody". The term "antibody" includes embodiments such as chimeric (human constant domain, non-human variable domain), single chain and humanized (human antibody with the exception of non-human CDRs) antibodies. Full-length IgG antibodies consisting of two heavy chains and two light chains are preferred in this invention. Fragments of antibodies as used within the scope of the invention are functional fragments which retain the same binding specificity as the non-fragmented antibody.

A "Fab fragment" refers to an antigen binding fragment of an immunoglobulin molecule, containing the variable regions of both light and heavy chains.

The term "antigen" is not particularly limited in its structure, as long as it comprises epitopes to which antigen-binding domains present in the first and the second binding protein bind. Antigens can be inorganic or organic substances. The term includes, for example, small molecules in body fluid such as drugs, toxins, autoantibodies, autoantigens, proteins, polypeptides, carbohydrates, nucleic acids and other molecules. Specific examples are a cancer target antigen, a receptor target antigen on immune cells, and a soluble target antigen selected e.g. from hormones and cytokines.

The term "epitope" includes any molecular determinant on a target antigen capable of being bound by an antigen binding protein as defined herein and is a region of a target antigen that is bound by an antigen binding protein that targets that antigen, and when the antigen is a protein, it may include specific amino acids that directly contact the antigen binding protein. In a conformational epitope, amino acid residues are separated in the primary sequence, but are located near each other on the surface of the molecule when the polypeptide folds into the native three-dimensional structure. A linear epitope is characterized by two or more amino acid residues which are located adjacent in a single linear segment of a protein chain. In other cases, the epitope may include determinants from posttranslational modifications of the target protein such as glycosylation, phosphorylation, sulfation acetylation, fatty acids or others.

The term "fused" refers to components (e.g. an Affilin molecule and a monoclonal antibody or a Fab fragment) are linked by peptide bonds, either directly or via a peptide linker.

The term "fusion protein" relates to a protein comprising at least a first protein joined to at least a second protein. A fusion protein is created through joining of two or more genes that code for separate proteins. Thus, a fusion protein may comprise a multimer of different or identical binding proteins which are expressed as a single, linear polypeptide. It may comprise one, two, three or even more binding proteins. A fusion protein as used herein comprises at least a first binding protein (e.g. Affilin) which is fused with at least a second binding protein, e.g. a monoclonal antibody or a fragment thereof. Such fusion proteins may further comprise additional domains that are not involved in binding of the target, such as but not limited to, for example, multimerization moieties, polypeptide tags, polypeptide linkers.

The term "Mabfilin" as used herein relates to a fusion protein comprising an Affilin which is fused with a monoclonal antibody.

The term "Fabfilin" as used herein relates to a fusion protein comprising an Affilin which is fused with a fragment of a monoclonal antibody, preferably with a Fab fragment.

The term "conjugate" as used herein relates to a protein comprising or essentially consisting of at least a first protein attached chemically to other substances such as to a second protein or a non-proteinaceous moiety. The conjugation can be performed by means of organic synthesis or by use of enzymes including natural processes of enzymatic post-translational modifications. Examples for protein conjugates are glycoproteins (conjugated protein with carbohydrate component) or lipoproteins (conjugated protein with lipid component). The molecule can be attached e.g. at one or several sites through any form of a linker. Chemical coupling can be performed by chemistry well known to someone skilled in the art, including substitution (e.g. N-succinimidyl chemistry), addition or cycloaddition (e.g. maleimide chemistry or click chemistry) or oxidation chemistry (e.g. disulfide formation). Some examples of non-proteinaceous polymer molecules which are chemically attached to binding proteins of the invention are hydroxyethyl starch, polyethylene glycol, polypropylene glycol, dendritic polymers, or polyoxyalkylene, and others.

A fusion protein or protein conjugate may further comprise one or more reactive groups or peptidic or non-peptidic moieties such as ligands or therapeutically or diagnostically relevant molecules such as radionuclides or toxins. It may also comprise small organic or non-amino acid based compounds, e.g. a sugar, oligo- or polysaccharide, fatty acid, etc. Methods for attaching a protein of interest to such non-proteinaceous components are well known in the art, and are thus not described in further detail here.

The terms "bispecific binding molecule", "trispecific binding molecule", etc., generally "multispecific binding molecule", mean that the antigen binding molecule is able to specifically bind two, three, or multiple distinct epitopes, respectively. Typically, a bispecific antigen binding molecule comprises two antigen binding sites, each of which is specific for a different epitope. In certain embodiments the bispecific antigen binding molecule is capable of simultaneously binding two epitopes, particularly two epitopes expressed on two distinct cells. The term "bispecific binding molecule" or "bispecific binding protein" means that binding proteins of the present invention are capable of specifically binding to two different epitopes. Moreover, the bispecific binding protein of the present invention is capable of binding to two different epitopes at the same time. This means that a bispecific construct is capable of simultaneously binding to at least one epitope "A" and at least one epitope "B", wherein A and B are not the same. The two epitopes may be located on the same or different target antigens which means that the fusion molecules of the present invention can bind one target at two different epitopes or two target antigens each with its own epitope. Similarly, "trispecific binding molecules" and "multispecific binding molecules" are capable of binding three or multiple epitopes at the same time, respectively, wherein the epitopes may be located on the same or different antigens. The term "multivalent binding molecule" means that the binding protein of the present invention comprises at least two, three, or more binding proteins, e.g. protein "α", "β", "γ", "δ" etc.. All binding proteins of the invention are at least "bivalent" because they comprise at least two binding proteins (Affilin and antibody or antibody fragment).

Said binding proteins may bind specifically to the same or overlapping epitopes on a target antigen (monospecific), e.g. the composition of the binding protein may be described by (α)₂, (α)₃, (α)₄ or (β)₂, (β)₃, (β)₄ etc... In this case, the fusion molecules are monospecific but bivalent, trivalent, tetravalent, or multivalent for the epitope A or epitope B, respectively.

Alternatively, said binding proteins may bind to different, non-overlapping epitopes on the same or different target molecules and are thus classified as bispecific, trispecific, multispecific, etc., for example αβ, βγ, αδ, αβγ, αβγδ binding to epitopes AB, BC, AD, ABC or ABCD, respectively. For example, the binding proteins of the invention comprising a monoclonal antibody or Fab-fragment are bispecific. For example, fusion proteins comprising a HER2-specific Affilin and an EGFR-specific monoclonal antibody (referred to as "Mabfilin HER2/EGFR") or Fab fragment (referred to as "Fabfilin HER2/EGFR") or fusion proteins comprising a PD1-specific Affilin and a CD3-specific monoclonal antibody (referred to as "Mabfilin PD1/CD3") or Fab fragment (referred to as "Fabfilin PD1/CD3") or fusion proteins comprising a HER2-specific Affilin and a CD3-specific monoclonal antibody (referred to as "Mabfilin HER2/CD3") or Fab fragment (referred to as "Fabfilin HER2/CD3") are bispecific.

The term "multimeric binding molecules" refers to binding proteins that are multivalent and / or multispecific, comprising two or more moieties of binding protein α, β and/or γ etc., αα, βββ, ααβ, ααββ, αγγ, ββγ, αβγδδ, etc.. For example, ααβγ is trispecific and bivalent with respect to epitope A.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity.

To determine the sequence identity the sequence of a query protein is aligned to the sequence of a reference protein, for example, to SEQ ID NO: 1, SEQ ID NO: 2, SEQ ID NO: 3, or SEQ ID NO: 4, preferably to SEQ ID NO: 1 or SEQ ID NO: 4. Methods for alignment are well known in the art. For example, for determining the extent of an amino acid sequence identity of an arbitrary polypeptide relative to the amino acid sequence of SEQ ID NO: 1, SEQ ID NO: 2, SEQ ID NO: 3, or SEQ ID NO: 4, the SIM Local similarity program is preferably employed (Xiaoquin Huang and Webb Miller (1991), Advances in Applied Mathematics, vol. 12: 337-357), that is freely available (see also: http://www.expasy.org/tools/sim-prot.html). For multiple alignment analysis ClustalW is preferably used (Thompson et al. (1994) Nucleic Acids Res., 22(22): 4673-4680).

Each amino acid of the query sequence that differs from the reference amino acid sequence at a given position is counted as one difference. An insertion or deletion in the query sequence is also counted as one difference. For example, an insertion of a linker between two ubiquitin moieties is counted as one difference compared to the reference sequence. The sum of differences is then related to the length of the reference sequence to yield a percentage of non-identity. The quantitative percentage of identity is calculated as 100 minus the percentage of non-identity. In specific cases of determining the identity of ubiquitin muteins aligned against unmodified ubiquitin, differences in positions 45, 75 and/or 76 are not counted, in particular, because they are not relevant for the novel binding capability of the ubiquitin mutein but may be only modifications chosen for biochemical reasons. Generally, a ubiquitin *used as starting material for the modifications* has an amino acid identity of at least 95 %, or of at least 96 % or of at least 97 %, or of at least an amino acid sequence identity of 98 % to SEQ ID NO: 1, SEQ ID NO: 2, SEQ ID NO: 3, or SEQ ID NO: 4. Most preferred are starting proteins with an amino acid identity of at least 95 % to SEQ ID NO: 1 or SEQ ID NO: 4. Thus, a polypeptide which is, for example, 95 % "identical" to a reference sequence may comprise, for example, five point mutations or four point mutations and one insertion etc., per 100 amino acids, compared to the reference sequence.

In the present specification, the terms "target antigen", "target", "ligand", "antigen", and "binding partner" are all used synonymously and can be exchanged. A target preferably refers to a polypeptide or protein. The target is a naturally occurring or non-natural polypeptide or protein, or a polypeptide or protein with chemical modifications. Preferably the target is one of the targets defined herein below. The term "antigen", as used herein, is to be interpreted in a broad sense and includes any target moiety that is bound by the binding moieties of the binding proteins of the present invention.

The terms "protein capable of binding",, or ""binding protein",, or "binding EGFR" or "binding HER2" or "binding CD3" or "binding PD1" or "binding affinity for" according to this invention refer to a protein comprising a binding capability to a defined target antigen.

An "antigen binding site" refers to the site, i.e. one or more amino acid residues, of an antigen binding molecule which provide interaction with the antigen. For example, the antigen binding site of an antibody comprises amino acid residues from the complementarity determining regions. A native immunoglobulin molecule typically has two antigen binding sites, a Fab molecule typically has a single antigen binding site.

The term "dissociation constant" or "K_{D}" ,,defines the specific binding affinity. A high affinity corresponds to a low value of K_{D}. Thus, the expression,, a K_{D} of at least e.g. 10⁻⁷ M" means a value of 10⁻⁷ M or lower (binding more tightly). 1 x 10⁻⁷ M corresponds to 100 nM. A value of 10⁻⁵ M and below down to 10⁻¹² M can be considered as a quantifiable binding affinity. Depending on the application a value of 10⁻⁷ to 10⁻¹² M is preferred for chromatographic applications or for diagnostic or therapeutic applications. In accordance with the invention, the affinity for the target binding should be in the range of less than 7 x 10⁻⁷ M (700 nM). Final target binding affinity should be ideally below 10⁻⁹ M (1 nM) for medical applications.

The methods for determining the binding affinities are known *per se* and can be selected for instance from the following methods known in the art: Surface Plasmon Resonance (SPR) based technology, Bio-layer interferometry (BLI), enzyme-linked immunosorbent assay (ELISA), flow cytometry, fluorescence spectroscopy techniques, isothermal titration calorimetry (ITC), analytical ultracentrifugation, radioimmunoassay (RIA or IRMA) and enhanced chemiluminescence (ECL). Some of the methods are described in the Examples below.

### Detailed description of the embodiments of the invention

### Binding proteins of the invention comprising a first and a second binding protein (Mabfilin and Fabfilin).

The binding protein of the invention is comprising or essentially consisting of at least a first binding protein wherein the first binding protein is a ubiquitin mutein (Affilin) with specific binding affinity (K_{D}) of less than 700 nM to a first epitope and wherein the ubiquitin mutein has a sequence identity of at least 80 % and at maximum 95 % to the amino acid sequence defined by SEQ ID NO: 1 or by SEQ ID NO: 4; and at least a second binding protein wherein the second binding protein is a monoclonal antibody or a fragment thereof, each with binding specificity to a second epitope, and optionally a linker.

In the binding protein of the invention, the ubiquitin mutein is linked to the heavy chain or to the light chain of the monoclonal antibody or fragment thereof, preferably a Fab-fragment. In one embodiment, the ubiquitin mutein (Affilin) is linked to the C-terminus of the light chain of the monoclonal antibody or fragment thereof, in particular a Fab-fragment, or the ubiquitin mutein (Affilin) is linked to the C-terminus of the heavy chain of the monoclonal antibody or fragment thereof, in particular a Fab-fragment. In another embodiment, the ubiquitin mutein (Affilin) is linked to the N-terminus of the light chain of the monoclonal antibody or fragment thereof, in particular a Fab-fragment, or the ubiquitin mutein (Affilin) is linked to the N-terminus of the heavy chain of the monoclonal antibody or fragment thereof, in particular a Fab-fragment, or a combination thereof. Specific examples for these embodiments are provided in Table 1 (and for example, SEQ ID NO: 11-26). In a preferred embodiment, specific binding proteins comprise ubiquitin muteins linked directly or by a linker to the N- or C-terminus of the light or heavy chain of monoclonal antibodies or fragments thereof. In even more preferred embodiments, binding proteins comprising a ubiquitin mutein are linked to the C-terminus to the light or heavy chain of monoclonal antibodies or fragments (for example, SEQ ID NOs: 14, 15, 16, 20, 21, 22, 24, 26, see for example Table 1, for example PID 40, PID 42, PID 50, PID 52, PID 54, PID 56, PID 69, PID 71, PID 77, PID 79).

Preferred combinations of binding proteins comprise a ubiquitin mutein (Affilin) linked to the C-terminus of the heavy chain and to the N-terminus of the light chain of a monoclonal antibody or fragment thereof, preferably a Fab-fragment, a ubiquitin mutein (Affilin) linked to the C-terminus of the heavy chain and to the C-terminus of the light chain of a monoclonal antibody or fragment thereof, preferably a Fab-fragment, a ubiquitin mutein (Affilin) linked to the N-terminus of the heavy chain and to the N-terminus of the light chain of a monoclonal antibody or fragment thereof, preferably a Fab-fragment, or a ubiquitin mutein (Affilin) linked to the N-terminus of the heavy chain and to the C-terminus of the light chain of a monoclonal antibody or fragment thereof, preferably a Fab-fragment.

Table 1 represents examples of proteins disclosed in this invention, including Mabfilin and Fabfilin proteins and corresponding control proteins. Column one refers to the PID (protein identification number); the PID is used throughout the invention for the fusion proteins. Column two refers to the name of the protein and column three to the sequences comprising heavy and light chains of the respectively fusion protein. For example, PID 20 refers to "Cetuximab mAb CL 139791", referring to a fusion protein comprising Affilin-139791 fused to the C-terminus of the light chain (CL) of Cetuximab (monoclonal antibody = mAB) (comprising SEQ ID NO: 20 and SEQ ID NO: 5). For example, PID 52 refers to "Cetuximab Fab CL 141926", referring to a fusion proteins comprising Affilin-141926 fused to the C-terminus of the light chain (CL) of the Fab fragment of Cetuximab (comprising SEQ ID NO: 26 and SEQ ID NO: 51). Other abbreviations used: NH, fusion to N-terminus of heavy chain, CH, fusion to C-terminus of heavy chain, NL, fusion to N-terminus of light chain. Column four shows the specific targets of monoclonal antibody or fragments and of the fusions (for example, HER2/EGFR, refers to binding protein 1 (Affilin) binds to HER2, binding protein 2 (antibody) binds to EGFR. Control fusion proteins with ubiquitin or ubiquitin dimer bind to only one target via the antibody.

**Table 1.**

| **PID** | **name of the protein** | **fusion of SEQ ID NO:** | | **Target(s)** |
|---|---|---|---|---|
| **5** | Cetuximab mAb | 5 | 6 | EGFR |
| **11** | Cetuximab mAb NH 139791 | 11 | 6 | EGFR/EGFR |
| **12** | Cetuximab mAb NH 139864 | 12 | 6 | EGFR/EGFR |
| **13** | Cetuximab mAb NH 139819 | 13 | 6 | EGFR/EGFR |
| **14** | Cetuximab mAb CH 139791 | 14 | 6 | EGFR/EGFR |
| **15** | Cetuximab mAb CH 139864 | 15 | 6 | EGFR/EGFR |
| **16** | Cetuximab mAb CH 139819 | 16 | 6 | EGFR/EGFR |
| **17** | Cetuximab mAb NL 139791 | 17 | 5 | EGFR/EGFR |
| **18** | Cetuximab mAb NL 139864 | 18 | 5 | EGFR/EGFR |
| **19** | Cetuximab mAb NL 139819 | 19 | 5 | EGFR/EGFR |
| **20** | Cetuximab mAb CL 139791 | 20 | 5 | EGFR/EGFR |
| **21** | Cetuximab mAb CL 139864 | 21 | 5 | EGFR/EGFR |
| **22** | Cetuximab mAb CL 139819 | 22 | 5 | EGFR/EGFR |
| **23** | Cetuximab mAb NH 141926 | 23 | 6 | HER2/EGFR |
| **24** | Cetuximab mAb CH 141926 | 24 | 6 | HER2/EGFR |
| **25** | Cetuximab mAb NL 141926 | 25 | 5 | HER2/EGFR |
| **26** | Cetuximab mAb CL 141926 | 26 | 5 | HER2/EGFR |
| **27** | Cetuximab mAb CH Ubiquitin | 27 | 6 | EGFR |
| **28** | Cetuximab mAb CL Ubiquitin | 28 | 5 | EGFR |
| **29** | Cetuximab mAb NH Ubiquitin | 29 | 6 | EGFR |
| **30** | Cetuximab mAb NL Ubiquitin | 30 | 5 | EGFR |
| **31** | Cetuximab mAb NH 139864 NL 139864 | 12 | 18 | EGFR/EGFR |
| **32** | Cetuximab mAb CH 139864 NL 139864 | 15 | 18 | EGFR/EGFR |
| **33** | Cetuximab mAb NH 139864 CL 139864 | 12 | 21 | EGFR/EGFR |
| **34** | Cetuximab mAb CH 139864 CL 139864 | 15 | 21 | EGFR/EGFR |
| **35** | Cetuximab mAb NH 139819 CL 139791 | 13 | 20 | EGFR/EGFR |
| **38** | 145-2C11 Fab Fragment | 44 | 45 | CD3 |
| **40** | 145-2C11 Fab CL 128187 | 46 | 45 | PD1/CD3 |
| **41** | 145-2C11 Fab NL 128187 | 47 | 45 | PD1/CD3 |
| **42** | 145-2C11 Fab CH 128187 | 48 | 44 | PD1/CD3 |
| **43** | 145-2C11 Fab NH 128187 | 49 | 44 | PD1/CD3 |
| **44** | 145-2C11 Fab NH 128187 NL 128187 | 49 | 47 | PD1/CD3 |
| **45** | 145-2C11 Fab CH 128187 NL 128187 | 48 | 47 | PD1/CD3 |
| **46** | 145-2C11 Fab CH 128187 CL 128187 | 48 | 46 | PD1/CD3 |
| **47** | 145-2C11 Fab NH 128187 CL 128187 | 49 | 46 | PD1/CD3 |
| **48** | Cetuximab Fab fragment | 52 | 6 | EGFR |
| **49** | Cetuximab Fab NH 141926 | 53 | 6 | HER2/EGFR |
| **50** | Cetuximab Fab CH 141926 | 54 | 6 | HER2/EGFR |
| **51** | Cetuximab Fab NL 141926 | 51 | 25 | HER2/EGFR |
| **52** | Cetuximab Fab CL 141926 | 51 | 26 | HER2/EGFR |
| **53** | Cetuximab Fab NH 139819 | 55 | 6 | EGFR/EGFR |
| **54** | Cetuximab Fab CH 139819 | 56 | 6 | EGFR/EGFR |
| **55** | Cetuximab Fab NL 139819 | 51 | 19 | EGFR/EGFR |
| **56** | Cetuximab Fab CL 139819 | 51 | 22 | EGFR/EGFR |
| **57** | Cetuximab Fab NH 139090 | 57 | 6 | EGFR |
| **58** | Cetuximab Fab CH 139090 | **58** | 6 | EGFR |
| **59** | Cetuximab Fab NL 139090 | 51 | 59 | EGFR |
| **60** | Cetuximab Fab CL 139090 | 51 | 60 | EGFR |
| **61** | Cetuximab Fab NH Ubiquitin | 61 | 6 | EGFR |
| **62** | Cetuximab Fab CH Ubiquitin | 62 | 6 | EGFR |
| **63** | Cetuximab Fab NL Ubiquitin | 51 | 30 | EGFR |
| **64** | Cetuximab Fab CL Ubiquitin | 51 | 28 | EGFR |
| **66** | OKT3 mAb | 66 | 67 | CD3 |
| **67** | OKT3 Fab | 68 | 67 | CD3 |
| **68** | OKT3 mAb NH 142628 | 69 | 67 | HER2/CD3 |
| **69** | OKT3 mAb CH 142628 | 70 | 67 | HER2/CD3 |
| **70** | OKT3 mAb NL 142628 | 66 | 71 | HER2/CD3 |
| **71** | OKT3 mAb CL 142628 | 66 | 72 | HER2/CD3 |
| **72** | OKT3 mAb NH 139090 | 73 | 67 | CD3 |
| **73** | OKT3 mAb CH 139090 | 74 | 67 | CD3 |
| **74** | OKT3 mAb NL 139090 | 66 | 75 | CD3 |
| **75** | OKT3 mAb CL 139090 | 66 | 76 | CD3 |
| **76** | OKT3 Fab NH 142628 | 77 | 67 | HER2/CD3 |
| **77** | OKT3 Fab CH 142628 | 78 | 67 | HER2/CD3 |
| **78** | OKT3 Fab NL 142628 | 68 | 71 | HER2/CD3 |
| **79** | OKT3 Fab CL 142628 | 68 | 72 | HER2/CD3 |
| **80** | OKT3 Fab NH 139090 | 79 | 67 | CD3 |
| **81** | OKT3 Fab CH 139090 | 80 | 67 | CD3 |
| **82** | OKT3 Fab NL 139090 | 68 | 75 | CD3 |
| **83** | OKT3 Fab CL 139090 | 68 | 76 | CD3 |

**Binding proteins and conjugates of the invention comprising further functional moieties.** One embodiment of the invention covers a binding protein of the invention comprising at least one additional protein or molecule. In such embodiment, the additional protein can be a ubiquitin mutein (Affilin) with identical or different specificity for an antigen as the first binding protein. In this specific embodiment, the binding protein of the invention comprises a third binding protein wherein the third binding protein is a ubiquitin mutein with specific binding affinity of less than 700 nM to the same or a different epitope than the first protein and wherein the ubiquitin muteins have a sequence identity of at least 80 % and at maximum 95 % to the amino acid sequence defined by SEQ ID NO: 1 or by SEQ ID NO: 4, and wherein the third binding protein is linked to different termini of the second binding protein (antibody) than the first binding protein or to the N- or C-terminus of the first binding protein.

One embodiment of the invention covers a fusion protein or a conjugate comprising an Affilin-antibody fusion protein or conjugate further comprising at least one additional molecule, preferably selected from at least one member of the groups (i), (ii) and (iii) consisting of (i) a pharmacokinetic moiety modulating serum half-life selected from a polyethylene glycol, a human serum albumin (HSA), anti-human serum albumin, albumin-binding peptides, a polymer sequence forming a random coil, an immunoglobulin or immunoglobulin fragments, or a polysaccharide, and, (ii) a therapeutically active component, optionally selected from a monoclonal antibody or a fragment thereof retaining the binding specificity of said monoclonal antibody, a cytokine, a chemokine, a cytotoxic compound, an enzyme, or derivatives thereof, or a radionuclide, each retaining the specific activity of any one of said molecules, and (iii) a diagnostic component, optionally selected from a fluorescent compound, a photosensitizer or a radionuclide. The conjugate molecule can be attached e.g. at one or several sites through a peptide linker sequence or a carrier molecule. For example, a fusion protein of the invention could be coupled to a carrier suitable for further multi-toxophore conjugation. The carrier can be selected from polyethylene glycol (PEG) or HES or other suitable carriers. Further conjugation with proteinaceous or non-proteinaceous moieties to generate protein conjugates according to the invention can be performed applying chemical methods well-known in the art. In particular, coupling chemistry specific for derivatization of cysteine or lysine residues is applicable. In case of introduction of non-natural amino acids further routes of chemical synthesis are possible, e.g. "click chemistry" or aldehyde specific chemistry and others.

Conjugates thus obtained can be selected from one or more of the following examples: conjugation of the protein via lysine residues; conjugation of the protein via cysteine residues via maleimide chemistry; in particular, cysteine residues can be specifically introduced and can be located at any position suitable for conjugation of further moieties; peptidic or proteinogenic conjugations; "Tag" fusions - a protein or a peptide located either at the C- or N- terminus of the protein, fusion "tags" are, e.g., poly-histidine, HA-tag, FLAG-tag, Strep-tag, and others. These and other methods for covalently and non-covalently attaching a protein of interest to other functional components are well known in the art, and are thus not described in further detail here.

A further embodiment relates to binding proteins according to the invention, further comprising a pharmacokinetic moiety modulating serum half-life or biodistribution, preferably selected from polyethylene glycol (PEG), a human serum albumin, anti-human serum albumin, albumin-binding peptides, a polymer sequence forming a random coil or an immunoglobulin or immunoglobulin fragments, for example an Fc fragment. Several techniques for producing proteins with extended half-life are known in the art.

**First binding protein of the binding protein of the invention.** The binding proteins according to this invention comprise at least a first binding protein wherein the first binding protein is a ubiquitin mutein with specific binding affinity (K_{D}) of less than 700 nM to a first epitope and wherein the ubiquitin mutein has a sequence identity of at least about 80 % and at maximum about 95 % to the amino acid sequence defined by SEQ ID NO: 1 or by SEQ ID NO: 2 or by SEQ ID NO: 3 or by SEQ ID NO: 4.

The degree of modification of a ubiquitin mutein as used in the invention accounts for at least about 7 % and up to a total of about 20 % of amino acids compared to wild type ubiquitin of SEQ ID NO: 1 or SEQ ID NO: 4. In other words, this corresponds to 5-15 amino acid residues in a ubiquitin moiety which are modified in order to generate a new binding property to a target antigen (if two ubiquitin moieties are linked, 10 - 30 amino acids in total are modified to generate a new binding property). Most preferred are substitutions of 9 % to 15 % of all amino acids of SEQ ID NO: 1 or SEQ ID NO: 4 to generate a novel protein with newly created measurable binding properties to a target antigen. In other words, this corresponds to a modification of 6 to 11 amino acid residues to generate a novel protein with newly created measurable binding properties to a target antigen. Considering this, there is a sequence identity of the ubiquitin mutein (Affilin) to SEQ ID NO: 1 or SEQ ID NO: 4 of at least 80 %, at least 85 %, at least 86 %, at least 87 %, at least 88 %, at least 89 %, at least 90 %, at least 91 %, at least 92 %, at least 93 %, maximal 94%, in particular if substitutions and insertions are generating the novel binding property.

The derivatization of ubiquitin to generate a ubiquitin mutein that specifically binds a particular target antigen has been described in the art. For example, a library of different ubiquitin muteins can be created in which the sequence as shown in SEQ ID NO: 1 or SEQ ID NO: 2 or SEQ ID NO: 3 or SEQ ID NO: 4 has been altered. Preferably, the alteration is carried out at amino acids located in (i) region 2-11, or (ii) region 62-68 or (iii) in both regions simultaneously. However, further positions not comprised by these regions might be altered as well. Preferably, the alteration is a substitution, insertion or deletion as described in the art. The substitution of amino acid residues for the generation of the novel binding proteins derived from ubiquitin can be performed with any desired amino acid. This is described in detail in EP1626985B1, EP2379581B1, and EP2721152.

The step of modification of the selected amino acids is performed preferably on the genetic level by random mutagenesis of the selected amino acids. Preferably, the modification of ubiquitin is carried out by means of methods of genetic engineering for the alteration of a DNA belonging to the respective protein.

One preferred method of modification of the selected amino acids is by random mutagenesis of the multiple, selected amino acids at the genetic level. Methods to introduce such random mutagenesis are well known in the art. Assuming a random distribution of the 20 natural amino acids at e.g. 8 positions generates a pool of 20 to the power of 8 (20⁸ = 2.56 x 10¹⁰) theoretical ubiquitin muteins, each with a different amino acid composition and potentially different binding properties. This large pool of genes constitutes a library of different Affilin® molecules.

Subsequently, the library can be cloned into a phagemid vector (e.g. pCD87SA (Paschke, M. and W. Hohne (2005) Gene 350(1): 79-88)). The library may be displayed on phage and subjected to repeated rounds of panning against the respective target antigen. Ubiquitin muteins from enriched phage pools are cloned into expression vectors for individual protein expression. Preferably, expression of the ubiquitin mutein is then carried out in prokaryotic or eukaryotic organism to enable screening for specific binding proteins by established techniques, such as ELISA on automated high-throughput screening platforms. Identified clones with desired binding properties are then sequenced to reveal the amino acid sequences of target-binding Affilin molecules. In case of an Affilin® with one ubiquitin mutein moiety, the amino acid positions of the Affilin have to be aligned with the sequence given for ubiquitin (SEQ ID NO: 1) in order to identify the amino acid changes. In case of an Affilin molecule consisting of two ubiquitin moieties, the amino acid positions of the Affilin® have to be aligned with the sequence given for di-ubiquitin (SEQ ID NO: 4) in order to identify the amino acid changes.

The identified binding protein may be subjected to further maturation steps, e.g. by generating additional libraries based on alterations of the identified sequences and repeated phage display, ribosomal display, panning and screening steps as described above.

**Second binding protein of the bispecific binding protein of the invention.** The binding proteins of the invention comprise at least a second binding protein wherein the second binding protein is a monoclonal antibody or a fragment thereof, each with binding specificity to a second epitope, wherein the second epitope may be the same (or overlapping) or different from the epitope of the first binding protein.

Antibody fragments or derivatives thereof retaining their binding specificity that can be used in the present binding proteins include but are not limited to: (i) a Fab fragment (fragment, antigen binding region), a monovalent fragment consisting of the light chains with variable and constant domains and heavy chain variable region and first constant region domain (CH1) (Fc domains missing); (ii) a Fab' fragment, a Fab with the heavy chain hinge region; (iii) a F(ab')₂ fragment, a dimer of two Fab fragments linked by disulfide bonds at the heavy chain hinge region; (iv) a Fv fragment, consisting of a dimer of one heavy and one light chain variable domain in non-covalent association; (v) a single chain variable fragments (scFv) consisting of the heavy and light chains of immunoglobulins connected with a short linker peptide, (vi) a dAb fragment (domain antibody), which consists of a variable domain of a heavy chain (VH domain) or the variable domain of an antibody light chain (VL domain); (vii) an isolated complementarity determining region (CDR) having sufficient framework to specifically bind, e.g., an antigen binding portion of a variable region; (viii) multimeric formats such as minibodies, diabodies (scFv dimers), tribodies, tetrabodies or chemically conjugated Fab'-multimers. - Fab fragments are most preferred. Fab fragments behave like antibodies in terms of antigen recognition. Antibody fragments are smaller than full size antibodies and may penetrate tissues and tumors faster than full size monoclonal antibodies, potentially having favorable biodistribution profiles.

Various techniques for the generation of monoclonal antibodies and fragments thereof with desired binding properties are well known in the art and described. These methods comprise *in vivo* generation of antibodies through immunization of an animal with the antigen of interest and isolating cells that produce the antibody. Typically mice are immunized and cells from their spleens, e.g. B-cells, fused with myeloma cells, applying the so-called hybridoma technology, to generate clonal cell lines expressing the antibody of interest. Another technology for generating a cell line with expressing monoclonal antibodies or fragments thereof is described, for example, in WO 2004/076677. B cells are immortalized by infection Epstein Barr Virus (EBV) and growing clones secreting specific antibodies are selected. In addition, many *in-vitro* technologies have been described in the art to generate antibodies with specific binding including various display methods such as ribosome display, phage display, yeast display, bacterial display and others.

Monoclonal antibodies can be produced by various techniques which provide expressed antibodies in amounts suitable for purification, including continuous cell line cultures or batch fermentation. Such techniques are described, for example, in Harlow, Antibodies: A Laboratory Manual, Cold Spring Harbor Laboratory Press, 2nd ed. 1988). A suitable system for the expression of the recombinant antibodies or fragments thereof may be selected from, for example, bacteria, yeast, insects, mammalian cell lines or transgenic animals or plants as known in the art.

An Fab fragment can result from cleavage of an antibody with proteases; Fab' and F(ab')2 fragments can be generated by cleavage with pepsin or papain, respectively. Isolation of expressed antibodies or fragments is well described in the art and typically comprises Protein-A-based affinity chromatography combined with one or more further chromatography steps such as ion-exchange, size-exclusion, hydrophobic-interaction or other chromatography techniques.

Such isolated antibodies or fragments can subsequently be used for conjugation reactions with Affilin molecules to produce binding molecules of the invention.

In another embodiment, the binding protein can consist of or include one or more ubiquitin muteins that are fused to or conjugated with an antibody or fragment thereof binding via an epitope to the same molecular target (or antigen) such as, for example but not limited to, EGFR-monoclonal antibodies selected from Erbitux® (Cetuximab; Imclone), Vectibix® (Panitumumab; Amgen), EMD72000 (Matuzumab; Merck Serono/Takeda), antibody 806 (The Ludwig Institute for Cancer Research), or antibody 425 (Merck). However, any monoclonal antibody or fragment could be used for fusion or conjugation with Affilin molecules. FDA approved therapeutic monoclonal antibodies for autoimmune disease include for example but not limited to Infliximab (Remicade®, Janssen Biotech/Merck), Adalimumab (Humira®, BASF/CAT), Belimumab (Benlysta®, GSK), and Abciximab (ReoPro®, Eli Lilly), and for cancer include for example but not limited to Bevacizumab (Avastin®, Genentech/Roche), Trastuzumab (Herceptin®, Genentech), Rituximab (MabThera®, Rituxan®, Roche/Biogen Idec/Genentec), Denosumab (Prolia®, XGEVA®, Amgen).

**Linker comprised in the bispecific binding protein of the invention.** The binding proteins of the invention comprise a first binding protein (comprising one or two modified ubiquitin subunits) and a second binding protein and optionally a linker connecting the first and the second binding protein (IgG antibody or Fab fragment), and/or the binding proteins can be genetically fused to other functional protein moieties. The invention further comprises a fusion protein comprising a binding protein according to the invention wherein the at least first binding protein (Affilin protein) is genetically fused with the at least second binding protein (monoclonal antibody or Fab fragment), or a conjugate wherein the at least first binding protein is chemically linked to the at least second binding protein and optionally further to non-proteinaceous moieties. The binding proteins can include linkers at various positions (e.g., between Affilin and an antibody or antibody fragment or between two modified ubiquitin moieties or between two Affilin moieties). In the context of such fusion proteins of the invention, the term "linker" refers to a single amino acid or a polypeptide that joins at least two other protein molecules covalently .

The linker is genetically fused to the first and second binding proteins or protein moieties to generate a single, linear polypeptide chain. The length and composition of a linker may vary between at least one and up to about 30 amino acids. Preferably, the peptide linker has a length of between 1 and 20 amino acids; e.g. 1, 2, 3, 4, 5, 6, 7, 8, 9, 10, 11, 12, 13, 14, 15, 16, 17, 18, 19, or 20 amino acids.

It is preferred that the amino acid sequence of the peptide linker is not immunogenic to human beings, stable against proteases and does not form a secondary structure. An example is a linker comprised of small amino acids such as glycine, serine or alanine. The linkers can be glycine-rich (e.g., more than 50 % of the residues in the linker can be glycine residues). Preferred are glycine-serine-linkers of variable length consisting of glycines and serines only. In general, linkers of the structure (SGGG)ₙ or permutations of SGGG, e.g. (GGGS)ₙ, can be used wherein n can be any number between 1 and 6, preferably 1 or 2 or 3. Also preferred are linkers comprising the amino acids alanine, proline, and serine. Other linkers for the genetic fusion of proteins are known in the art and can be used. In one embodiment of the invention, the first binding protein (e.g. ubiquitin mutein) and the second binding protein (e.g. monoclonal antibody or fragment thereof) are linked via a (G₃S)₄ linker. Examples for linkers are shown in SEQ ID NO: 31-39. Moreover, a non-peptide linker such as polyethylene glycol or an alternative polymer could be used.

In case of chemical conjugates of the binding proteins of the invention, the term "linker" refers to any chemical moiety which connects the EGFR binding protein with other proteinaceous or non-proteinaceous moieties either covalently or non-covalently, e.g., through hydrogen bonds, ionic or van der Waals interactions, such as two complementary nucleic acid molecules attached to two different moieties that hybridize to each other. Such linkers may comprise reactive groups which enable chemical attachment to the protein through amino acid side chains.

**Target antigen.** The binding proteins of the invention comprise or essentially consist of a first binding protein wherein the first binding protein has a specific binding affinity (K_{D}) of less than 700 nM to an epitope of an antigen; and a second binding protein wherein the second binding protein is a monoclonal antibody or a fragment thereof, having specific binding affinity to a second epitope of an antigen.

A wide variety of molecular targets can be specifically bound and these include molecules expressed on the cell surface, such as receptors for growth factors, neurotransmitters, hormones or transporters, adhesion molecules and the like. In addition, the target protein may be a soluble protein. The first binding protein and the second binding protein of the invention have specific binding affinity to target antigens selected from a cancer target antigen, preferably EGFR, PDGFR, FGFR, VEGFR, HGFR, HER2, HER3, HER4, PD1, CD19, CD20, CD33, CD52, CD30, EpCAM, receptors of the insulin, Trk, Eph, AXL, LTK, TIE, ROR, RET, KLG, RYK, MuSK, transferrin receptor families, most preferably EGFR, HER2, VEGFR, EpCAM, PD1; a receptor target antigen on immune cells, preferably PD1, CD3, CD4, CD8, CD20, MHC, T-cell receptor, B-cell receptor, CTLA-4, most preferably PD1 and CD3; a soluble target antigen may be selected from hormones, cytokines, growth factors, or any bioactive peptide.

Further, binding proteins of the invention may comprise moieties which are directed towards targets, the binding of which leads to an increased *in vivo* serum half-life of the binding protein of the invention. Such targets include e.g. serum albumin and others. The receptor can be a tyrosine kinase receptor and the binding proteins as described in the Examples were focused on the epidermal growth factor (EGF) receptor (EGFR). The epidermal growth factor receptor (EGFR) is the cell-surface receptor for members of the epidermal growth factor family (EGF-family) (NCBI reference: NP_005219). EGFR is known for its role in lung cancer, head and neck cancer and colorectal cancer. The term "epidermal growth factor receptor" or "EGFR" comprises all polypeptides which show a sequence identity of at least 70 %, 80 %, 85 %, 90 %, 95 %, 96 % or 97 % or more, or 100 % to NP_005219 and have the functionality of EGFR. The term "EGFR" comprises related polypeptides, including allelic variants, splice variants, derivative variants, substitution variants, deletion variants, and/or insertion variants including the addition of an N-terminal methionine, fusion polypeptides, and interspecies homologs. For isoforms, see for example, Albitar et al. Molecular Cancer 2010, 9: 166. In particular, the term "EGFR" comprises the class III variant of EGFR (EGFRvlll) (deletion of exons 2-7, deletion of amino acids 5 - 274, see Wikstrand et al., J NeuroViro 1998, 4: 148-158). The term "EGFR" as understood herein also comprises EGFR class I, class II, class IV, class V, class VI and class VII mutants and variants thereof (see Wikstrand et al., *supra*)*.* An EGFR polypeptide can include terminal residues, such as tag residues, signal peptide sequence residues, targeting residues, amino terminal methionine residues, lysine residues. Reference to EGFR includes variants, isoforms and species homologs of human EGFR. The term EGFR also comprises naturally occurring mutant forms (see for example Humphrey et al. PNAS (USA) 87:4207-4211 (1990)). "EGFR" may be a native sequence EGFR or an amino acid sequence variant thereof. The extracellular part of the mature EGFR consists of 621 amino acids and four receptor domains: Domain I encompasses residues 1-165, domain II residues 166-312, domain III residues 313-481 and domain IV 482-621 (see for example Cochran et al. (2004) J. Immunol. Methods, 287, 147-158).

The involvement in many cancers validates EGFR as a useful therapeutic target and supports the search for improved understanding of receptor biology and the development of improved therapies. Potential causes of the modest efficacy of current EGFR antagonists include the inability to effectively compete with ligand, especially in the presence of autocrine signaling; insufficient down-regulation of receptor; lack of inhibition of constitutively active EGFRvlll; and mutational escape. Thus, novel binders capable of downregulation and/or inhibition via different modes of action would be beneficial and multivalent and/or multispecific binders against EGFR hold the potential to be more effective in this respect.

HER2 (Human Epidermal Growth Factor Receptor 2) is a 185-kDa receptor first described in 1984 (Schlechter et al (1984) Nature 312:513-516). Amplification or over-expression of this gene has been shown to play an important role in the pathogenesis and progression of certain aggressive types of breast cancer, and HER2 is known as an important biomarker and target of therapy for the disease. Other tumors where HER2 plays a role include ovarian cancer and gastric cancer. Human HER2 is represented by the NCBI reference NP_004439. The term "HER2" comprises all polypeptides which show a sequence identity of at least 70 %, 80 %, 85 %, 90 %, 95 %, 96 % or 97 % or more, or 100 % to NP_004439 and have the functionality of HER2. The term "HER2" comprises related polypeptides, including allelic variants, splice variants, derivative variants, substitution variants, deletion variants, and/or insertion variants including the addition of an N-terminal methionine, fusion polypeptides, and interspecies homologs.

CD3 is a glycoprotein on T-cell surface and is responsible for transmission of antigen recognition to the intracellular signaling pathways. CD3 is involved in T-cell activation and proliferation. Human CD3 is represented by the NCBI reference NP_000724.1 (mouse CD3 NP_031674.1). CD3 is composed of four distinct chains. In mammals, the complex contains a CD3γ chain, a CD3δ chain, and two CD3ε chains. These chains associate with a molecule known as the T-cell receptor (TCR) and the ζ-chain to generate an activation signal in T lymphocytes. The TCR, ζ-chain, and CD3 molecules together comprise the TCR complex. The term "CD3" comprises all polypeptides which show a sequence identity of at least 70 %, 80 %, 85 %, 90%, 95%, 96% or 97 % or more, or 100 % to NP_000724.1 and have the functionality of CD3. The term "CD3" comprises related polypeptides, including allelic variants, splice variants, derivative variants, substitution variants, deletion variants, and/or insertion variants including the addition of an N-terminal methionine, fusion polypeptides, and interspecies homologs.

PD1 (programmed cell death protein 1) is a negative regulator in immune response. Human PD1 is represented by the NCBI reference NP_005009.2 (mouse PD1 NP_032824.1). The 55 kDa transmembrane protein has an N-terminal signal peptide and a hydrophobic transmembrane region (see, for example The EMBO J., 11(11):3887-3895, 1992). The term" PD1" comprises all polypeptides which show a sequence identity of at least 70 %, 80 %, 85 %, 90 %, 95%, 96% or 97 % or more, or 100 % to NP_005009.2 and have the functionality of PD1. The term "PD1" comprises related polypeptides, including allelic variants, splice variants, derivative variants, substitution variants, deletion variants, and/or insertion variants including the addition of an N-terminal methionine, fusion polypeptides, and interspecies homologs.

**Advantages of proteins of the invention.** Although bispecific molecules based on antibody-formats are known, there are still major drawbacks, for example suboptimal physicochemical properties or low production yields of those molecules. The binding proteins of the invention provide molecular formats which are easy to engineer and the resulting molecules have favorable physicochemical properties (such as solubility and stability), high-level expression, and allow easy production methods. Thus, the binding proteins of the invention show advantageous biochemical properties and open a new route for the development of multispecific compounds for flexible applications.

One major advantage of bivalent or bispecific molecules comprising two binding proteins according to the invention is that bispecific molecules can be obtained which can be used to bring the respective target substances in spatial proximity to each other. This is achieved by coupling two binding proteins with specificities for different target antigens. The effect of bringing the targets in spatial proximity to each other would not be possible by application of two independent binding proteins targeting the respective antigens individually. Accordingly, a suitable bispecific binding protein can cross-link two different cell types, for example immune cells and cancer cells to accomplish immunotherapeutic strategies.

A further advantage of binding proteins of the invention is increased specificity for specific target cells. This is particularly important since many surface target antigens overexpressed in cancer cells are also expressed on non-tumor cells. A binding protein with specificity for two targets upregulated on cancer cells shows its activity selectively on the cancer cells having high levels of both surface antigens but not on normal cells having low levels of either surface antigen. A fusion protein with specificities for more than one target can improve potency thereby rendering efficient therapies with little side effects.

A further advantage of bivalent or bispecific molecules comprising two binding proteins according to the invention is the increase of the apparent affinity for the same target compared to the affinity of the binding proteins comprising only one binding protein. Due to the increased apparent affinity and/or targeting of two epitopes on the same or different targets, binding proteins of the invention hold the potential of improved efficacy in the treatment of diseases.

**Bispecific binding proteins of the invention.** Examples of bispecific binding proteins consisting of Affilin proteins binding to tumor associated antigens (for example, EGFR and HER2) and an anti-EGFR monoclonal antibody or anti-EGFR antibody fragments (Fab) are shown (Mabfilin EGFR/EGFR; Mabfilin HER2/EGFR; Fabfilin EGFR/EGFR; Fabfilin HER2/EGFR). Further embodiments include bispecific binding proteins consisting of an Affilin protein binding to a cell surface protein 1 (PD1) and an anti-cell surface protein 2 (CD3) monoclonal antibody or Fab fragment (Mabfilin PD1/CD3; Fabfilin PD1/CD3). Even further embodiments include bispecific binding proteins consisting of Affilin proteins binding to a tumor associated antigen (for example, HER2) and an anti-CD3 monoclonal antibody or Fab fragment (Mabfilin HER2/CD3; Fabfilin HER2/CD3).

The ubiquitin mutein and the antibody comprised by the bispecific binding protein of the invention may specifically target the same antigen, e.g. the same cell-surface protein (e.g., EGFR). The binding of the ubiquitin mutein and the antibody may be to distinct (e.g., non-overlapping) or same (e.g. overlapping) epitopes on the target.

The bispecific binding protein of the invention comprises a first binding protein which comprises (i) an amino acid sequence selected from the group consisting of SEQ ID NO: 7 to 10, or (ii) an amino acid sequence that exhibits at least 80 % sequence identity to one or more of the amino acid sequences of SEQ ID NO: 7 to 10.

In one embodiment of the invention, in order to generate a measurable binding affinity with a K_{D} of at least e.g. 10⁻⁷ M to a target, a ubiquitin is at least substituted in 5 amino acids corresponding to positions 62, 63, 64, 65, 66 of SEQ ID NO: 1, preferably in combination with an insertion of 2-10 amino acids in the loop region corresponding to positions 8 - 11 of SEQ ID NO: 1, preferably between positions 9 and 10 of SEQ ID NO: 1. Examples are SEQ ID NO: 7 (Affilin-139791) and SEQ ID NO: 9 (Affilin-139819). At least residue positions 62, 63, 64, 65, 66 of SEQ ID NO: 1 correspond to positions which contain target interaction residues.

In another embodiment of the invention, two ubiquitin moieties are at least substituted in 5 amino acids selected from and corresponding to regions 2-11 and 62-66, in particular positions 62, 63, 64, 65, 66 of SEQ ID NO: 1 and in positions 6 and 8, and the two ubiquitin moieties are connected directly or via a peptide linker. Examples are SEQ ID NO: 8 (Affilin-139864) or SEQ ID NO: 43 (Affilin-128187).

**Mabfilin or Fabilin EGFR/EGFR.** The EGFR binding Affilin molecules bind to cell surface expressed EGFR with high affinity. Preferred EGFR binding molecules are based on a ubiquitin mutein with substitutions of at least 5 amino acids at the C-terminal part of ubiquitin within region 62-68 wherein at least 3 amino acids of these amino acids show a specific motif and a loop insertion between positions 9 and 10 of ubiquitin (e.g. see Affilin-139791, SEQ ID NO: 7, and Affilin-139819, SEQ ID NO: 9). Further preferred EGFR binding proteins comprise two modified ubiquitin moieties linked via a peptide linker (preferably of 3-15 amino acids) and each having different modifications in regions 2-10 and 62-66 of SEQ ID NO: 1 (e.g. see SEQ ID NO: 8, Affilin-139864). Also preferred are ubiquitin muteins with binding affinity of at least 700 nM to EGFR wherein the ubiquitin muteins have an amino acid sequence that exhibits at least 80 % sequence identity to one or more of the amino acid sequences of SEQ ID NO: 7, SEQ ID NO: 8, or SEQ ID NO: 9. Preferred binding molecules of the invention comprise two binding proteins specifically binding to at least two epitopes of EGFR and comprise SEQ ID NO: 11-22 or exhibit at least 80 % sequence identity to one or more of the amino acid sequences of SEQ ID NO: 11-22.

The binding protein of the invention comprises a second binding protein which comprises or consists of a monoclonal antibody or fragment thereof with specificity for EGFR. The function of EGFR can be inhibited by specific monoclonal EGFR antibodies that block the binding of ligands to the extracellular part of the receptor or inhibit the signaling of said receptor. For example, the monoclonal EGFR antibody Cetuximab is known to inhibit the function of EGFR. Cetuximab is used for the treatment of metastatic colorectal cancer as well as head and neck cancer. The antibody is frequently combined with chemotherapeutics and/or radionuclide approaches to increase the therapeutic efficacy. In accordance with a preferred embodiment, the antibody specifically binding to EGFR is Cetuximab or the Fab fragment of Cetuximab.A binding protein comprising an EGFR-specific Affilin fused to Cetuximab (Mabfilin or Fabfilin EGFR/EGFR) provides binding to two different sites on EGFR; the advantage is that possible synergistic effects are pursued caused by allosteric regulation or alterations in homo- or hetero-dimerization propensity of the receptor. Another EGFR specific human monoclonal antibody is for example Panitumumab (Vectibix®, Amgen). Panitumumab binds with high affinity to EGFR and is used particularly for the treatment of metastatic colorectal cancer.

It was surprisingly found that a bispecific binding molecule having a novel format comprising or consisting of an anti-EGFR monoclonal antibody or fragment and an EGFR-specific Affilin is able to bind with high specificities to EGFR. **Mabfilin or Fabfilin HER2/EGFR.** A multispecific binding protein comprising a HER2 specific Affilin and Cetuximab addresses two related growth factor receptors (HER2 and EGFR) which are often involved in cancer located on the same cell or on different cells. It was surprisingly found that a bispecific binding molecule consisting of an anti-EGFR monoclonal antibody and a HER2-specific Affilin is able to bind specifically to both EGFR and HER2. Preferred binding molecules of the invention comprise polypeptides specifically binding to HER2 and to EGFR simultaneously. **Mabfilin or Fabfilin PD12/CD3.** Preferred binding molecules of the invention comprise polypeptides specifically binding to cell surface protein 1 (PD1) and to cell surface protein 2 (CD3). It was surprisingly found that a bispecific binding molecule is able to bind specifically to both targets, e.g. that the bispecific binding molecule is able to bind simultaneously to PD1 and to CD3.

In further embodiments of the invention, the bispecific binding protein of the invention comprises a first binding protein specifically binding to PD1 which comprises (i) an amino acid sequence corresponding to SEQ ID NO: 43 (Affilin 128187), or (ii) an amino acid sequence that exhibits at least 80 % sequence identity to SEQ ID NO: 43.

In accordance with a preferred embodiment, the antibody fragment specifically binding to CD3 is 145-2C11. In accordance with a preferred embodiment, the monoclonal antibody specifically binding to CD3 is OKT3.

**Mabfilin or Fabfilin HER2/CD3.** In a further embodiment, binding molecules of the invention comprise polypeptides specifically binding to HER2 and to CD3. The advantage of such fusion proteins is a crosslinking of cancer and immune cells represented by a combination of anti CD3/anti HER2. It was surprisingly found that a bispecific binding molecule is able to bind specifically to both targets, e.g. that the bispecific binding molecule is able to bind simultaneously to HER2 and to CD3.

All Mabfilins or Fabfilins are exemplified in the Figures and Examples, as described below. In addition, such constructs can be easily expressed in conventional expression systems.

**Methods of identification of Affilin molecules (mutagenesis).** By way of example, starting point for the mutagenesis can be for example the cDNA or genomic DNA of ubiquitin according to SEQ ID NOs: 1-4 or of ubiquitin of at least 95% identity to SEQ ID NO: 1 or SEQ ID NO: 4. Furthermore, the gene coding for the ubiquitin protein can also be prepared synthetically. The DNA of ubiquitin according to SEQ ID NOs: 1-4 (or DNA of ubiquitin of at least 95% identity to SEQ ID NO: 1 or SEQ ID NO: 4), can be prepared, altered, and amplified by methods known to those skilled in the art. Different procedures known *per se* are available for mutagenesis, such as methods for site-specific mutagenesis, methods for random mutagenesis, mutagenesis using PCR or similar methods. All methods are known to those skilled in the art.

In a preferred embodiment of the invention the amino acid positions to be mutagenized are predetermined. In each case, a library of different mutants is generally established which is screened using methods known *per se.* Generally, a pre-selection of the amino acids to be modified can be performed based on structural information available for the ubiquitin protein to be modified. The selection of different sets of amino acids to be randomized leads to different libraries.

**Selection of Affilin molecules.** The gene pool libraries obtained as described above can be combined with appropriate functional genetic elements which enable expression of proteins for selection methods such as display methods. The expressed proteins are contacted with a target molecule to enable binding of the partners to each other if a binding affinity does exist. This process enables identification of those ubiquitin muteins which have a binding activity to the target molecule. See, for example, WO 2011/073214, WO 2011/073208, and WO 2011/073209 for more details of the selection method.

Contacting is preferably performed by means of a suitable presentation and selection method such as the phage display, ribosomal display, mRNA display or cell surface display, yeast surface display or bacterial surface display methods, preferably by means of the phage display method. For complete disclosure, reference is made also to the following references: Hoess, Curr. Opin. Struct. Biol.. 3 (1993), 572-579; Kay et al., Phage Display of Peptides and Proteins-A Laboratory Manual (1996), Academic Press. The methods mentioned above are known to those skilled in the art and can be used in the invention including modifications thereof.

The determination whether the modified protein has a quantifiable binding affinity with respect to a predetermined binding partner can be performed preferably by one or more of the following methods: ELISA, surface plasmon resonance spectroscopy, fluorescence spectroscopic methods, flow cytometry, isothermal titration calorimetry, analytical ultracentrifugation, or others.

**Method of production.** Bispecific binding molecules of the invention may be prepared by any of the many conventional and well known techniques such as plain organic synthetic strategies, solid phase-assisted synthesis techniques or by commercially available automated synthesizers. On the other hand, they may also be prepared by conventional recombinant techniques alone or in combination with conventional synthetic techniques. Conjugates according to the present invention may be obtained by combining compounds by chemical methods, e.g. lysine or cysteine-based chemistry, as described herein above.

According to another aspect of the invention, an isolated polynucleotide encoding a binding protein of the invention is provided. Also encompassed are polypeptides encoded by the polynucleotides of the invention. The invention further provides an expression vector comprising the isolated polynucleotide of the invention, and a host cell comprising the isolated polynucleotide or the expression vector of the invention.

For example, one or more polynucleotides which encode for a bispecific or bivalent binding protein of the invention may be expressed in a suitable host and the produced binding protein can be isolated. Vectors comprising said polynucleotides are covered by the invention. In a further embodiment the invention relates to a vector comprising the nucleic acid molecule of the invention. A vector means any molecule or entity (e.g., nucleic acid, plasmid, bacteriophage or virus) that can be used to transfer protein coding information into a host cell.

The present invention furthermore relates to an isolated cell comprising the nucleic acid molecule of the invention or the vector of the invention. Suitable host cells include prokaryotes or eukaryotes. Various mammalian or insect cell culture systems can also be employed to express recombinant proteins.

The invention also relates in an embodiment to a host cell or a non-human host carrying the vector of the invention. A host cell is a cell that has been transformed, or is capable of being transformed, with a nucleic acid sequence and thereby expresses a gene of interest. The term includes the progeny of the parent cell, whether or not the progeny is identical in morphology or in genetic make-up to the original parent cell, so long as the gene of interest is present.

In accordance with the present invention, the host may be a transgenic non-human animal transfected with and/or expressing the proteins of the present invention. In a preferred embodiment, the transgenic animal is a non-human mammal.

In another aspect, provided is a method of producing the binding protein of the invention, comprising the steps of a) culturing the host cell of the invention under conditions suitable for the expression of the binding protein and b) isolating the produced binding protein. The invention also relates to a binding protein produced by the method of the invention. Suitable conditions for culturing a prokaryotic or eukaryotic host are well known to the person skilled in the art.

One aspect of the present invention is directed to a method for the preparation of a binding protein according to the invention as detailed above, said method comprising the following steps: a. preparing a nucleic acid encoding a binding protein as defined above, b. introducing said nucleic acid into an expression vector; c. introducing said expression vector into a host cell; d. cultivating the host cell; e. subjecting the host cell to culturing conditions under which a fusion protein is expressed, thereby producing a fusion protein as described above; f. optionally isolating the protein produced in step (e), g. optionally conjugating the protein with further functional moieties as described above. Cultivation of cells and protein expression for the purpose of protein production can be performed at any scale, starting from plates and tubes over small volume shaker flasks to large fermenters, applying technologies well-known to any skilled in the art.

Following the expression of the ubiquitin protein modified as described herein, it can be further purified and enriched by methods known *per se.* The selected methods depend on several factors known *per se* to those skilled in the art, for example the expression vector used, the host organism, the intended field of use, the size of the protein and other factors.

In general, isolation of purified protein from the cultivation mixture can be performed applying conventional methods and technologies well known in the art, such as centrifugation, precipitation, flocculation, different embodiments of chromatography, filtration, dialysis, concentration and combinations thereof, and others.

For simplified purification, the protein modified as described herein can be fused to other peptide sequences having an increased affinity to separation materials. Preferably, such fusions are selected that do not have a detrimental effect on the functionality of the ubiquitin mutein or can be separated after the purification due to the introduction of specific protease cleavage sites. Such methods are also known to those skilled in the art.

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook J, Russell DW, (2001), Molecular Cloning: A laboratory manual. 3rd ed., Cold Spring Harbor Laboratory Press, New York.

**Methods for characterization of the binding proteins.** The further characterization of binding proteins of the invention can be performed in the form of the isolated, soluble proteins. The appropriate methods are known to those skilled in the art or described in the literature. Such methods include the determination of physical, biophysical and functional characteristics of the proteins. The affinity and specificity of the variants isolated can be detected by means of biochemical standard methods such as SPR analysis or ELISA as known to those skilled in the art and as discussed above and in the Examples. For stability analysis, for example spectroscopic or fluorescence-based methods in connection with chemical or physical unfolding are known to those skilled in the art, including e.g. "differential scanning fluorimetry" (DSF). Functional characterization can be performed in appropriate cell-based assays or *in vivo* experiments. Exemplary methods for characterization of binding proteins are discussed above and outlined in the Examples section of the present specification.

**Uses of the binding proteins of the invention.** In a further aspect of the invention, a binding protein or conjugate is used in medicine, in particular for use in medical treatment or diagnosis, preferably in cancer or in autoimmune diseases. Furthermore, the binding proteins of the invention can be used for preparing diagnostic means for *in vitro* or *in vivo* use as well as therapeutic means. The proteins according to the invention can be used as direct effector molecules (modulator, antagonist, agonist) or antigen-recognizing molecules.

The membrane protein EGFR is known to be upregulated in tumor cells, resulting in uncontrolled growth of tumor cells and in the formation of metastases. New therapies for cancer patients include an inhibition of EGFR by targeted therapeutics such as for example the monoclonal antibodies Cetuximab or Panitumumab. An advantage of the binding proteins of the invention is the improved clustering of receptors upon binding of EGFR at multiple epitopes compared to antibody. This leads to enhanced downregulation of the EGFR signaling pathway and thus improved therapeutic efficacy.

The membrane protein HER2 is also known to be upregulated in tumor cells, in particular in breast cancer. New therapies for cancer patients include an inhibition of HER2 by targeted therapeutics such as for example the monoclonal antibodies Trastuzumab or Pertuzumab. It is well known that inhibition of several targets can significantly improve therapeutic efficacy of the treatment, compared to single target treatment alone. Thus, binding proteins of the invention binding two targets at the same time are superior to single, mono-specific agents such as conventional monoclonal antibodies.

The pharmaceutical composition comprising the binding proteins of the invention can be used for treatment of cancer in which EGFR and/or HER2 is relevant for the development of the disease including but not limited to colorectal, breast, lung, head and neck, ovarian, cervical, prostate, pancreatic cancer.

CD3 is expressed on immune cells, i.e. on T cells, and is known to be involved in the activation of T-cells. Targeting of CD3 with antibodies or fragments has effects on T-cell responses. PD1 is expressed on thymocytes and on activated T-cells, B-cells and macrophages exploiting a negative regulation in immune responses. Upon stimulation of T-cells, T-cells and the antigen-presenting cell are in close to each other (immunological synapse). PD1 plays a role in autoimmune diseases, for example in autoimmune diabetes, systemic lupus erythematosus, rheumatoid arthritis, Sjögren's syndrome, or multiple sclerosis. As a modulator of the immune system PD-1 also seems to be involved in chronic viral infections like HIV or HCV. It is also known that tumor cells presenting PD-1 are able to escape from the immune system.

The advantage of bispecific PD1-CD3-binding molecules is the specific targeting to T-cells by the CD3-Fab-fragment and simultaneously binding to PD1 on these cells (PD1-Affilin). It is expected that the resulting effect of the simultaneous binding results in a downregulation of the activity of T-cells .The bispecific anti-CD3/anti-PD1 fusion proteins of the invention and pharmaceutical compositions comprising the binding proteins of the invention can be used particularly for treatment of autoimmune diseases or transplantations.

The advantage of bispecific HER2-CD3-binding molecules is the specific targeting to T-cells by the CD3-antibody or CD3-Fab-fragment and simultaneously binding to HER2 on tumor cells (Affilin). The bispecific anti-CD3/anti-HER2 fusion proteins of the invention and pharmaceutical compositions comprising the binding proteins of the invention can be used particularly for treatment of cancer.

The compositions are adapted to contain a therapeutically or diagnostically effective dose of the binding protein of the invention. The amount of protein to be administered depends on the organism to be treated, the type of disease, the age and weight of the patient and further factors known *per se.*

The invention covers a pharmaceutical composition containing the binding protein or conjugate or a combination or the nucleic acid molecule of the invention, the vector of the invention, and/or the host cell or non-human host thereof and a pharmaceutically acceptable carrier. Further described herein is a diagnostic agent comprising the binding protein or conjugate or the nucleic acid molecule of the invention, the vector of the invention, and/or the host cell or non-human host with a diagnostically acceptable carrier. The compositions contain a pharmaceutically or diagnostically acceptable carrier and optionally can contain further auxiliary agents and excipients known *per se.* These include for example but are not limited to stabilizing agents, surface-active agents, salts, buffers, coloring agents etc.

The pharmaceutical composition comprising the binding protein can be in the form of a liquid preparation, a lyophilisate, a cream, a lotion for topical application, an aerosol, in the form of powders, granules, tablets, suppositories, or capsules, in the form of an emulsion or a liposomal preparation. The compositions are preferably sterile, non-pyrogenic and isotonic and contain the pharmaceutically conventional and acceptable additives known *per se.* Additionally, reference is made to the regulations of the U.S. Pharmacopoeia or Remington's Pharmaceutical Sciences, Mac Publishing Company (1990).

In the field of human and veterinary medical therapy and prophylaxis pharmaceutically effective medicaments containing at least one binding protein in accordance with the invention can be prepared by methods known *per se.* Depending on the galenic preparation these compositions can be administered parenterally by injection or infusion, systemically, rectally, intraperitoneally, intramuscularly, subcutaneously, transdermally or by other conventionally employed methods of application. The type of pharmaceutical preparation depends on the type of disease to be treated, the route of administration, the severity of the disease, the patient to be treated and other factors known to those skilled in the art of medicine.

A pharmaceutical composition according to the invention may be present in the form of a composition, wherein the different active ingredients and diluents and/or carriers are admixed with each other, or may take the form of a combined preparation, where the active ingredients are present in partially or totally distinct form. A suitable carrier or excipient may be a liquid material which can serve as a vehicle or medium for the active ingredient. An example for such a combination or combined preparation is a kit-of-parts.

In a still further aspect the specification discloses diagnostic compositions comprising binding proteins according to the invention specifically binding specific targets/antigens or its isoforms together with diagnostically acceptable carriers.

Since enhanced EGFR and HER2 expression is correlated with tumor malignancy, it is desirable to develop diagnostics for non-invasive imaging in order to gain information about EGFR and HER2 expression status in patients. Furthermore, EGFR and HER2 imaging could be useful for the assessment of the response of a patient to a therapeutic treatment. For example, using a protein of the invention labelled with a suitable radioisotope or fluorophore can be used for non-invasive imaging to determine the location of tumors and metastasis (for review see for example Milenic et al. 2008 Cancer Biotherapy & Radiopharmaceuticals 23: 619-631; Hoeben et al., 2011, Int. Journal Cancer 129: 870-878). Due to their pharmacokinetic characteristics, intact antibodies are not suitable for routine imaging. Due to their small size and high affinity, radiolabelled or fluorescently labelled binding proteins of the invention comprising antibody fragments but not full antibodies are expected to be much better suited for use as diagnostics for imaging.

It is expected that a fusion protein of the invention can be advantageously applied in therapy. In particular, the molecules are expected to show superior tumor targeting effect and desired biodistribution and thus, reduced side effects.

Pharmaceutical compositions of the invention may be manufactured in any conventional manner.

### EXAMPLES

The following Examples are provided for further illustration of the invention.

The invention, however, is not limited thereto, and the following Examples merely show the practicability of the invention on the basis of the above description. For a complete disclosure of the invention reference is made also to the literature cited in the application.

### Example 1. Expression and purification of Mabfilin proteins (EGFR/EGFR and HER2/EGFR)

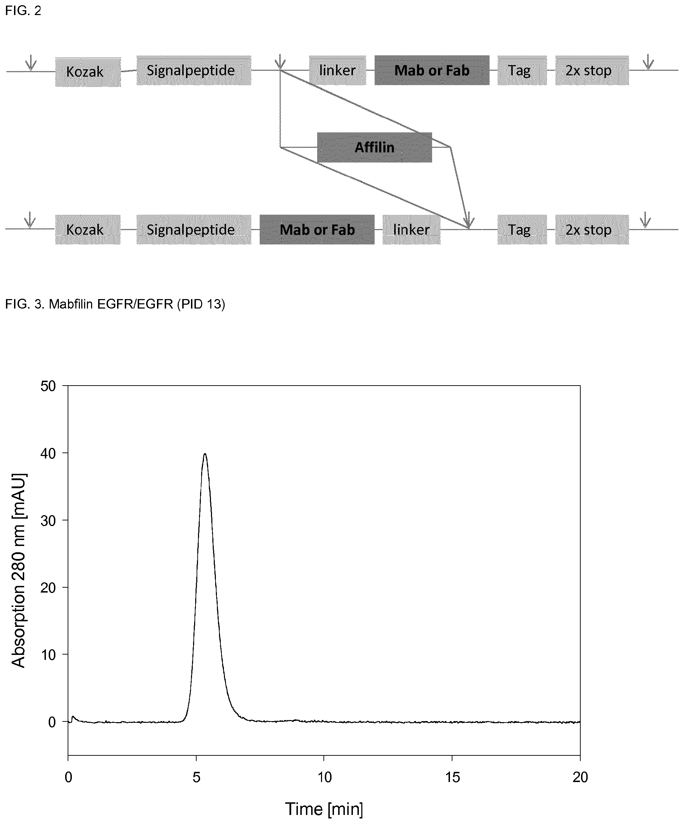

The cloning strategy for fusion proteins with an Affilin and a monoclonal antibody is outlined in Figure 2. The figure shows a schematic representation for preferred gene expression cassettes for the mammalian expression of the binding proteins of the invention (shown in 5' to 3' direction). The expression cassette comprises a Kozak-sequence, a suitable signal peptide, a sequence for the heavy or light chain of the monoclonal antibody ("Mab"), a suitable linker, a suitable Tag for purification and immunological detection, and two stop codons (2x stop). Each expression cassette is flanked by a restriction side (illustrated by an arrow). The first binding protein (Affilin) is inserted as shown. Any ubiquitin mutein or other binding protein could be inserted according to this expression strategy.

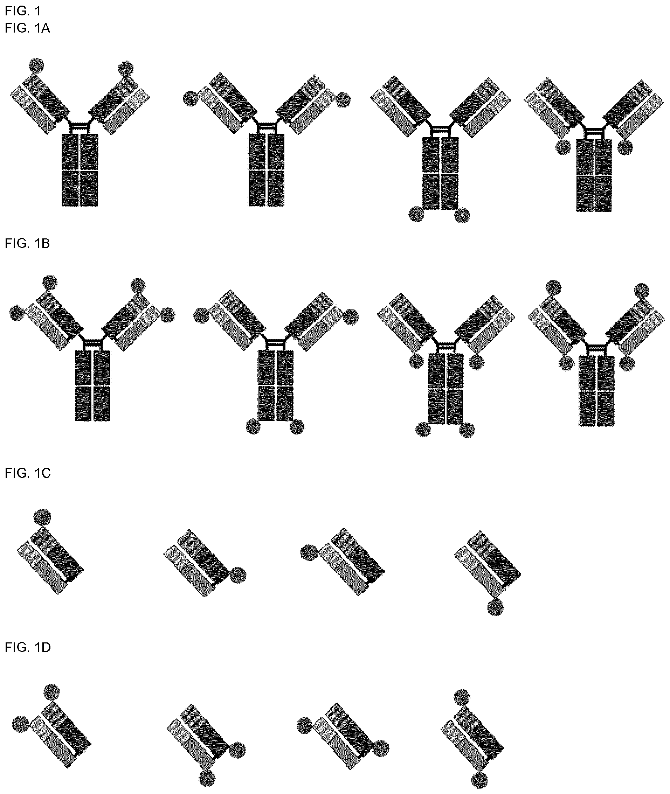

EGFR specific Affilin-139791 (SEQ ID NO:7), Affilin-139864 (Seq ID NO: 8), Affilin-139819 (SEQ ID NO: 9) or HER2 specific Affilin (Affilin-141926, SEQ ID NO: 10) were genetically fused to the anti-EGFR binding antibody (heavy chain Cetuximab SEQ ID NO: 5; light chain Cetuximab SEQ ID NO: 6) via a 15 amino acid linker (SEQ ID NO: 31) yielding the Affilin-antibody binding proteins of the present invention. For the expression of heavy chain Cetuximab or fusion proteins with heavy chain Cetuximab (SEQ ID NO: 5), signal sequence MAVLGLLFCLVTFPSCVLS (SEQ ID NO: 41) was used. For the expression of light chain Cetuximab or fusion proteins with light chain Cetuximab (SEQ ID NO: 6), signal sequence MVSTPQFLVFLLFWIPASRS (SEQ ID NO: 42) was used. For specific purification and detection, a tag of 6 histidin residues (SEQ ID NO: 50) was added to a fusion protein with a light antibody chain and a strep-tag (SEQ ID NO: 51) was added to a fusion protein with a heavy antibody chain. Binding proteins as outlined in Figure 1 were expressed: for example, the anti-EGFR Affilin (for example, Affilin-139791, -139864, -139819) or the anti-HER2 Affilin (for example, Affilin-141926) was fused to the N-terminus of the heavy chain of the anti EGFR antibody (examples: PID 11, PID 12, PID 13, PID 23); to the C- terminus of the heavy chain of the anti EGFR antibody (examples: PID 14, PID 15, PID 16, PID 24); to the N-terminus of the light chain of the anti EGFR antibody (examples: PID 17, PID 18, PID 19, PID 25); and to the C- terminus of the light chain of the anti EGFR antibody (examples: PID 20, PID 21, PID 22, PID 26). See Table 1 for a further explanation of PID.

For each construct, a control fusion protein with unmodified ubiquitin (SEQ ID NO: 2) was used instead of the target-specific Affilin (control fusion proteins, see for example SEQ ID NO: 27-30 (PID 27, PID 28, PID 29, PID 30).

Further complex binding proteins were expressed using methods known to those skilled in the art and as described in further detail below: an EGFR-specific Affilin is linked to the N-terminus of the heavy chain of Cetuximab and an EGFR-specific Affilin is linked to the N-terminus of the light chain of Cetuximab (PID 31); an EGFR-specific Affilin is linked to the C-terminus of the heavy chain of Cetuximab and an EGFR-specific Affilin is linked to the N-terminus of the light chain of Cetuximab (PID 32); an EGFR-specific Affilin is linked to the N-terminus of the heavy chain of the Cetuximab and an EGFR-specific Affilin is linked to the C-terminus of the light chain of Cetuximab (PID 33); an EGFR-specific Affilin is linked to the C-terminus of the heavy chain of Cetuximab and an EGFR-specific Affilin is linked to the C-terminus of the light chain of Cetuximab (PID 34).

The DNA for the expression of Cetuximab (SEQ ID NOs: 5 and 6), and the binding proteins as outlined above were transiently transfected into FreeStyle™ 293-F cells and expressed in serum-free/animal component-free media. FreeStyle™ 293 F cells (1 x 10⁶ cells) were cultured in a 24 deep-well plate for 24 h in 2 mL FreeStyle™ 293 F expression medium for 24 h. The cells were transfected with the expression vectors and polyethylenimin. Detection of Cetuximab antibody light chain fused His-tag in supernatant after 72 h of expression was realized by Penta-His-Antibody- or detection of the heavy chain fusion by anti-Strep-tag Antibody- , respectively in combination with a second HRP conjugated antibody, in western blot analysis. Expression for each fusion protein was confirmed by western blot analysis.

Proteins were isolated from the supernatants by Fc-specific purification followed by Protein A affinity chromatography (GE-Healthcare cat no 17-0402-01) with an ÄKTAxpress® (GE Healthcare) and gel filtration. Further analysis included SDS-PAGE, SE-HPLC and RP-HPLC using standard protocols known to those skilled in the art. Protein concentrations were determined by absorbance measurement at 280 nm. Affilin antibody binding proteins and control antibodies could be expressed and purified. Yields are listed in Table 2.

**Table 2. Expression yields (Mabfilin proteins)**

| **PID** | **fusion site** | **target Affilin** | **target mAB** | **concentration [mg/mL]** | **purified protein mass [mg]** | |
|---|---|---|---|---|---|---|
| 13 | NH | EGFR | EGFR | 0.277 | 2.216 | |
| 16 | CH | EGFR | EGFR | 0.129 | 1.032 | |
| 19 | NL | EGFR | EGFR | 0.398 | 3.98 | |
| 22 | CL | EGFR | EGFR | 0.355 | 3.685 | |
| 23 | NH | HER2 | EGFR | 0.601 | 1.359 | |
| 24 | CH | HER2 | EGFR | 0.199 | 0.315 | |
| 25 | NL | HER2 | EGFR | 0.23 | 0.4086 | |
| 26 | CL | HER2 | EGFR | 0.156 | 0.604 | |
| 35 | NH/CL | EGFR | EGFR | 0.146 | 1.022 | |

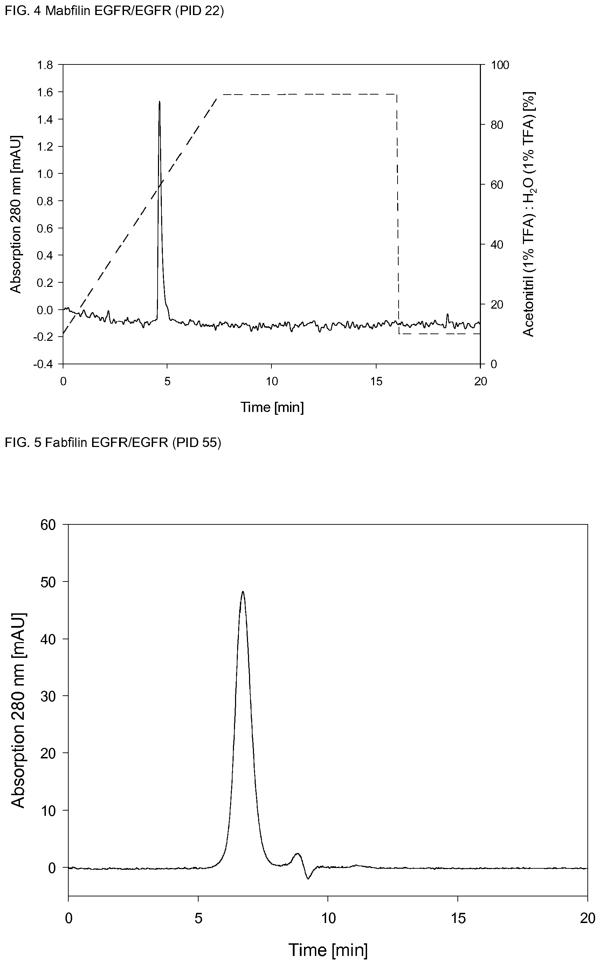

After purification, analytical size exclusion chromatography (SE-HPLC) was performed using a Dionex HPLC system and a Superdex™ 200 5/150 GL column (Grace). Analytical reversed phase chromatography (RP-HPLC) was performed using a Dionex HPLC system and a Vydac 214MS54 C4 (4.6 x 250 mm, 5 µm, 300 Å) column (GE Healthcare). Examples are shown in Figure 3 and Figure 4. The bispecific proteins eluted as a single monomeric peak confirming favorable biophysical properties. No impurities and product-intermediates were detected.

### Example 2. Production of Fabfilin proteins (HER2/EGFR, EGFR/EGFR)

Affilin molecules were fused to different sites of Fab fragment of Cetuximab. Fabfilin proteins were generated, wherein the HER2 binding Affilin (Affilin-141926, SEQ ID NO: 10) and EGFR binding Affilin (Affilin-139819, SEQ ID NO: 9) were genetically fused to the anti-EGFR Fab-fragment (SEQ ID NO: 6, SEQ ID NO: 52) via 15 amino acid (Gly₄Ser)₃ peptide linkers (SEQ ID NO: 31) yielding the bispecific Fab-fragment fusion proteins (Fabfilin). The cloning strategy for Fabfilin proteins (Fab) is analog to the one described for Mabfilin proteins (Figure 2). The Anti-HER2 Affilin (SEQ ID No: 10) ,the anti EGFR-Affilin (SEQ ID NO: 9) and suitable controls were fused to the N-terminus of the heavy chain of the Cetuximab-Fab-fragment (PID 49, PID 53, PID 57, PID 61), to the C- terminus of the heavy chain of the Cetuximab-Fab-fragment (PID 50, PID 54, PID 58, PID 62), to the N-terminus of the light chain Cetuximab-Fab-fragment (PID 51, PID 55, PID 59, PID 63), and to the C- terminus of the light chain of Cetuximab-Fab-fragment (PID 52, PID 56, PID 60, PID 64). Instead of a first binding protein (Affilin), control fusion proteins were constructed with di-ubiquitin (SEQ ID No: 4) and ubiquitin (SEQ ID NO: 2). Fabfilins were produced by transient mammalian expression using Expi293™ Expression System (Thermo Scientific) as described for Mabfilins in Example 1. Production of binding proteins was detected by SDS-PAGE 6 days after transfection. For each fusion protein, expression could be confirmed. The proteins of the invention were purified from supernatant by one-step purification using KappaSelect column matrix (GE Healthcare) and SigmaPrep spin column (Sigma Aldrich). Analysis included SDS-PAGE, SE-HPLC and RP-HPLC. Protein concentrations were determined by absorbance measurement at 280 nm. Yields are listed in Table 3.

**Table 3. Yields of Fabfilin proteins after purification**

| **PID** | **fusion site** | **target of Affilin** | **target of Fab** | **purified fusion protein [mg]** | **concentration [mg/mL]** |
|---|---|---|---|---|---|
| 52 | CL | HER2 | EGFR | 0.520 | 0.304 |
| 56 | CL | EGFR | EGFR | 0.450 | 0.263 |
| 51 | NL | HER2 | EGFR | 0.368 | 0.215 |
| 55 | NL | EGFR | EGFR | 0.415 | 0.243 |
| 50 | CH | HER2 | EGFR | 0.716 | 0.418 |
| 49 | NH | HER2 | EGFR | 0.416 | 0.244 |

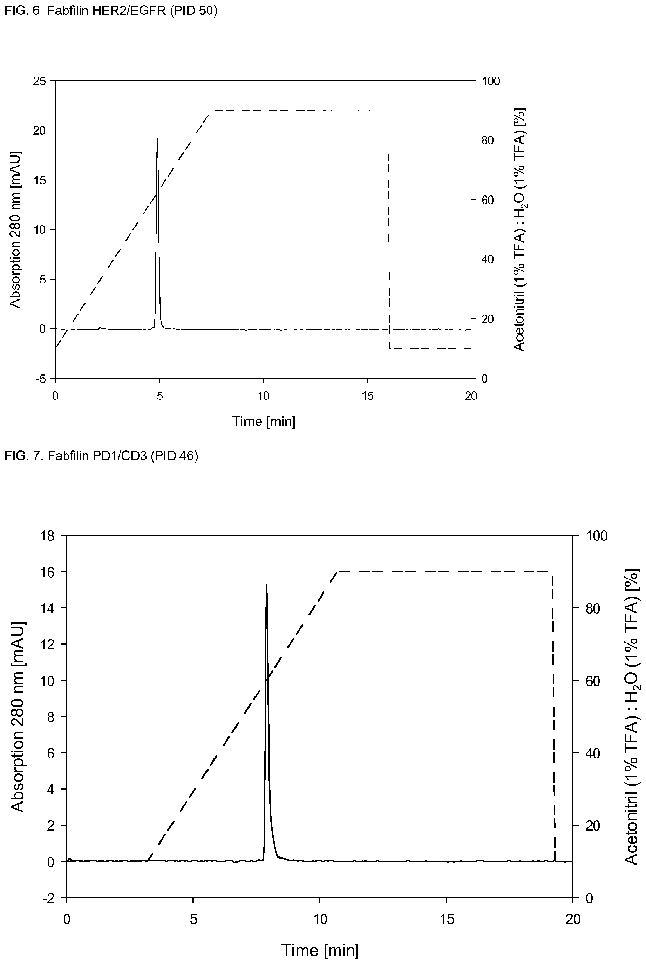

After purification, SE-HPLC was performed using a Dionex HPLC system and a Superdex™ 5/150 GL (GE Healthcare) and RP-HPLC using a Dionex HPLC system and Vydac 214MS54 C4 (4.6 x 250 mm, 5 µm, 300 Å) column (Grace). The elution as single peak from SE-HPLC and RP-HPLC demonstrated that the binding proteins are found in a most favorable conformation/fold (Figure 5, Figure 6).

### Example 3. Expression and purification of Mabfilin and Fabfilin HER2/CD3 proteins

Fab fragments of the monoclonal antibody 145-2C11 are specific for the cell surface protein T3-epsilon of CD3. Alternatively, the anti-CD3-monoclonal antibody OKT3 (Muromonab) was used. Both antibodies bind to the ε-chain of CD3. 145-2C11 and OKT3 are able to modify the activity of T-cells. Other anti-CD3-antibodies such as but not limited to KT3, 17A2, UCHT1, MOM-18160-F would also be suitable.

HER2 binding Affilin (Affilin-142628, SEQ ID No: 63) was genetically fused to the anti CD3 antibody (PID 66) or Fab-fragment (PID 67) via 15 amino acid (Gly4Ser)₃ peptide linkers (SEQ ID NO:31) yielding the bispecific antibody or Fab-fragment fusion proteins. Several binding proteins were expressed (PID 68, PID 69, PID 70, PID 71, PID 72, PID 73, PID 74, PID 75, PID 76, PID 77, PID 78, PID 79, PID 80, PID 81, PID 82, PID 83). For expression, the sequences included suitable signal sequences (OKT3 light chain and fused OKT3 light chain (SEQ ID NOs: 67, 71, 72, 75 and 76) included signal peptide SEQ ID NO: 65; OKT3 heavy chain, OKT3 Fab heavy chain and fused OKT3 (Fab) heavy chains included signal peptide SEQ ID NO: 64). For detection and specific purification, light chains were tagged with His-Tag-sequences (6x His, SEQ ID NO: 50) and heavy chains were tagged with Strep-Tag (WSHPQFEK, SEQ ID NO: 51). The fusion molecules of the invention, OKT3 antibody and Fab fragment and control proteins were produced by transient mammalian expression using Expi293™ Expression System (Thermo Scientific) similar to described methods in Example 2. Production of binding proteins was detected by SDS-PAGE 6 days after transfection. For each fusion protein, expression could be confirmed.

The proteins were purified from supernatant by immobilized metal ion affinity chromatography (IMAC) using Ni-NTA affinity column (GE Healthcare) with an AKTAprime® system followed by gelfiltration via Superdex 200 matrix using an ÄKTAvant® system. Analysis included SDS-PAGE, SE-HPLC and RP-HPLC. Protein concentrations were determined by absorbance measurement at 280 nm.

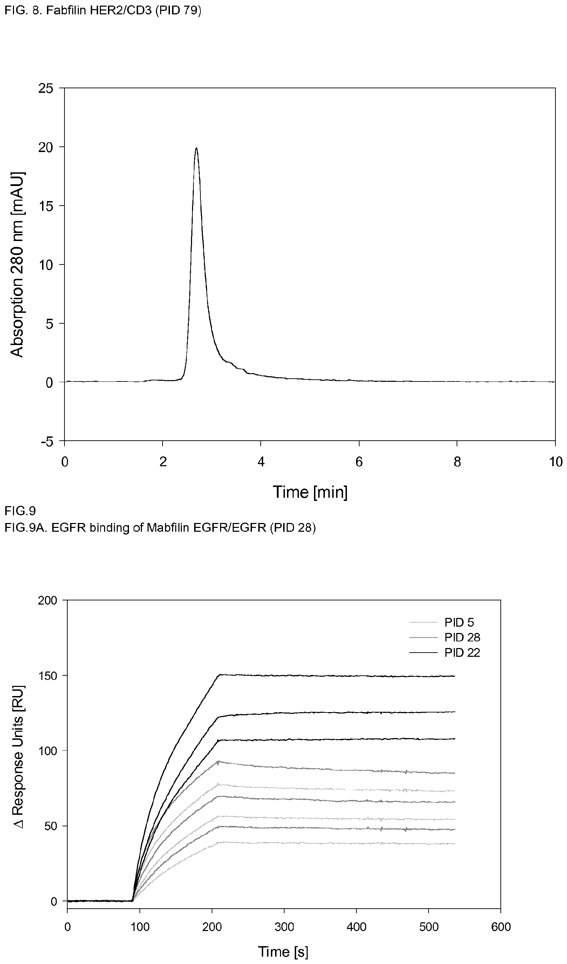

After purification size exclusion chromatography (SE-HPLC) has been performed using a Dionex HPLC system and a Superdex™ 200 5/150 GL (GE Healthcare). RP chromatography (RP-HPLC) has been performed using a Dionex HPLC system and Vydac 214MS54 C4 (4.6 x 250 mm, 5 µm, 300 Å) column (Grace). The elution as single peak from SE-HPLC and RP-HPLC confirmed the most favorable conformation/fold of the fusion proteins (Figure 8). All binding proteins can be expressed and highly purified by chosen purification strategy via Ni-NTA affinity chromatography and gelfiltration.

### Example 4. Expression and purification of Fabfilin PD1/CD3 proteins

The PD1-specific Affilin (Affilin 18, SEQ ID NO: 43) has been genetically fused to the anti CD3 specific Fab-fragment (light chain: SEQ ID NO: 44, heavy chain: SEQ ID NO: 45) via 20 to 23 amino acid peptide linkers yielding the bispecific Fab-fragment fusion proteins (Fabfilin). The cloning strategy is outlined in Figure 2. For Mabfilins, a *BsaI* restriction site for inserting the Affilin molecule into the vector was used. To facilitate fewer cloning steps, for Fabfilins PD1/CD3 *SphI* and *BlpI* restriction sites were used.

Several binding proteins as outlined in Figure 1C and Figure 1D were expressed: the PD1 specific Affilin (SEQ ID NO: 43, Affilin-128187) was fused to the N-terminus of the heavy chain of the anti-CD3-Fab-fragment (PID 43), to the C- terminus of the heavy chain of the anti-CD3-Fab-fragment (PID 42), to the N-terminus of the light chain of the anti-CD3-Fab-fragment (PID 41), and to the C- terminus of the light chain of the anti-CD3-Fab-fragment (PID 40). PD1-specific Affilin is linked to the N-terminus of the heavy chain and to the N-terminus of the light chain of the anti-CD3-Fab-fragment (PID 44), to the C-terminus of the heavy chain and to the N-terminus of the light chain of the CD3-Fab-fragment (PID 45), to the C-terminus of the heavy chain and to the C-terminus of the light chain of the anti-CD3-Fab-fragment (PID 46), or to the N-terminus of the heavy chain and to the C-terminus of the light chain of anti-CD3-Fab-fragment (PID 47). For expression and purification, the same signal sequences and tags as described in Example 1 were used.

The anti-CD3-Fab-fragment, the anti-PD1-Affilin, and Fabfilin PD1/CD3 proteins (PID 40, PID 41, PID 42, PID 43, complex proteins: PID 44, PID 45, PID 46, PID 47) were expressed in serum-free/animal component-free media. Production of fusion proteins was detected 8 days after transfection using *Strep*-Tactin®-HRP conjugate.

The proteins of the invention were initially purified from supernatant by Strep-Tactin affinity chromatography (IBA GmbH) on an ÄKTAxpress® system (GE Healthcare). Further purification of the binding proteins was conducted by CD3-affinity chromatography on previously CNBr-activated Sepharose 4B. Analysis included SDS-PAGE, SE-HPLC and RP-HPLC. Protein concentrations were determined by absorbance measurement at 280 nm. Fusion proteins have been expressed and purified; yields are listed in Table 4.

**Table 4. Expression yields of selected Fabfilin PD1/CD3 proteins after purification**

| **PID** | **fusion site** | **purified fusion protein [µg]** | **Concentration [µg/mL]** |
|---|---|---|---|
| 43 | NH | 81 | 180 |
| 42 | CH | 92 | 92 |
| 40 | CL | 110 | 140 |
| 46 | CH/CL | 221 | 185 |
| 47 | NH/CL | 177 | 136 |
| 45 | CH/NL | 37 | 82 |

After purification, SE-HPLC has been performed using a Dionex HPLC system and a TSKgel G3000 SW XLcolumn (Tosoh Bioscience LLC). RP chromatography (RP HPLC) has been performed using a Dionex HPLC system and Vydac 214MS54 C4 (4.6 x 250 mm, 5 µm, 300 Å) column. The elution as single peak from SE-HPLC and RP-HPLC demonstrated that the binding proteins are found in a most favorable conformation/fold (Figure 7). All bispecific PD1 - Affilin-CD3-Fab-fragment binding proteins can be expressed and highly purified by 2-step affinity chromatography.

### Example 5. Mabfilin and Fabfilin proteins are thermally stable

Thermal stability was determined by differential scanning fluorimetry. 2 µg of each probe were transferred to a MicroAmp® Optical 384-well plate, and SYPRO Orange dye was added at suitable dilution. A temperature ramp from 25 to 95 °C was programmed with a heating rate of 1 °C per minute (ViiA-7 Applied Biosystems). Fluorescence was constantly measured at an excitation wavelength of 520 nm and the emission wavelength at 623 nm (ViiA-7, Applied Biosystems). Mabfilin proteins for EGFR/EGFR and HER2/EGFR have thermal stabilities with thermal transition midpoints between Tm= 63.9 °C and 69.0 °C, Fabfilin proteins specific for EGFR/EGFR and HER2/EGFR have single conformation transitions at about 70°C, Fabfilin proteins specific for PD1/CD3 have thermal stabilities between Tₘ= 61.4°C and 63.6°C. The isolated Mabfilin and Fabfilin HER2/CD3 proteins have thermal stabilities between Tm = 62.5°C and 69.8°C. Table 5 shows the melting temperatures of Mabfilin or Fabfilin proteins compared to controls.

**Table 5. Melting temperatures of Mabfilin or Fabfilin proteins compared to controls**

| **PID** | **fusion side** | **target Affilin/Mab** | **target for Affilin/Fab** | **Tm [°C]** |
|---|---|---|---|---|
| 5 | n.a. | n.a./EGFR | | 69.0 |
| 13 | NH | EGFR/EGFR | | 68.6 |
| 16 | CH | EGFR/EGFR | | 67.9 |
| 19 | NL | EGFR/EGFR | | 69.0 |
| 22 | CL | EGFR/EGFR | | 65.8 |
| 23 | NH | HER2/EGFR | | 67.5 |
| 24 | CH | HER2/EGFR | | 67.4 |
| 25 | NL | HER2/EGFR | | 67.9 |
| 26 | CL | HER2/EGFR | | 63.9 |
| 35 | NH/CL | EGFR/EGFR | | 65.8 |
| 48 | n.a. | | n.a./EGFR | 72.1 |
| 52 | CL | | HER2/EGFR | 71.7 |
| 56 | CL | | EGFR/EGFR | 71.4 |
| 64 | CL | | n.a./EGFR | 72.1 |
| 59 | NL | | n.a./EGFR | 70.7 |
| 51 | NL | | HER2/EGFR | 70.2 |
| 55 | NL | | EGFR/EGFR | 71.0 |
| 63 | NL | | n.a./EGFR | 71.0 |
| 58 | CH | | n.a./EGFR | 72.1 |
| 50 | CH | | HER2/EGFR | 71.7 |
| 57 | NH | | n.a./EGFR | 72.1 |
| 49 | NH | | HER2/EGFR | 71.4 |
| 38 | n.a. | | n.a./CD3 | 62.1 |
| 43 | NH | | PD1/CD3 | 63.6 |
| 42 | CH | | PD1/CD3 | 62.4 |
| 40 | CL | | PD1/CD3 | 62.3 |
| 46 | CH/CL | | PD1/CD3 | 61.4 |
| 47 | NH/CL | | PD1/CD3 | 61.6 |
| 45 | CH/NL | | PD1/CD3 | 61.7 |
| 66 | n.a. | CD3 | | 69.8 |
| 67 | n.a. | | CD3 | 69.0 |
| 75 | CL | CD3 | | 68.8 |
| 83 | CL | | CD3 | 69.1 |
| 71 | CL | HER2/CD3 | | 62.6 |
| 79 | CL | | HER2/CD3 | 68.1 |

### Example 6. Mabfilin and Fabfilin (EGFR/EGFR; HER2/EGFR) proteins bind to both epitopes

A CM5 sensor chip (GE Healthcare) was equilibrated with SPR running buffer. Surface-exposed carboxylic groups were activated by passing a mixture of EDC and NHS to yield reactive ester groups. This enabled the immobilization of 700-1500 RU extracellular domain EGFR-Fc (on-ligand) or 700-1500 RU extracellular domain HER2-Fc (on-ligand), diluted in coupling buffer, on one of the flow cells. IgG-Fc (off-ligand) diluted in coupling buffer was immobilized on another flow cell at a ratio of 1:3 (hlgG-Fc:target) to the target. Ligand binding to the surface was achieved by covalent interactions between the ester groups of the matrix and the primary amine groups of the ligands. Injection of ethanolamine after ligand immobilization deactivates the remaining active ester groups on the surface. Non-covalently bound ligands were removed by the injection of a glycine-buffer (pH 2.0). Upon binding to the ligand, protein analyte accumulated on the surface increasing the refractive index. This change in the refractive index was measured in real time and plotted as response or resonance units (RU) versus time. The analytes were applied to the chip in serial dilutions with a flow rate of 30 µl/min. Binding studies were carried out by the use of the Biacore® 3000 (GE Healthcare); data evaluation was operated via the BIAevaluation 3.0 software, provided by the manufacturer, by the use of the Langmuir 1:1 model (RI=0). All HER2/EGFR binding proteins bind with high affinity to both targets EGFR and HER2, confirming the bispecific nature of these proteins.

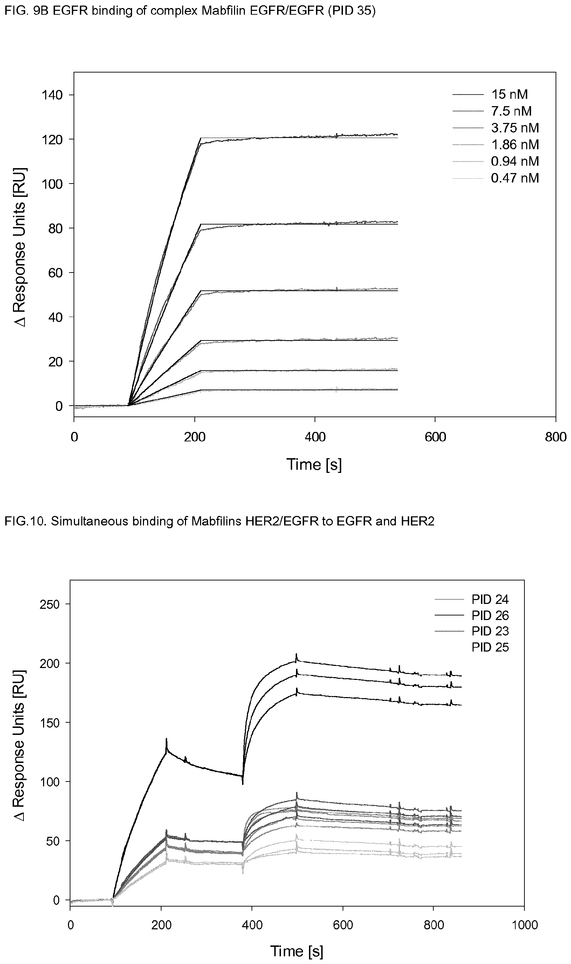

The sensorgrams of the Mabfilin EGFR/EGFR proteins are shown in Figure 9, evaluated dissociation constants (K_{D}) were standardized against off-target and indicated. Figure 9A shows high affinity of the fusion protein to the targets, in particular EGFR-specific Affilin fused to the C-terminus of the light chain of the antibody. Binding levels of the Mabfilins are higher compared to Cetuximab whereas EGFR binding of the control fusion protein is comparable to Cetuximab. Fusion of EGFR-specific Affilin to Cetuximab enhance the binding level of the fusion protein to EGFR, in particular fusions to the C-termini of the light or heavy chains.

Binding proteins of the invention further comprise *complex* Mabfilins EGFR-EGFR, i.e. fusion proteins of EGFR binding Affilins and Cetuximab, for example NH139791-NL139864 (SEQ ID NO: 11 and SEQ ID NO: 18), PID 32, NH139864-CH139864 (SEQ ID NO: 12 and SEQ ID NO: 15), PID 34, PID 31, PID 33, or PID 35. Figure 9B shows the measurement of binding-rate values of the complex Mabfilin EGFR/EGFR fusion protein which is fused to the C-terminus of the light chain and to the N-terminus of the heavy chain of Cetuximab. All binding proteins of the invention bind with high affinity to their respectively target. Particularly binding proteins, wherein the Affilin is fused to the C-terminus of the light chain, show higher binding levels than Cetuximab representing higher binding affinities. Table 6 shows the binding of Mabfilin HER2/EGFR to both targets.

### Table 6: Binding of Mabfilin HER2/EGFR proteins to both targets

**Table 6A: Binding of Mabfilin proteins to EGFR**

| **PID** | **fusion site** | **kₒₙ [M⁻¹** x **s⁻¹]** | **k_{off} [s⁻¹]** | **K_{D} [M]** |
|---|---|---|---|---|
| Cetuximab | - | 6.21 x 10⁵ | 6.29 x 10⁴ | 1.01 x 10⁻⁹ |
| 23 | NH | 3.79 x 10⁵ | 4.12 x 10⁻⁴ | 1.09 x 10⁻⁹ |
| 24 | CH | 4.66 x 10⁵ | 1.65 x 10⁻⁴ | 3.53 x 10⁻¹⁰ |
| 25 | NL | 1.08 x 10⁵ | 1.72 x 10⁻⁴ | 1.59 x 10⁻⁹ |
| 26 | CL | 5.84 x 10⁵ | 5.91 x 10⁻⁴ | 1.01 x 10⁻⁹ |

**Table 6B: Binding of Mabfilin proteins to Her2**

| **PID** | **fusion site** | **kₒₙ [M⁻¹ x s⁻¹]** | **k_{off} [s⁻¹]** | **K_{D} [M]** |
|---|---|---|---|---|
| 23 | NH | 9.03 x 10⁴ | 3.05 x 10⁻⁴ | 3.37 x 10⁻⁹ |
| 24 | CH | 7.53 x 10⁴ | 4.96 x 10⁻⁴ | 6.58 x 10⁹ |
| 25 | NL | 8.23 x 10⁴ | 2.36 x 10⁻⁴ | 2.87 x 10⁻⁹ |
| 26 | CL | 3.11 x 10⁵ | 4.46 x 10⁻⁴ | 1.43 x 10⁻⁹ |

A co-expression of the receptor proteins EGFR and HER2 in various forms of cancer (e.g. breast, colorectal and prostate cancer) is associated with poor prognosis for the patients. In Figure 10, a chip with immobilized extracellular domain HER2-Fc was used. Mabfilin HER2/EGFR was injected (extracellular domain HER2-Fc-coupled chip), after 350 sec, extracellular domain EGFR-Fc was injected at concentrations between 100 nM and 25 nM in 1:2 dilution series. Figure 10 shows the simultaneous binding of Mabfilin variants to both targets which might increase selectivity and efficiency particularly in medical applications, for example in molecular imaging.

Table 7A shows affinity data for Fabfilin proteins (EGFR/EGFR and HER2/EGFR) for EGFR, Table 7B for Fabfilin proteins (HER2/EGFR) for HER2. All binding proteins bind with high affinity to EGFR or EGFR and HER2 respectively.

**Table 7A: Binding of Fabfilin proteins to EGFR**

| **PID** | **fusion site** | **target affilin/Fab** | **Kₒₙ [(M*s)⁻¹]** | **K_{off} [s⁻¹]** | **K_{D} [nM]** |
|---|---|---|---|---|---|
| Cetux. Fab | - | EGFR | 16,3x10⁵ | 2,15x10⁻³ | 1,32 |
| 49 | NH | HER2/EGFR | 5,57x10⁵ | 1,71x10⁻³ | 3,07 |
| 51 | NL | HER2/EGFR | 3,02x10⁵ | 0,658x10⁻³ | 2,18 |
| 55 | NL | EGFR/EGFR | 4,29x10⁵ | 0.188x10⁻³ | 0,439 |
| 50 | CH | HER2/EGFR | 10,3x10⁴ | 1,86x10⁻³ | 1,8 |
| 52 | CL | HER2/EGFR | 12x10⁵ | 1,14x10⁻³ | 0,946 |
| 56 | CL | EGFR/EGFR | 8,5x10⁵ | 0.2x10⁻³ | 0,235 |

**Table 7B: Binding of Fabfilin proteins to HER2-Fc**

| **PID** | **fusion site** | **Target affilin/Fab** | **Kₒₙ [(M*s)⁻¹]** | **K_{off} [s⁻¹]** | **K_{D}d [nM]** |
|---|---|---|---|---|---|
| 49 | NH | HER2/EGFR | 0,359x10⁵ | 7,03x10⁻³ | 195 |
| 51 | NL | HER2/EGFR | 0,234x10⁵ | 5,54x10⁻³ | 237 |
| 50 | CH | HER2/EGFR | 0,824x10⁵ | 8,27x10⁻³ | 99,8 |
| 52 | CL | HER2/EGFR | 1,49 x 10⁵ | 3,87x10⁻³ | 25,9 |

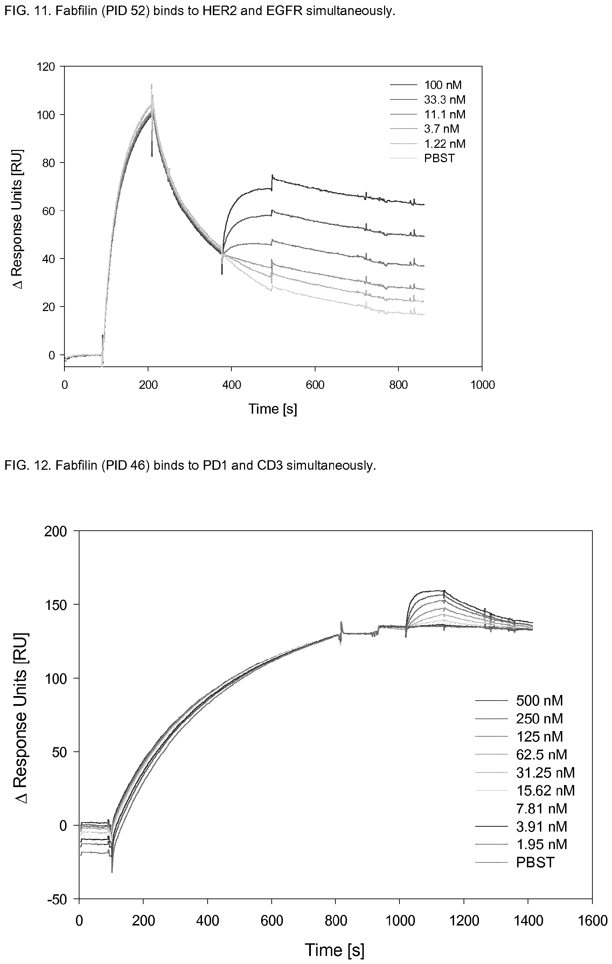

For simultaneous binding of both targets a chip with immobilized HER2 was used, 150 s after the injection of the binding proteins, soluble EGFR was injected and the dissociation was monitored for 300 s. Figure 11 shows the simultaneous binding of Fabfilin proteins to both targets.

### Example 7. Fabfilin PD1/CD3 proteins bind both targets simultanously

For SPR-analysis of PD1/CD3, a CM5 sensor Chip (GE Healthcare) with immobilized target PD1 (about 800 RU) was prepared. CD3 was 10 times biotinylated and immobilized on SA coupled sensor chip (GE Healthcare) of about 200 RU. Specific binding of biotin and streptavidin provides a strong interaction of the target protein on the chip. Injection of free biotin blocks unbound streptavidin avoiding unspecific interaction during measurement. Determination of dissociation constant and data evaluation was equal to Example 6. All binding proteins bind with high affinity to the target protein. Table 8 shows affinity data for Fabfilin PD1/CD3 proteins. All binding proteins bind with high affinity to both targets PD1 and CD3, confirming the bispecificity.

### Table 8. Binding of Fabfilin PD1/CD3 protein

**Table 8A: for PD1**

| **PID** | **Fusion site** | **kₒₙ [M⁻¹ x s⁻¹]** | **k_{off} [s⁻¹]** | **K_{D} [M]** |
|---|---|---|---|---|
| Affilin | - | 1.48 x 10⁵ | 1.34 x 10⁻⁴ | 0.91 x 10⁻⁹ |
| 43 | NH | 4.96 x 10⁴ | 2.29 x 10⁻⁴ | 4.61 x 10⁻⁹ |
| 42 | CH | 138 | 9.45 x 10⁻⁵ | 686 x 10⁻⁹ |
| 40 | CL | 7.78 x 10³ | 2.82 x 10⁻⁴ | 36.25 x 10⁻⁹ |
| 46 | CH/CL | 2.66 x 10⁴ | 3.22 x 10⁻⁵ | 1.21 x 10⁻⁹ |
| 47 | NH/CL | 7.95 x 10⁴ | 1.51 x 10⁻⁴ | 1.90 x 10⁻⁹ |
| 45 | CH/NL | 1.23 x 10⁵ | 3.69 x 10⁻⁵ | 0.30 x 10⁻⁹ |

**Table 8B: for CD3**

| **PID** | **Fusion site** | **kₒₙ [M-¹ x s⁻¹]** | **k_{off} [s⁻¹]** | **K_{D} [M]** |
|---|---|---|---|---|
| Fab | - | 2.21 x 10⁵ | 2.96 x 10⁻³ | 13.39 x 10⁻⁹ |
| 43 | NH | 2.84 x 10⁵ | 3.06 x 10⁻³ | 10.77 x 10⁻⁹ |
| 42 | CH | 5.87 x 10⁵ | 1.26 x 10⁻³ | 2.14 x 10⁻⁹ |
| 40 | CL | 3.81 x 10⁵ | 1.5 x 10⁻³ | 3.92 x 10⁻⁹ |
| 46 | CH/CL | 7.79 x 10⁵ | 1.01 x 10⁻³ | 1.30 x 10⁻⁹ |
| 47 | NH/CL | 1.42 x 10⁶ | 8.58 x 10⁻⁴ | 0.60 x 10⁻⁹ |
| 45 | CH/NL | 2.66 x 10⁵ | 2.43 x 10⁻³ | 9.11 x 10⁻⁹ |

For example, the K_{D}-values of the complex fusion protein PID 46 are in a nanomolar range for both targets, confirming that the high binding affinities of the Affilin and the Fab-fragment are combined in these proteins.

For detection of simultaneous binding, a chip with immobilized PD1 was used, 50 s after the injection of the binding proteins, CD3 was added and the dissociation was monitored for 120 s. Figure 12 shows the simultaneous binding of Fabfilins to both targets.

### Example 8. Mabfilin and Fabfilin HER2/CD3 bind both targets simultaneously

A CM5 sensor Chip (GE Healthcare) with immobilized HER2 (about 2500 RU) and CD3 (about 2800 RU) was prepared. Table 9A shows affinity data for bispecific Mabfilin (HER2/CD3) and Fabfilin (HER2/CD3) binding proteins for HER2 target. Table 9B shows further affinity data for described proteins binding to CD3 target. All binding proteins bind specific to the respective target protein CD3 or HER2.

### Table 9: Binding of Mabfilin HER2/CD3 and Fabfilin HER2/CD3

**Table 9A. Binding to HER2**

| **PID** | **fusion site** | **target Affilin/Ab** | **Kₒₙ (M*s)⁻¹]** | **K_{off} [s⁻¹]** | **K_{D} [nM]** |
|---|---|---|---|---|---|
| Affilin | - | HER2 | 4.4*10⁵ | 1.51*10⁻³ | 3.43 |
| 71 | CL | HER2/CD3 (mAb) | 6.28*10⁵ | 1.2*10⁻⁴ | 0.191 |
| 79 | CL | HER2/CD3 (Fab) | 5.75*10⁵ | 6.43*10⁻⁴ | 1.12 |

**Table 9B: Binding to CD3**

| **PID** | **fusion site** | **target Affilin/Ab** | **Kₒₙ [(M*s)⁻¹]** | **K_{off} [s⁻1]** | **K_{D} [nM]** |
|---|---|---|---|---|---|
| Mab | - | CD3 | 5.93*10³ | 9.39*10³ | 1580 |
| Fab | - | CD3 | 1.6*10³ | 1.47*10⁻² | 9190 |
| 71 | CL | HER2/CD3 (mAb) | 5.17*10⁴ | 1.74*10⁻³ | 33.7 |
| 79 | CL | HER2/CD3 (Fab) | 1.33*10⁴ | 8.84*10⁻² | 662 |

For simultaneous binding of both targets a chip with immobilized HER2 was used, 150 s after the injection of the binding proteins, soluble CD3 was injected and the dissociation was monitored for 300 s. Figure 13 shows the simultaneous binding of PID 71 to both targets.

### Example 9. Mabfilin proteins bind specific to EGFR or HER2 overexpressing cell lines

Flow cytometry was used to analyze the interaction of binding proteins with cellular presented EGFR or HER2. EGFR or HER2 overexpressing CHO-K1 cells were used (negative control: empty vector control CHO-K1 cells). Cells were detached from the culture flask bottom and diluted in pre-cooled FACS blocking buffer and a cell suspension dilution was prepared for cell staining. As the cell number was determined, the cells were adjusted to 1×10³ cells/ml. Then, diluted cell suspension was transferred into a 96 well plate (Greiner) in triplicate for each cell line. Different concentrations of binding proteins were added to the cells and incubated. The supernatants were removed and 100 µl/well of goat anti-human IgG Alexa Fluor 488 antibody 1:1000 diluted in FACS blocking buffer were added. Flow cytometry measurement was conducted on the Guava Easy Cyte HT device from Millipore at excitation wavelength 499 nm and emission wavelength 520 nm. Binding of fusion proteins to human EGFR exogenously expressed on cells was confirmed (see Table 10).

**Table 10: Binding of Mabfilin EGFR/EGFR proteins to cellular EGFR**

| **PID** | **fusion site** | **target Affilin/mAB** | **K_{D} [nM]** | **EC₅₀ [nM]** |
|---|---|---|---|---|
| Mab | - | -/EGFR | 1.2 | 1.2 |
| 16 | CH | EGFR/EGFR | 0.6 | 0.6 |
| 22 | CL | EGFR/EGFR | 0.9 | 0.9 |
| 13 | NH | EGFR/EGFR | 0.8 | 0.8 |
| 19 | NL | EGFR/EGFR | 0.5 | 0.8 |
| 35 | NH/CL | EGFR/EGFR | 1.5 | 1.4 |

All binding proteins tested specifically bind to extracellular EGFR and to extracellular HER2. No non-specific binding was observed on cell lines that did not express EGFR and HER2. All fusion proteins bind specifically to the respective target cells but not to the vector control cells. Figure 14 demonstrates binding of bispecific Fabfilin EGFR/EGFR (Figure 14A), Fabfilin HER2/EGFR (Figure 14B) and control proteins to EGFR overexpressing cell line and Fabfilin HER2/EGFR (Figure 14C) specifically binds to HER2 overexpressing cells. All proteins bind specific to the cellular presented target.

### Example 10. Fabfilin PD1/CD3, Mabfilin HER2/CD3 and Fabfilin (HER2/CD3, HER2/EGFR) bind specific to target overexpressing cell lines

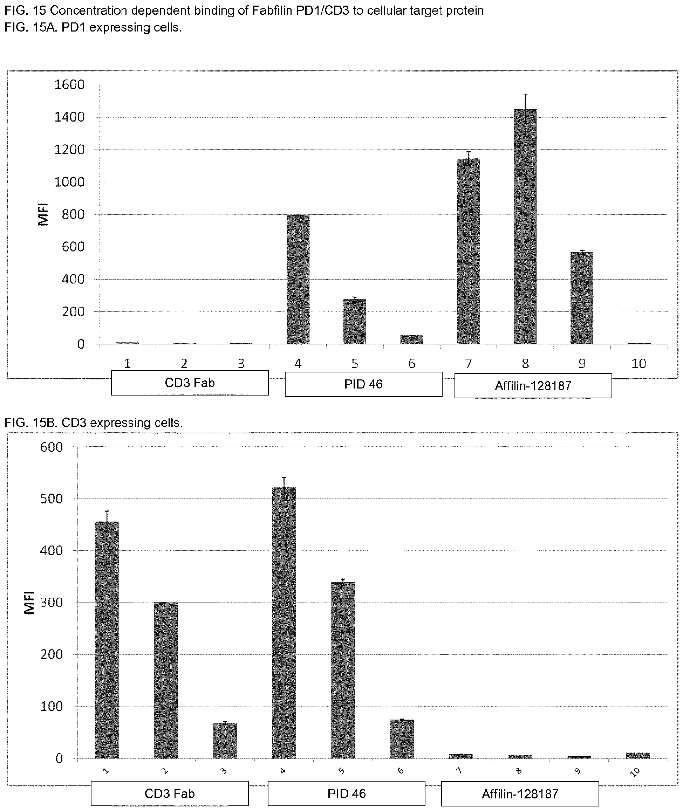

For cellular target binding, flow cytometry was used to analyze the interaction of Fabfilin proteins with PD1 and CD3. PD1 overexpresssing HeLa cells and fresh T-cells were used. Affilin-128187 and 145-2C11 Fab fragment were used as positive control. Applied methods for flow cytometry were analog to methods as described above (Example 9), except the detection of bound Fabfilins by rabbit anti strep-tag antibody in 1:300 dilution and with Alexa488 labeled anti rabbit IgG antibody. Figure 15 demonstrates specific binding of Fabfilin PD1/CD3 to cellular presented PD1 and CD3. Even for the lowest used concentration, clear binding to cellular target was still detectable. No non-specific binding was observed on cell lines that did not express the respective target.

For CD3 binding of HER2/CD3 bispecific proteins, CD3 positive Jurkat cell line and CD3 negative cell line K562 were used. Binding to cellular HER2 was determined as described above. Figure 16 shows that all proteins bind specific to the respective cellular presented target.

### Example 11: Mabfilin and Fabfilin proteins (EGFR/EGFR) show strong proliferation inhibition on EGFR overexpressing tumor cell lines

One important property of bispecific molecules is binding to different epitopes of a target like for example the membrane receptor EGFR. Thereby, the binding molecules might interfere with activation of EGFR on tumor cell lines and as result, specifically avoid stimulation of proliferation by growth factor stimulation. To determine proliferation inhibition for EGFR, an assay was developed, wherein EGFR overexpressing tumor cell line A431 was specifically stimulated by EGF. To compare different fusion proteins, the assay was accomplished in 96-well format. EGFR overexpressing A431 cells were seeded into the wells of a 96-well culture plate and incubated for 24 h in RPMI 1460 (2.5 mM Ultraglutamine-1, Lonza) culture medium without any additional supplement at 37°C, 5 % CO₂ and 95 % humidity. Fusion proteins were diluted into 1640 RPMI (2.5 mM Ultraglutamin-1, Lonza) containing EGF. 24 h after seeding of cells, different Mabfilin EGFR/EGFR and Fabfilin EGFR/EGFR proteins were added. Incubation of cells with described binding proteins was done for further 72 h at 37°C, 5 % CO₂ and 95 % humidity. Proliferation of A431 cells was determined by WST-1 proliferation assay (Roche). WST-1 ready to use reagent was added to the wells according to the manufacturer and incubated for 4 h at 37°C, 5 % CO₂ and 95 % humidity. The WST-1 tetrazolium salt is specifically reduced to a formazan derivate by viable cells. The content of formazan derivate correlates to the quantity of metabolic active cells. The amount of formazan was measured photometrically at 450 nm using a Tecan Infinite plate reader. Reference at 620 nm was subtracted to decrease background influence.

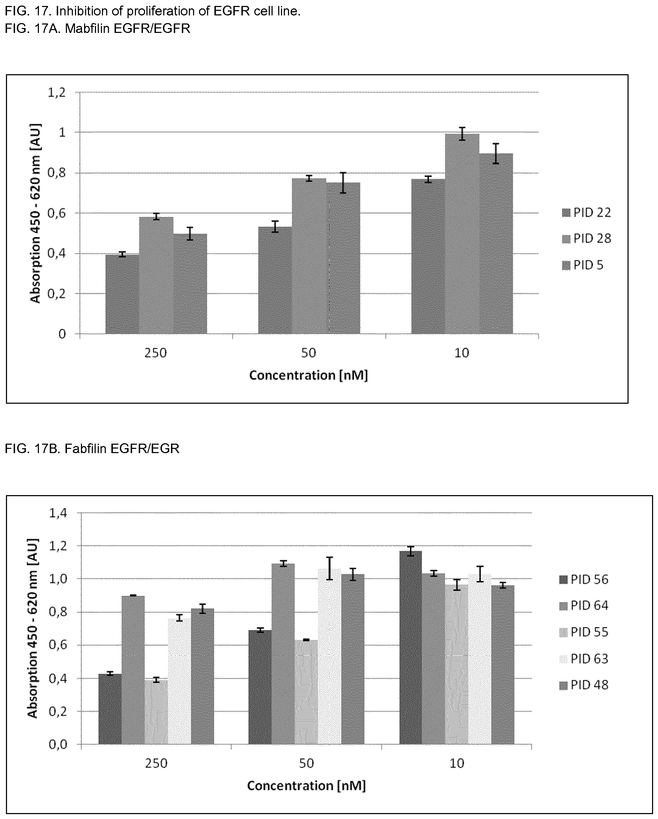

Figure 17 shows proliferation inhibition of A431 after incubation with different EGFR binding proteins of this invention. Mabfilin EGFR/EGFR and Fabfilin EGFR/EGFR proteins stronger inhibit cell proliferation than EGFR-binding control antibodies or Fab fragments. Fusion proteins of this invention have an increased biological effect on EGFR overexpressing tumor cells. Tested proteins might be able to overcome limitations of current tumor therapies, e.g. wherein EGFR overexpression is confirmed.

## Claims

1. A binding protein, comprising at least a first binding protein wherein the first binding protein is a ubiquitin mutein with specific binding affinity (K_{D}) of less than 700 nM to a first epitope and wherein the ubiquitin mutein has a sequence identity of at least 80 % to the amino acid sequence defined by SEQ ID NO: 1 or by SEQ ID NO: 2 or by SEQ ID NO: 3 or by SEQ ID NO: 4, and at least a second binding protein wherein the second binding protein is a monoclonal antibody or a fragment thereof with binding specificity to a second epitope, and optionally a linker between at least the first binding protein and at least the second binding protein.

2. The binding protein according to claim 1, wherein the first binding protein is linked to the heavy chain or to the light chain of the second binding protein, preferably wherein the first binding protein is linked to the C-terminus of the light or heavy chain of the monoclonal antibody or a fragment thereof, or the first binding protein is linked to the N-terminus of the light or heavy chain of the monoclonal antibody or a fragment thereof, or a combination thereof.

3. The binding protein according to claim 1 or claim 2, wherein the first binding protein and the second binding protein have specific binding affinity to an epitope on a target antigen, selected from at least one member of the group consisting of a cancer target antigen, a receptor target antigen on immune cells, or a soluble target antigen selected from hormones, and cytokines wherein the first and the second binding protein bind to different epitopes.

4. The binding protein according to any of the preceding claims, wherein the first binding protein comprises an amino acid sequence selected from the group consisting of SEQ ID NO: 7 to 10 or 43 or an amino acid sequence that exhibits at least 80 % sequence identity to one or more of the amino acid sequences of SEQ ID NO: 7 to 10 or 43.

5. The binding protein according to any of the preceding claims, wherein the first binding protein comprises a ubiquitin mutein with specific binding affinity (K_{D}) of less than 700 nM to EGFR or HER2 or PD1, and wherein the second binding protein comprises or consists of a monoclonal antibody or a fragment thereof with binding specificity to EGFR or HER2 or CD3.

6. The binding protein according to any of the preceding claims, wherein the first binding protein comprises a ubiquitin mutein with specific binding affinity (K_{D}) of less than 700 nM to EGFR, and wherein the second binding protein comprises of a monoclonal antibody or fragment thereof with binding specificity to EGFR.

7. The binding protein according to any of the preceding claims, wherein the first binding protein comprises a ubiquitin mutein with specific binding affinity (K_{D}) of less than 700 nM to HER2, and wherein the second binding protein comprises a monoclonal antibody or fragment thereof with binding specificity to EGFR, or wherein the first binding protein comprises a ubiquitin mutein with specific binding affinity (K_{D}) of less than 700 nM to PD1, and wherein the second binding protein comprises a monoclonal antibody or fragment thereof with binding specificity to CD3, or wherein the first binding protein comprises a ubiquitin mutein with specific binding affinity (K_{D}) of less than 700 nM to HER2, and wherein the second binding protein comprises a monoclonal antibody or fragment thereof with binding specificity to CD3.

8. The binding protein according to any of the preceding claims, wherein the first and the second binding protein bind to non-overlapping epitopes.

9. The binding protein according to any of the preceding claims, further comprising at least one additional molecule, preferably further comprising a further binding protein.

10. A nucleic acid molecule encoding the binding protein as defined in any one of claims 1 to 9.

11. A vector comprising the nucleic acid molecule of claim 10.

12. A host cell or a non-human host comprising a binding protein as defined in any one of claims 1 to 9, a nucleic acid as defined in claim 10, or a vector of claim 11.

13. The binding protein of any one of claims 1 to 9 or the nucleic acid molecule of claim 10 or the vector of claim 11 or the host cell of claim 12 for use in medicine.

14. A composition comprising the binding protein as defined in any one of claims 1 to 9; the nucleic acid molecule as defined in claim 10; the vector as defined in claim 11; and/or the host cell as defined in claim 12.

15. A method for the production of the binding protein of any of the preceding claims 1 to 9 that specifically binds to a pre-defined epitope comprising culturing of the host cell of claim 12 under suitable conditions in order to obtain said binding protein and optionally isolating said binding protein.

## Patentansprüche

1. Bindeprotein umfassend mindestens ein erstes Bindeprotein, wobei es sich bei dem ersten Bindeprotein um ein Ubiquitin-Mutein mit einer spezifischen Bindungsaffinität (K_{D}) von weniger als 700 nM für ein erstes Epitop handelt, und wobei das Ubiquitin-Mutein eine Sequenzidentität von mindestens 80% zu der durch SEQ ID NO: 1 oder SEQ ID NO: 2 oder SEQ ID NO: 3 oder SEQ ID NO: 4 definierten Aminosäuresequenz aufweist, und mindestens ein zweites Bindeprotein, wobei es sich bei dem zweiten Bindeprotein um einen monoklonalen Antikörper, oder ein Fragment davon, mit Bindungsspezifität für ein zweites Epitop handelt, und optional einen Linker zwischen dem mindestens ersten Bindeprotein und dem mindestens zweiten Bindeprotein.

2. Das Bindeprotein nach Anspruch 1, wobei das erste Bindeprotein mit der schweren Kette oder mit der leichten Kette des zweiten Bindeproteins verbunden ist, vorzugsweise wobei das erste Bindeprotein mit dem C-Terminus der leichten oder schweren Kette des monoklonalen Antikörpers oder eines Fragments davon verbunden ist, oder das erste Bindeprotein mit dem N-Terminus der leichten oder schweren Kette des monoklonalen Antikörpers oder eines Fragments davon verbunden ist, oder eine Kombination davon.

3. Das Bindeprotein nach Anspruch 1 oder Anspruch 2, wobei das erste Bindeprotein und das zweite Bindeprotein eine spezifische Bindungsaffinität für ein Epitop auf einem Zielantigen aufweisen, welches ausgewählt ist aus mindestens einem Mitglied der Gruppe bestehend aus einem Krebs-Zielantigen, einem Rezeptor-Zielantigen auf Immunzellen, oder einem löslichen Zielantigen ausgewählt aus Hormonen und Zytokinen, wobei das erste und das zweite Bindeprotein an unterschiedliche Epitope binden.

4. Das Bindeprotein nach einem der vorhergehenden Ansprüche, wobei das erste Bindeprotein eine Aminosäuresequenz umfasst, welche ausgewählt ist aus der Gruppe bestehend aus SEQ ID NOs: 7 bis 10 oder 43, oder welche mindestens 80% Sequenzidentität zu einer oder mehreren der Aminosäuresequenzen von SEQ ID NOs: 7 bis 10 oder 43 aufweist.

5. Das Bindeprotein nach einem der vorhergehenden Ansprüche, wobei das erste Bindeprotein ein Ubiquitin-Mutein mit einer spezifischen Bindungsaffinität (K_{D}) von weniger als 700 nM für EGFR oder HER2 oder PD1 umfasst, und wobei das zweite Bindeprotein einen monoklonalen Antikörper, oder ein Fragment davon, mit Bindungsspezifität für EGFR oder HER2 oder CD3 umfasst oder aus einem solchen monoklonalen Antikörper oder Fragment davon besteht.

6. Das Bindeprotein nach einem der vorhergehenden Ansprüche, wobei das erste Bindeprotein ein Ubiquitin-Mutein mit einer spezifischen Bindungsaffinität (K_{D}) von weniger als 700 nM für EGFR umfasst, und wobei das zweite Bindungsprotein einen monoklonalen Antikörper, oder ein Fragment davon, mit Bindungsspezifität für EGFR umfasst.

7. Das Bindeprotein nach einem der vorhergehenden Ansprüche, wobei das erste Bindeprotein ein Ubiquitin-Mutein mit einer spezifischen Bindungsaffinität (K_{D}) von weniger als 700 nM für HER2 umfasst, und wobei das zweite Bindeprotein einen monoklonalen Antikörper, oder ein Fragment davon, mit Bindungsspezifität für EGFR umfasst, oder wobei das erste Bindeprotein ein Ubiquitin-Mutein mit einer spezifischen Bindungsaffinität (K_{D}) von weniger als 700 nM für PD1 umfasst, und wobei das zweite Bindeprotein einen monoklonalen Antikörper, oder ein Fragment davon, mit Bindungsspezifität für CD3 umfasst, oder wobei das erste Bindeprotein ein Ubiquitin-Mutein mit einer spezifischen Bindungsaffinität (K_{D}) von weniger als 700 nM für HER2 umfasst, und wobei das zweite Bindeprotein einen monoklonalen Antikörper, oder ein Fragment davon, mit Bindungsspezifität für CD3 umfasst.

8. Das Bindeprotein nach einem der vorhergehenden Ansprüche, wobei das erste und das zweite Bindeprotein an nicht-überlappende Epitope binden.

9. Das Bindeprotein nach einem der vorhergehenden Ansprüche, weiterhin umfassend mindestens ein zusätzliches Molekül, vorzugsweise weiterhin umfassend ein weiteres Bindeprotein.

10. Nukleinsäuremolekül, welches das Bindeprotein nach einem der Ansprüche 1 bis 9 kodiert.

11. Vektor umfassend das Nukleinsäuremolekül nach Anspruch 10.

12. Wirtszelle oder nicht-menschlicher Wirt, umfassend ein Bindeprotein nach einem der Ansprüche 1 bis 9, ein Nukleinsäuremolekül nach Anspruch 10, oder einen Vektor nach Anspruch 11.

13. Das Bindeprotein nach einem der Ansprüche 1 bis 9, oder das Nukleinsäuremolekül nach Anspruch 10, oder der Vektor nach Anspruch 11, oder die Wirtszelle nach Anspruch 12, für die Verwendung in der Medizin.

14. Zusammensetzung umfassend das Bindeprotein nach einem der Ansprüche 1 bis 9; das Nukleinsäuremolekül nach Anspruch 10; den Vektor nach Anspruch 11; und/oder die Wirtszelle nach Anspruch 12.

15. Verfahren zur Herstellung des Bindeproteins nach einem der vorhergehenden Ansprüche 1 bis 9, welches spezifisch an ein vordefiniertes Epitop bindet, umfassend das Züchten der Wirtszelle nach Anspruch 12 unter Bedingungen, welche geeignet sind, das Bindeprotein zu erhalten, und optional das Isolieren des Bindeproteins.

## Revendications

1. Protéine de liaison, comprenant au moins une première protéine de liaison, où la première protéine de liaison est une ubiquitine-mutéine ayant une affinité de liaison spécifique (K_{D}) inférieure à 700 nM pour un premier épitope et où l'ubiquitine-mutéine présente une identité de séquence d'au moins 80 % avec la séquence d'acides aminés définie par SEQ ID NO: 1 ou par SEQ ID NO: 2 ou par SEQ ID NO: 3 ou par SEQ ID NO: 4, et au moins une deuxième protéine de liaison, où la deuxième protéine de liaison est un anticorps monoclonal ou un fragment de celui-ci ayant une spécificité de liaison pour un deuxième épitope, et éventuellement un lieur entre ladite au moins première protéine de liaison et ladite au moins deuxième protéine de liaison.

2. Protéine de liaison selon la revendication 1, où la première protéine de liaison est reliée à la chaîne lourde ou à la chaîne légère de la deuxième protéine de liaison, de préférence où la première protéine de liaison est reliée à l'extrémité C-terminale de la chaîne légère ou lourde de l'anticorps monoclonal ou d'un fragment de celui-ci, ou la première protéine de liaison est reliée à l'extrémité N-terminale de la chaîne légère ou lourde de l'anticorps monoclonal ou d'un fragment de celui-ci, ou une combinaison des précédents.

3. Protéine de liaison selon la revendication 1 ou la revendication 2, où la première protéine de liaison et la deuxième protéine de liaison ont une affinité de liaison spécifique pour un épitope sur un anticorps cible, sélectionné parmi au moins un membre du groupe constitué par un antigène cible de cancer, un antigène cible de récepteur sur des cellules immunitaires, ou un antigène cible soluble sélectionné parmi des hormones et cytokines, où la première et la deuxième protéine de liaison se lient à des épitopes différents.

4. Protéine de liaison selon n'importe lesquelles des revendications précédentes, où la première protéine de liaison comprend une séquence d'acides aminés sélectionnée dans le groupe constitué par SEQ ID NO: 7 à 10 ou 43 ou une séquence d'acides aminés qui présente au moins 80 % d'identité de séquence avec une ou plusieurs des séquences d'acides aminés de SEQ ID NO: 7 à 10 ou 43.

5. Protéine de liaison selon n'importe lesquelles des revendications précédentes, où la première protéine de liaison comprend une ubiquitine-mutéine ayant une affinité de liaison spécifique (K_{D}) inférieure à 700 nM pour EGFR ou HER2 ou PD1, et où la deuxième protéine de liaison comprend ou est constituée par un anticorps monoclonal ou un fragment de celui-ci présentant une spécificité de liaison pour EGFR ou HER2 ou CD3.

6. Protéine de liaison selon n'importe lesquelles des revendications précédentes, où la première protéine de liaison comprend une ubiquitine-mutéine ayant une affinité de liaison spécifique (K_{D}) inférieure à 700 nM pour EGFR, et où la deuxième protéine de liaison comprend un anticorps monoclonal ou fragment de celui-ci présentant une spécificité de liaison pour EGFR.

7. Protéine de liaison selon n'importe lesquelles des revendications précédentes, où la première protéine de liaison comprend une ubiquitine-mutéine ayant une affinité de liaison spécifique (K_{D}) inférieure à 700 nM pour HER2, et où la deuxième protéine de liaison comprend un anticorps monoclonal ou fragment de celui-ci présentant une spécificité de liaison pour EGFR, ou où la première protéine de liaison comprend une ubiquitine-mutéine ayant une affinité de liaison spécifique (K_{D}) inférieure à 700 nM pour PD1, et où la deuxième protéine de liaison comprend un anticorps monoclonal ou fragment de celui-ci présentant une spécificité de liaison pour CD3, ou où la première protéine de liaison comprend une ubiquitine-mutéine ayant une affinité de liaison spécifique (K_{D}) inférieure à 700 nM pour HER2, et où la deuxième protéine de liaison comprend un anticorps monoclonal ou fragment de celui-ci présentant une spécificité de liaison pour CD3.

8. Protéine de liaison selon n'importe lesquelles des revendications précédentes, où la première et la deuxième protéine de liaison se lient à des épitopes non chevauchants.

9. Protéine de liaison selon n'importe lesquelles des revendications précédentes, comprenant en outre au moins une molécule supplémentaire, de préférence comprenant en outre une protéine de liaison supplémentaire.

10. Molécule d'acide nucléique codant la protéine de liaison telle que définie dans l'une quelconque des revendications 1 à 9.

11. Vecteur comprenant la molécule d'acide nucléique selon la revendication 10.

12. Cellule hôte, ou hôte non humain, comprenant une protéine de liaison telle que définie dans l'une quelconque des revendications 1 à 9, un acide nucléique tel que défini dans la revendication 10, ou un vecteur selon la revendication 11.

13. Protéine de liaison selon l'une quelconque des revendications 1 à 9 ou molécule d'acide nucléique selon la revendication 10 ou vecteur selon la revendication 11 ou cellule hôte selon la revendication 12 pour une utilisation en médecine.

14. Composition comprenant la protéine de liaison telle que définie dans l'une quelconque des revendications 1 à 9 ; la molécule d'acide nucléique telle que définie dans la revendication 10 ; le vecteur tel que défini dans la revendication 11 ; et/ou la cellule hôte telle que définie dans la revendication 12.

15. Procédé de production de la protéine de liaison selon l'une quelconque des revendications 1 à 9 qui se lie spécifiquement à un épitope prédéfini, comprenant la mise en culture de la cellule hôte selon la revendication 12 dans des conditions appropriées pour obtenir ladite protéine de liaison et éventuellement, l'isolement de ladite protéine de liaison.
